# EUROPÄISCHE PATENTSCHRIFT

(11) **EP 0 767 220 B1**
(45) Veröffentlichungstag und Bekanntmachung des Hinweises auf die Patenterteilung: **28.04.1999**
(21) Anmeldenummer: 96114578.6
(22) Anmeldetag: 12.09.1996
(51) Int. Cl.: C09B 29/36, C07C 255/23, C07D 213/64, C07D 213/80, C07D 213/85, C07D 213/75, C07D 221/16, C07D 417/06, C09B 31/153, C09B 62/006, C09B 44/00

(54) **Pyridonmethidazofarbstoffe**
Pyridonmethidazo dyes
Colorants azoiques dérivés de méthides de pyridone

(30) Priorität: 25.09.1995 DE 19535501
(43) Veröffentlichungstag der Anmeldung: 09.04.1997
(73) Patentinhaber: BAYER AG, 51368 Leverkusen (DE)
(72) Erfinder: Hamprecht, Rainer, Dr., 51519 Odenthal (DE)

(56) Entgegenhaltungen:
- AU-B- 491 554
- DE-A- 4 020 768
- DE-A- 4 329 296
- LIEBIGS ANNALEN DER CHEMIE, Dezember 1987, WEINHEIM DE, Seiten 1131-1132, XP002023274 M.MITTELBACH ET AL.: "Untersuchungen zur Struktur der Dimeren des Malononitrils, Cyanessigesters und Cyanacetamids"

## Beschreibung

Die Erfindung betrifft Pyridonmethidazofarbstoffe, Verfahren zu ihrer Herstellung, ihre Verwendung sowie pyridonmethidhaltige Kupplungskomponenten.

Aus DE-A 4 020 768 und DE-A 4 329 296 sind bereits Azofarbstoffe bekannt, die eine Kupplungskomponente auf Basis von Methylen-Triazolo-Pyridinen enthalten. Diese Azofarbstoffe weisen jedoch noch einige anwendungstechnische Nachteile auf. So ist beispielsweise ihr Aufbauvermögen sowie ihre Färbebadstabilität bei der Polyesterfärbung nicht zufriedenstellend.

Es wurden nun Pyridonmethidazofarbstoffe gefunden, die in Form der freien Säure der Formel (I) oder ihrer tautomeren Form entsprechen 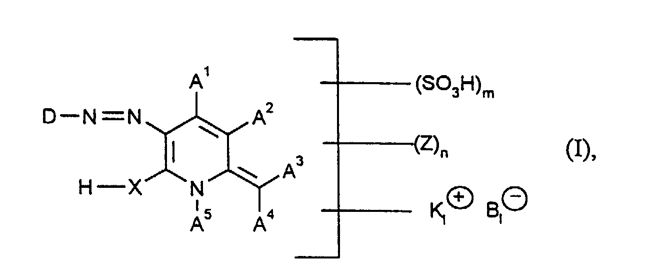 worin
- D: der Rest einer carbo- oder heterocyclische Diazokomponente ist,
- A¹ und A²: unabhängig voneinander H oder einen für Pyridone typische Substituenten
bedeuten,
- A³ und A⁴: unabhängig voneinander einen elektronenziehenden Rest bedeuten, oder zusammen mit dem gemeinsamen C-Atom eine cyclische methylenaktive Verbindung bilden,
- A⁵: für H, einen Rest der Formel T, -OT¹, -NH₂, -NHT, -NT₂, -NHCOH, -NHCOT, -N=CH-T, -N=CT₂ oder -NHSO₂T steht,
worin
- T¹ =: Alkyl, Cycloalkyl oder Aralkyl bedeutet und T für T¹ steht oder die Bedeutung von T² bis T⁵ annehmen kann, mit T² = Alkenyl
- T³ =: Alkinyl
- T⁴ =: Aryl
- T⁵ =: Hetaryl
oder
- A¹ und A²: und/oder
- A² und A³: und/oder
- A⁴ und A⁵: zusammen mit den jeweils dazwischenliegenden Atomen einen ungesättigten, gegebenenfalls substituierten 5- oder 6-gliedrigen Carbo- oder Heterocyclus bilden, mit der Maßgabe, daß bei einer Ringbildung unter Beteiligung eines der beiden Reste A³ und A⁴ der nicht beteiligte Rest einen elektronenziehenden Rest bedeutet,
- X: für O, NH, NT, NCOT, NCO₂T oder NSO₂T steht,
- K^{⊕}: -NH₃^{⊕}, -NHT₂^{⊕}, -NH₂T^{⊕}, -NT₃^{⊕} oder Cycloimmoniumion,
- B^{⊖}: Anion
- Z: ein faserreaktiver Rest ist
- l: für 0 bis 2,
- m: für 0 bis 8 und
- n: für 0 bis 6 steht.

Bevorzugt sind Pyridonmethidazofarbstoffe, die der Formel (I) oder ihrer tautomeren Form entsprechen,
worin
- A¹ und A²: unabhängig voneinander H oder einen Rest der Formel T, -COH, -CO-T, -CO₂T, -CN, -CONH₂, -CONHT, -CONT₂, CF₃, -NH₂, -NHT, -NT₂, -NH-COT, -NT-COT, -NHSO₂T, -NTSO₂T, -NO₂, -NO, -SO₂T, -OH, -OT, -OCOT, -OCO₂T, -OSO₂T, Cl, Br, J
bedeuten, wobei
- T: die Bedeutung von T¹, T², T³, T⁴ oder T⁵ annehmen kann.

Die erfindungsgemäßen Farbstoffe der Formel (I) können in verschiedenen tautomeren Formen vorliegen. Eine Form entspricht beispielsweise der Formel (I), eine andere entspricht beispielsweise der folgenden Formel

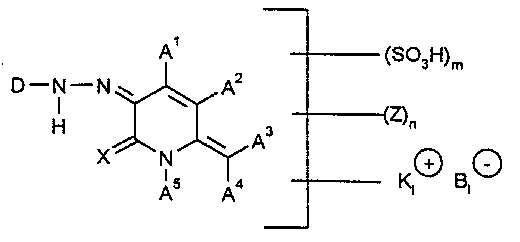

Vorzugsweise wird unter einem elektronenanziehenden Rest ein Rest verstanden, dessen Hammett'sche Substituentenkonstante σ (para) > 0 ist, wobei einer der beiden Substituenten A³ oder A⁴ vorzugsweise einen σ (para)-Wert > 0,300 besitzt. Eine entsprechende Auflistung von Hammett'schen Substituentenkonstanten findet sich z.B. in Sykes, Reaktionsmechanismen der organischen Chemie, 9. Aufl., Weinheim, VCH Verlagsgesellschaft, 1988, oder kann nach bekannten Verfahren bestimmt werden.
- T¹: steht vorzugsweise für C₁-C₂₀-Alkyl, C₄-C₈-Cycloalkyl oder C₆-C₁₀-Aryl-C₁-C₈-Alkyl, das jeweils unsubstituiert oder durch einen oder mehrere Substituenten aus der Gruppe OH, C₁-C₁₀-Alkoxy, 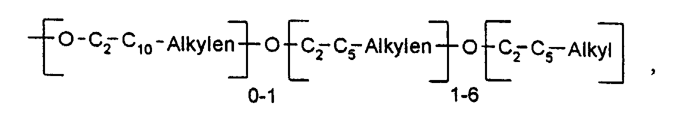 insbesondere
C₁-C₁₀-Alkoxy-C₂-C₅-alkoxy, C₁-C₅-Alkoxy-C₂-C₅-alkoxy-C₂-C₅-alkoxy, -OCOH, COH, -OCOT, -OSO₂T, -O(CH₂CH₂O)₁₋₆COT, -OCO₂T, -COT, -SO₂T, -CO₂T, -CN, -CONH₂, -CONHT, -CONT₂, -CO₂H, -SO₂NH₂, -SO₂NHT, -SO₂NT₂, -CF₃, -NO₂, Halogen, wie F, Cl, Br und J substituiert ist.
- T² und T³: stehen unabhängig voneinander vorzugsweise für C₂-C₂₀-Alkenyl oder C₂-C₂₀-Alkinyl, das jeweils unsubstituiert ist oder durch einen oder mehrere Substituenten aus der Gruppe der bereits unter T¹ genannten Substituenten substituiert ist.
- T⁴: steht vorzugsweise für C₆-C₁₆-Aryl, das unsubstituiert oder durch einen oder mehrere Substituenten aus der Gruppe der bereits unter T¹ genannten Substituenten substituiert ist, wobei als weitere Substituenten -NH₂, -NHT, -NT₂, -NHCOH, -NHCOT, NHSO₂T, C₂-C₁₀-Alkenyl, C₂-C₁₀-Alkinyl, C₁-C₁₀-Alkyl, C₄-C₈-Cycloalkyl sowie C₆-C₁₀-Aryl- C₁-C₆-Alkyl in Frage kommen.
- T⁵: steht vorzugsweise für einen 5- oder 6-gliedrigen aromatischen Heterocyclus, der 1 bis 3 gleiche oder verschiedene Heteroatome aus der Gruppe N, O, S, NH, SO, SO₂ enthält und unsubstituiert oder mit einem oder mehreren Substituenten aus der Gruppe der bereits unter T¹ genannten Substituenten substituiert ist und der gegebenenfalls mit einem oder zwei Aryl- oder Hetarylringen annelliert ist.

Bevorzugte Farbstoffe der Formel (I) sind solche, worin 1 = 1 oder 2 und n = 0, und m kleiner als 1, vorzugsweise 0 ist, die nachfolgend als "kationische Farbstoffe" der Formel (I) bezeichnet werden.

Ebenfalls bevorzugt sind Farbstoffe der Formel (I), worin 1 = m = n = 0 bedeuten, die nachfolgend als "Dispersionsfarbstoffe der Formel (I)" bezeichnet werden.

Bevorzugt sind weiterhin Farbstoffe der Formel (I), worin 1 = 0 und m und/oder n ungleich 0 sind. Diese erfindungsgemäßen Farbstoffe werden, sofern n = 0 ist, nachfolgend als "Säurefarbstoffe der Formel (I)" und sofern n ungleich 0 ist, nachfolgend als "Reaktivfarbstoffe der Formel (I)" bezeichnet.

Als Säurefarbstoffe der Formel I werden auch solche verstanden, die COOH-Gruppen tragen und worin m = 0 ist.

### Dispersionsfarbstoffe

Unter den Dispersionfarbstoffen der Formel (I) sind solche bevorzugt, worin
- A³ und A⁴: unabhängig voneinander für -CN, -CO₂T, -CONH₂, -CONHT, -CONT₂, CF₃, -CHO, -COT, -SO₂T, -SO₃T⁴, -SO₃T⁵, SO₂NH₂, SO₂NHT, SO₂NT₂, -SOT, -CH=NH, -CH=NT, -CT=NH, -CT=NT, 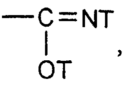 -CO-CO₂T, -NO₂, -NO, T⁴ oder T⁵
stehen, wobei vorzugsweise A³ und A⁴ nicht gleichzeitig für T⁴ und/oder T⁵ stehen,
oder
- A³ und A⁴: zusammen mit dem C-Atom, an das sie gebunden sind, für eine cyclische methylenaktive Verbindung der Formel (IIa) bis (IIv) stehen, wobei diese Reste in Form von 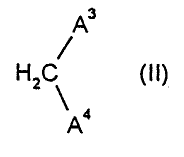 angegeben sind: 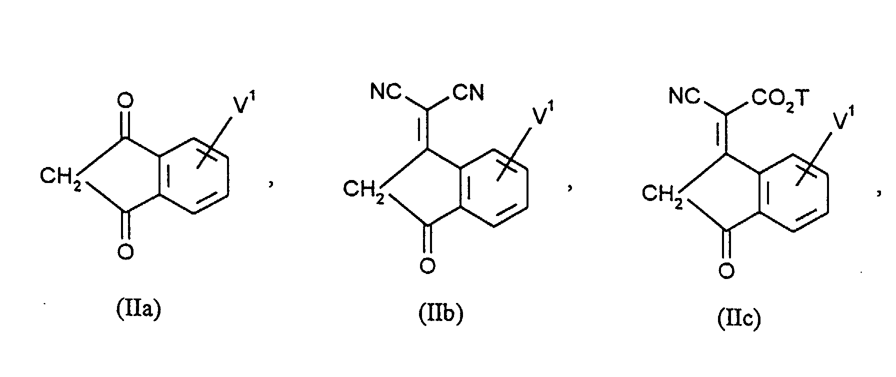 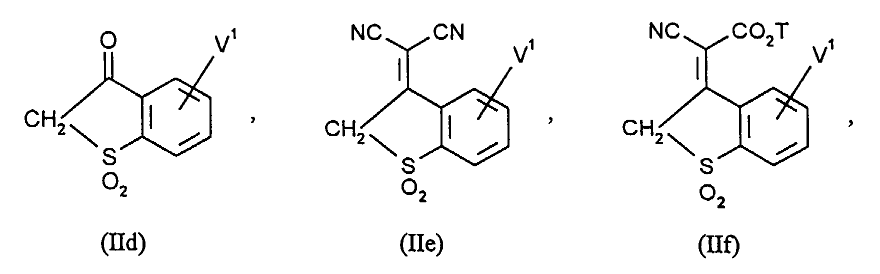 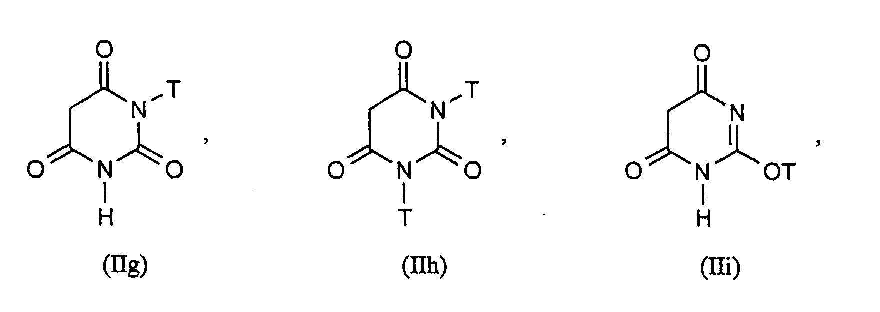 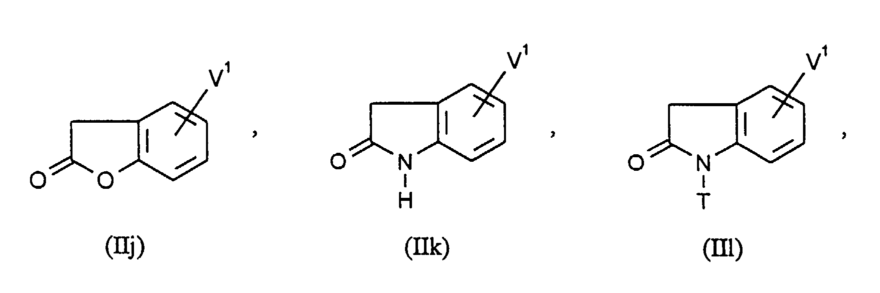 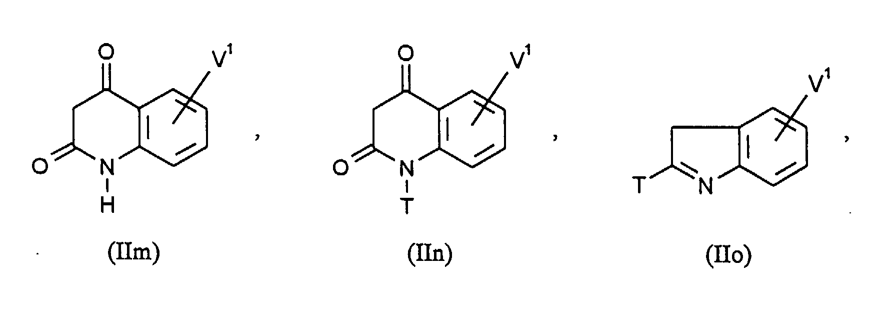 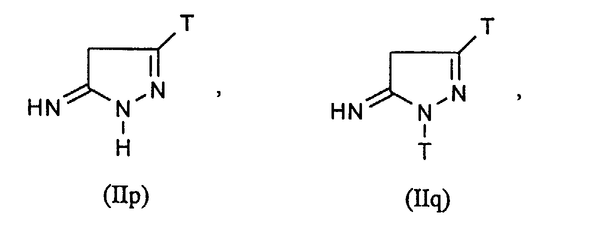 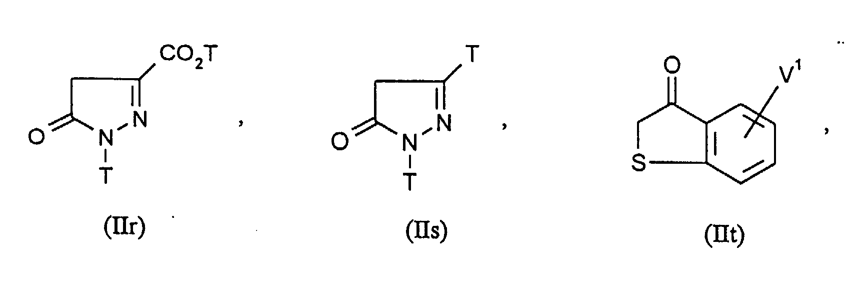 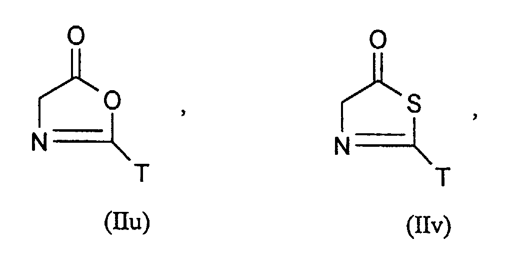
worin
- V¹: für H oder einen Substituenten, insbesondere Cl, Br, CH₃, -CO₂T¹, -CN, -NO₂, -CF₃ oder -SO₂T¹ steht und worin A¹, A², A⁵, D und X die vorgenannten Bedeutungen haben.

Unter den Dispersionfarbstoffen der Formel (I) sind solche bevorzugt, worin
- A¹: für H, T, -COH, -COT, -CO₂T, -CN, -CONH₂, -CONHT, -CONT₂, CF₃, OH oder Halogen, insbesondere Cl, Br und J steht,
- A²: für H, T, -COH, -COT, -CN, -CO₂T, -CONH₂, -CONHT, -CONT₂, -CF₃, -NO₂, -NO, Cl, Br, J steht,
- A³ und A⁴: unabhängig voneinander für -CN, -CO₂T, -CONH₂, -CONHT, -CONT₂, CF₃, -CHO, -COT, -SO₂T, -SO₃T⁴, -SO₃T⁵, SO₂NH₂, SO₂NHT, SO₂NT₂, -SOT, -CH=NH, -CH=NT, -CT=NH, -CT=NT, -CO-CO₂T, -NO₂, -NO, T⁴ oder T⁵ stehen, wobei vorzugsweise A³ und A⁴ nicht gleichzeitig für T⁴ und/oder T⁵ stehen, oder
- A² und A³: zusammen mit den zwischen ihnen liegenden C-Atomen den Rest eines gegebenenfalls durch R¹ substituierten annellierten Indenringes oder
- A³ und A⁴: zusammen mit dem C-Atom, an das sie gebunden sind, einen Carbo- oder Heterocyclus der Formel (IIa) bis (IIv) bilden,
- A⁵: H, T¹, T², T³, T⁴, -NH₂, -NHT, NT₂, -NHCOT, -NHCOH, -NHSO₂T,-N=CT₂ bedeutet oder A⁴ und A⁵ zusammen mit den dazwischen liegenden Atomen des Pyridonringes einen annellierten Ring der Formel 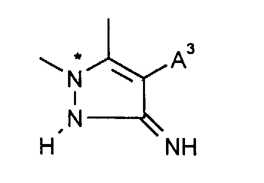 bilden, wobei das mit * markierte N-Atom dem Pyridon-Stickstoff entspricht,
- T¹: C₁-C₁₀-Alkyl, C₅-C₇-Cycloalkyl oder C₆-C₁₀-Aryl-C₁-C₆-Alkyl bedeutet, die gegebenenfalls durch einen oder mehrere Substituenten aus der Gruppe -OH, -C₁-C₁₀-Alkoxy, -O[(CH₂)₂₋₁₀-O]₁₋₆-Alkyl, insbesondere -C₁-C₁₀-Alkoxy-C₂-C₅-alkoxy, -C₁-C₅-Alkoxy-C₂-C₅-alkoxy-C₂-C₅-alkoxy oder -O-(CH₂-CH₂O 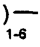 Alkyl
-OCOT, -OSO₂T, -O-(CH₂-CH₂O 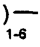 COT, -COT, -SO₂T, -CO₂T, -CN, -CO₂H, -CONT₂, -CF₃, Cl, Br, J, substituiert sind,
- T², T³: unabhängig voneinander C₂-C₁₀-Alkenyl oder Alkinyl bedeuten, die gegebenenfalls durch einen oder mehrere Substituenten aus der Gruppe OH, -C₁-C₆-Alkoxy, -OCOT, -OCOH, -CO₂T, -CO₂H, -CN, Cl, Br, J substituiert sind,
- T⁴: Phenyl bedeutet, das gegebenenfalls durch einen oder mehrere Substituenten wie -C₁-C₁₀-Alkoxy, 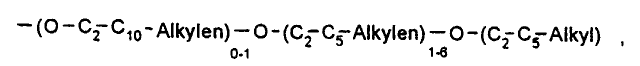 insbesondere
C₁-C₁₀-Alkoxy-C₂-C₅-alkoxy, C₁-C₅-Alkoxy-C₂-C₅-Alkoxy-C₂-C₅-alkoxy, -OCOH, -OCOT, -OSO₂T, -COH, -COT, -SO₂T, -CO₂T, -CN, -CF₃, -CCl₃, -NO₂, -NO, -CO₂H, -CONH₂, -CONHT, -CONT₂, -SO₂NT², -C₁-C₁₀-Alkyl, gegebenenfalls substituiert durch -OH, -CN, -CO₂-C₁-C₆-Alkyl, Cl, Br, J, -C₂-C₁₀-Alkenyl, gegebenenfalls substituiert durch -OH, -CN, -CO₂-C₁-C₆-Alkyl, Cl, Br, J, -C₂-C₁₀-Alkinyl, gegebenenfalls substituiert durch C₁-C₁₀-Alkoxy, -OH, -OCOH, -OCOT, Cl, Br, J, substituiert ist,
- T⁵: Thiophen, Furan, Pyrrol, 1,2-Isothiazol, 1,3-Thiazol, Pyrrazol, Oxazol, Isooxazol, Imidazol, Triazol, Pyridin, Pyridazin, Pyrimidin, Pyrazin, Triazin, Oxazin, Benzthiazol, Benzimidazol, Benzoxazol, Chinolin, Isochinolin, Indol, Cumaron, Thionaphthen, Tetrazol, gegebenenfalls substituiert durch 1 bis 3 Substituenten, wie bereits als Substituenten für Phenyl beschrieben sind,
- X: O, -NCOT, -NCO₂T, -NSO₂T,
- D: einen Rest der Formel (IIIa) bis (IIIu) bedeutet, 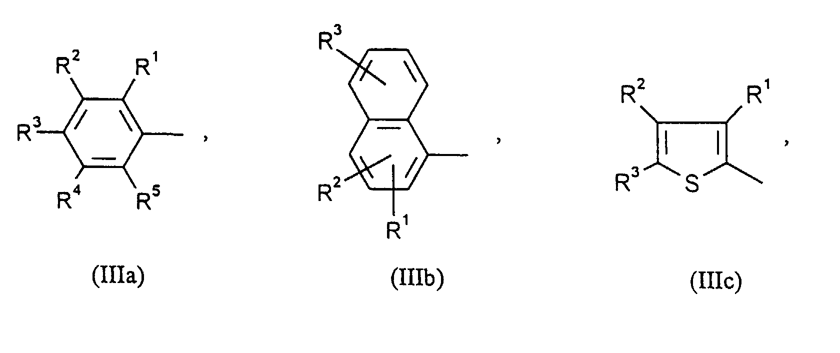 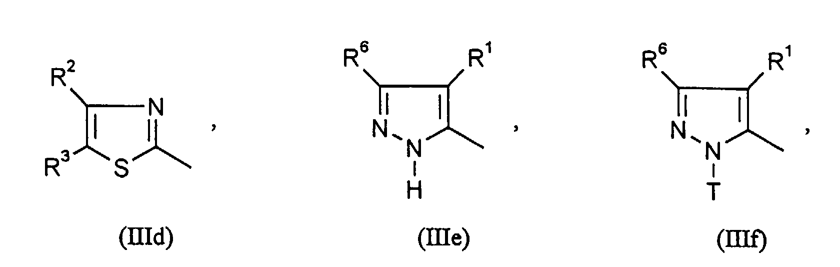 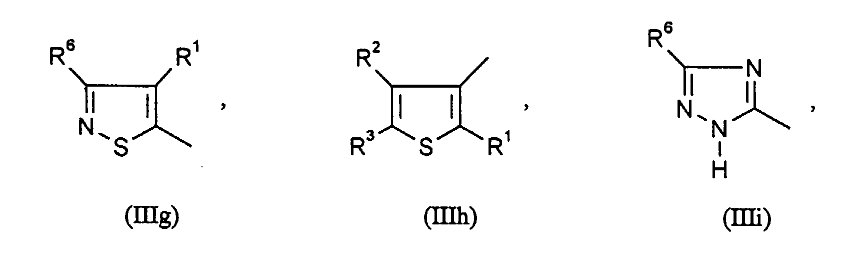 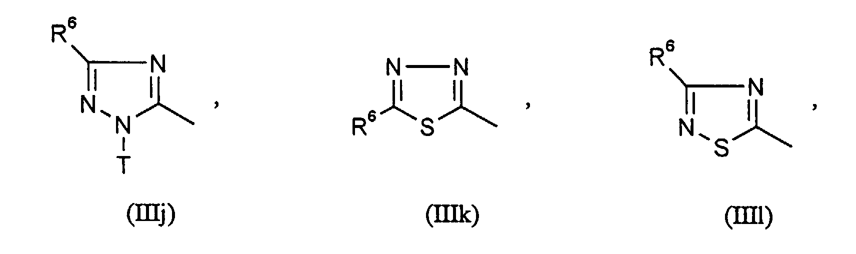 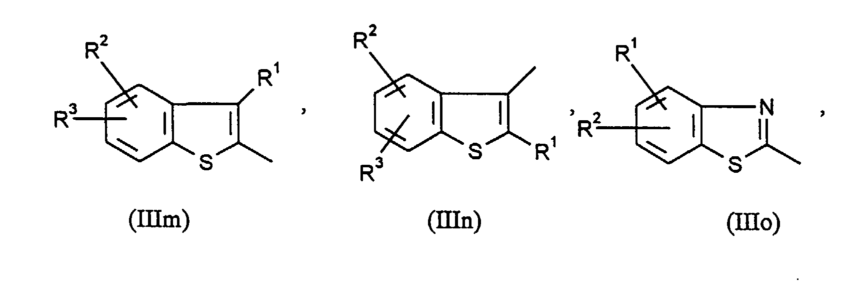 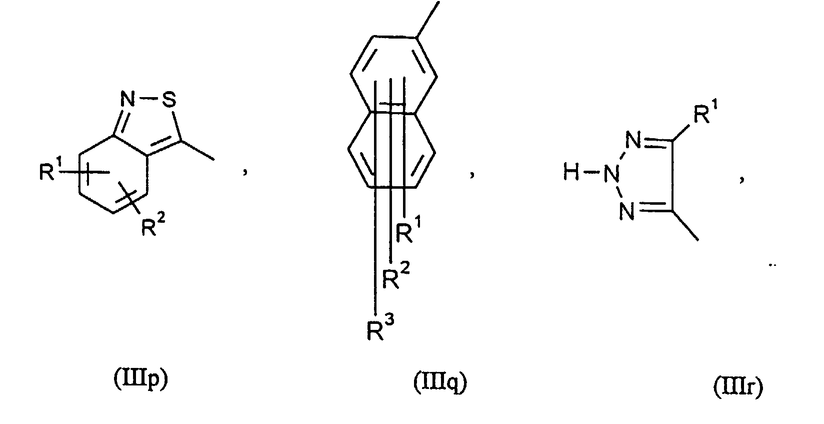 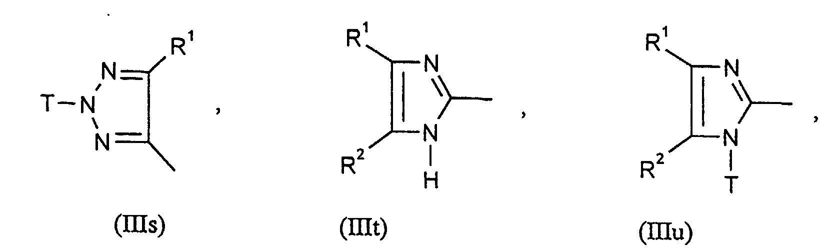
- R¹, R², R³, R⁴, R⁵ und R⁶: unabhängig voneinander
H, T, F, Cl, Br, J, -CN, -NO₂, -CH=O, -COT, -CO₂T¹, -CONH₂, -CONHT, CONT₂, -CF₃, -SO₂NH₂, -SO₂NHT, -SO₂NT₂, -SOT, -SO₂T, -SO₃T, -OT, -OH, -OCOT, -OCO₂T, -OSO₂T, -NH₂, -NHT, -NT₂, 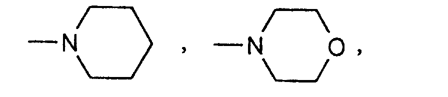 -NHCOH, -NHCOT, -NHSO₂T oder -COCO₂T bedeuten,
wobei
- R³: zusätzlich -N=N-T⁴ oder -N=N-T⁵ bedeuten kann, und
- R⁶: zusätzlich ST bedeuten kann.

Unter Dispersionfarbstoffen der Formel (I) sind solche besonders bevorzugt, worin
- A¹: H, T¹, T⁴ oder -CF₃,
- A²: H, -CN, -CO₂T¹, -CONHT¹ oder -CF₃,
- A³ und A⁴: unabhängig voneinander -CN, -CO₂T¹, -CONHT¹, -CF₃, -CHO, -COT, -SO₂T, -NO₂ , -T⁴ oder -T⁵ bedeuten,
wobei A³ und A⁴ nicht gleichzeitig für T⁴ oder T⁵ stehen,
- A³ und A⁴: zusammen mit dem C-Atom, an das sie gebunden sind, eine cyclische methylenaktive Verbindung der Formel (IIa), (IId) oder (IIh) bilden, wobei die Reste der cyclischen methylenaktiven Verbindung in Form von 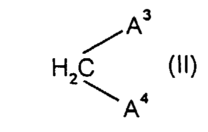 angegeben sind,
- A⁵: H, T¹, T², T⁴ oder
- A⁴ und A⁵: zusammen mit den dazwischenliegenden Atomen einen annellierten Ring der Formel 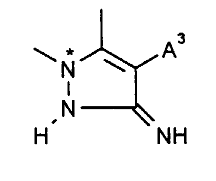 bilden,
wobei das mit * markierte N-Atom dem Pyridon-Stickstoff entspricht,
- D: einen Rest der Formel 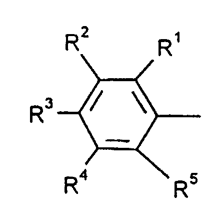 bedeutet, worin
- R¹ und R⁵: unabhängig voneinander für H, CF₃, Cl, Br, -CN, -NO₂, -CO₂T¹, T¹, T⁵, -SO₂T¹, -SO₂T⁴, -OT¹, -OT², -OT⁴, -OCOT¹, -OCOT⁴, -OSO₂T¹ oder -OSO₂T⁴ stehen,
- R² und R⁴: unabhängig voneinander H, Cl, Br, -NO₂, -CF₃, T¹, -OT¹, -OT², -OT⁴, -OCOT¹, -OCOT⁴, -OSO₂T¹ oder -OSO₂T⁴ bedeuten,
- R³: für H, Cl, Br, -CN, -NO₂, -CF₃, -CO₂T¹, T¹, T⁵, -OT¹, -OT², -OT⁴, -OCOT¹, -OCOT⁴, -SO₃T¹ oder -SO₂T⁴ steht,
- T¹: für C₁-C₈-Alkyl steht, das gegebenenfalls substituiert ist durch C₁-C₈-Alkoxy, -C₁-C₈-Alkoxy-C₂-C₅-alkoxy, -OCOT¹, -CO₂T¹, Cl, Br, -CN oder T⁴,
- T²: für C₂-C₈-Alkenyl steht, das gegebenenfalls substituiert ist durch -C₁-C₈-Alkoxy, -C₁-C₈-Alkoxy-C₂-C₅-alkoxy, -CN, -CO₂T¹, Cl oder Br,
- T⁴: für Phenyl steht, das gegebenenfalls substituiert ist durch Cl, Br, T¹, OT¹, -CF₃, -NO₂, -CN oder -CO₂T¹,
- T⁵: Oxazol, Phenyloxazol, Benzoxazol, Thiazol, Benzthiazol, Thiadiazol oder Thiophen bedeutet, das gegebenenfalls substituiert ist durch Cl, Br, T¹, -NO₂ und/oder -CO₂T¹ und
- X: O bedeutet.

Unter den Dispersionsfarbstoffen der Formel (I) sind solche ganz besonders bevorzugt, worin
- A¹: für H, T¹ oder -CF₃ steht,
- A²: H, -CN oder -CO₂T¹ bedeutet,
- A³: für -CN oder -CO₂T¹ steht,
- A⁴: A³ bedeutet, oder
- A³ und A⁴: zusammen mit dem C-Atom, an das sie gebunden sind, eine cyclische methylenaktive Verbindung der Formel (IIa) oder (IId) bilden, wobei die Reste der cyclischen methylenaktiven Verbindung in Form von 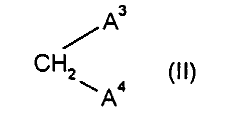 angegeben sind,
- A⁵: für H, T¹ oder T² steht,
- D: für einen Rest der Formel 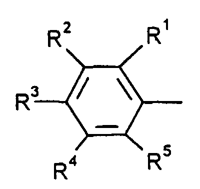 steht, worin
- R¹ und R⁵: unabhängig voneinander H, Cl, Br, -CN, -NO₂, -CO₂T¹, T¹, -OT¹ oder -OT⁴ bedeuten,
- R² und R⁴: unabhängig voneinander H, Cl, Br, -NO₂, T¹ oder -OT¹ bedeuten,
- R³: für H, Cl, Br, -CN, -NO₂, T¹, -CO₂T¹ oder -OT¹ steht,
- T¹: für C₁-C₆-Alkyl oder C₁-C₄-T⁴ steht, gegebenenfalls substituiert durch C₁-C₆-Alkoxy, -C₁-C₄-Alkoxy-C₂-C₅-alkoxy, -CO₂T¹,
- T²: gegebenenfalls durch Cl und/oder Br substituiertes C₂-C₆-Alkenyl bedeutet,
- T⁴: für Phenyl steht, das gegebenenfalls substituiert ist durch Cl, Br, -NO₂ und/oder -CO₂T¹ und
- X: O bedeutet.

Die erfindungsgemäßen Dispersionsfarbstoffe der Formel I besitzen eine gute Lichtechtheit, hohe Farbstärke sowie ein gutes Ziehvermögen auf Polyester und färben Polyester in brillanten Tönen.

### Kationische Farbstoffe

Unter den kationischen Farbstoffen der Formel (I) sind solche bevorzugt, worin A¹ bis A⁵ und X die für die Dispersionsfarbstoffe der Formel (I) genannten Bedeutungen besitzen und
- D: für einen Rest der Formel (IIIa) bis (IIIu), bevorzugt (IIIa) steht,
wobei K^{⊕} an einen oder mehreren beliebigen Stellen der Reste D und/oder A¹ bis A⁵ gebunden ist,
- B^{⊖}: ein Anion bedeutet,
- l: für 1 oder 2 steht und m vorzugsweise 0 ist.

Als Anionen B^{⊖} sind farblose, organische und anorganische Anionen bevorzugt, beispielsweise Fluorid, Chlorid, Bromid, Iodid, Perchlorat, Tetrafluoroborat, Hydroxid, Hydrogensulfat, Sulfat, Dihydrogenphosphat, Hydrogenphosphat, Phosphat, Hydrogencarbonat, Carbonat, Methylsulfat, Ethylsulfat, Cyanat, Thiocyanat, Tri- und Tetrachlorozinkat, Tetrachloroferrat, Hexafluorsilikat und Anionen gesättigter oder ungesättigter aliphatischer, cycloaliphatischer, aromatischer oder heterocyclischer Carbon- und Sulfonsäuren wie Formiat, Acetat, Hydroxyacetat, Cyanacetat, Propionat, Hydroxypropionat, Oxalat, Citrat, Lactat, Tartrat, das Anion der Cyclohexancarbonsäure, Phenylacetat, Benzoat, das Anion der Nikotinsäure, Methansulfonat, Ethansulfonat, Benzolsulfonat, Chlorbenzolsulfonat, Toluolsulfonat. Wenn es sich um mehrwertige Anionen handelt, z.B. um Sulfat oder Oxalat, dann steht in Formel (I) B^{⊖} für ein Äquivalent solch eines mehrwertigen Anions.

Bevorzugte kationische Diazokomponenten D sind Reste D¹ der Formel 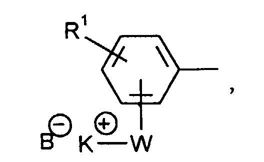 worin
- R¹, K^{⊕} und B^{⊖}: die vorgenannte Bedeutung haben und
- W: eine direkte Bindung oder ein Brückenglied bedeutet.

Als Brückenglieder W kommen beispielsweise die folgenden Reste in Betracht: 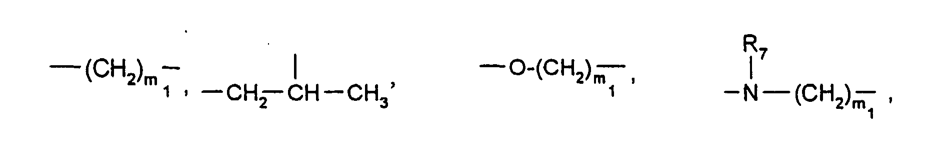 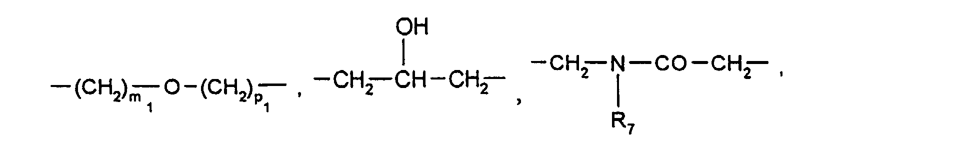 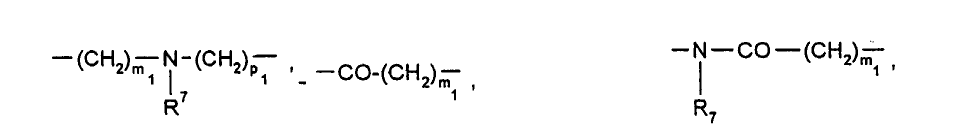 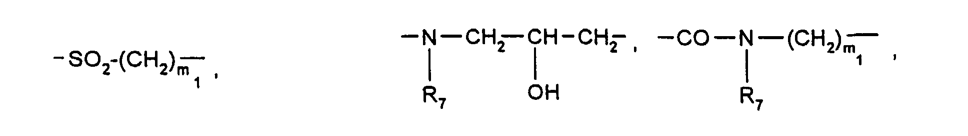 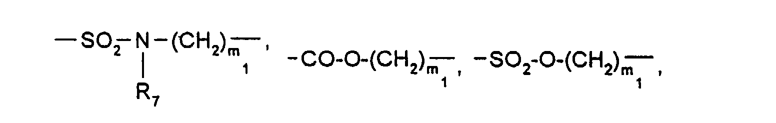 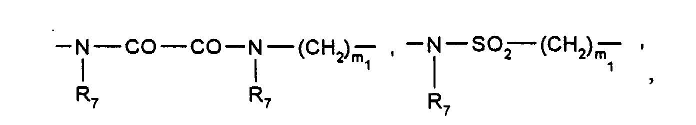 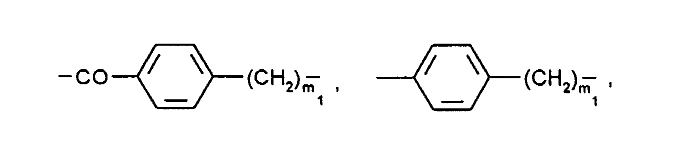 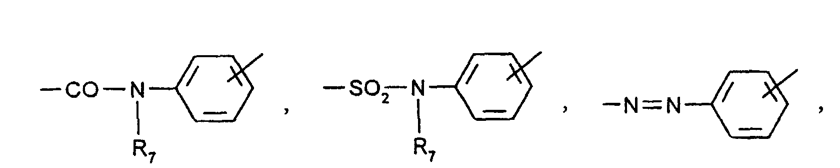 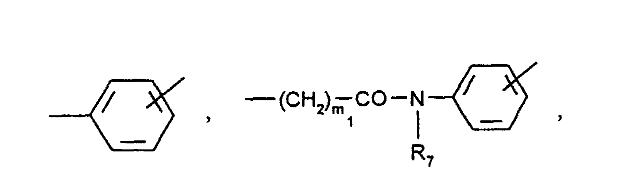 worin
- R₇: Wasserstoff, Methyl oder Ethyl bedeutet und
- m₁ und p₁: unabhängig voneinander für eine Zahl von 1 bis 4 stehen.

Geeignete Reste K^{⊕} sind beispielsweise:

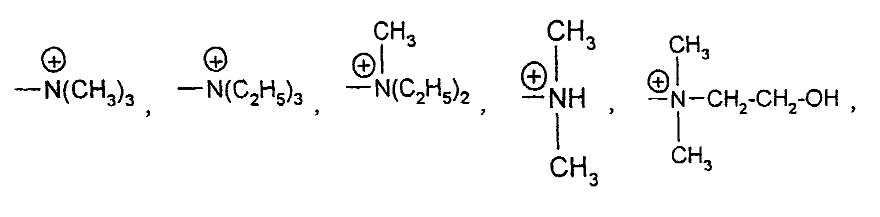

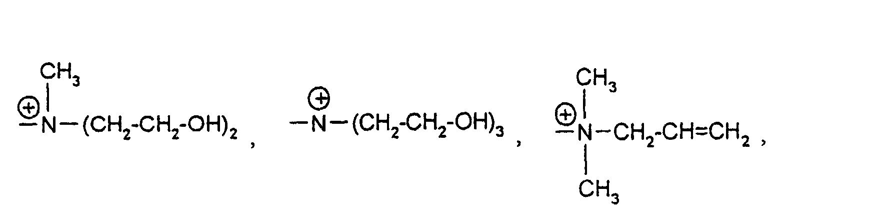

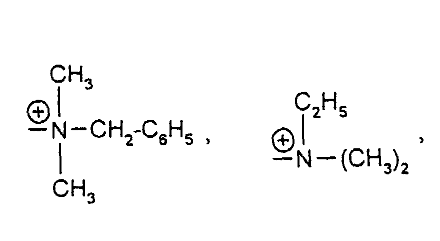

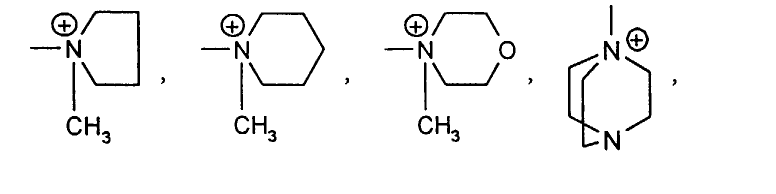

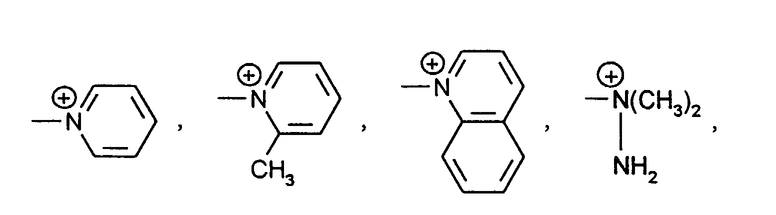

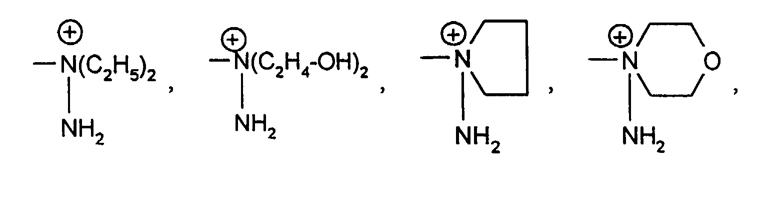

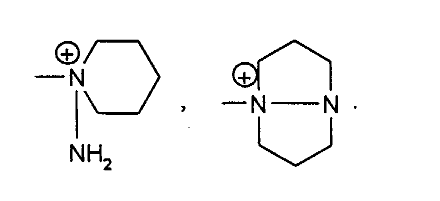

### Säurefarbstoffe

Unter den Säurefarbstoffen der Formel (I) sind solche bevorzugt, worin D, A¹ bis A⁵ und X die für die Dispersionsfarbstoffe der Formel (I) genannten Bedeutungen besitzen, wobei an mindestens einem dieser Reste mindestens eine SO₃H-Gruppe und/oder eine COOH-Gruppe gebunden ist. Besonders bevorzugte Säurefarbstoffe der Formel (I) sind solche, worin m = 1 bis 4 bedeutet.

Sulfonsäuregruppen-haltige Reste D sind beispielsweise Reste D², worin D² einen gegebenenfalls ein- bis viermal durch C₁-C₄-Alkyl, C₁-C₄-Alkoxy, Trifluormethyl, C₁-C₄-Alkylcarbonylamino, Benzoylamino, C₁-C₄-Alkylsulfonylamino, Benzolsulfonylamino, Cyano, Halogen, Nitro, C₁-C₄-Alkylsulfonyl, Phenylsulfonyl, Tolylsulfonyl, Chlorphenylsulfonyl, Carboxyl, C₁-C₄-Alkoxycarbonyl, Aminocarbonyl, Aminosulfonyl, N-mono- oder di-C₁-C₄-alkylsubstituiertes Aminocarbonyl bzw. -sulfonyl, oder einen Benzthiazolyl substituierten Phenyl-, Phenylazophenyl- oder Naphthylrest bedeutet, der 1 oder 2 SO₃H, OSO₃H oder Phenyl -SO₂-NH-SO₂-Gruppen trägt.

Geeignete Reste D² entsprechen in Form ihrer Diazokomponenten bspw. den folgenden:

1-Aminobenzol-2-, -3- oder 4-sulfonsäure, 1-Aminobenzol-2,4- oder -2,5-disulfonsäure, 1-Amino-2-methylbenzol-4-sulfonsäure, 1-Amino-3-methylbenzol-4-sulfonsäure, 1-Amino-4-methylbenzol-2- oder -3-sulfonsäure, 2 -Nitranilin-4-sulfonsäure, 4-Nitranilin-2-sulfonsäure, 2-Chloranilin-4- oder -5-sulfonsäure, 3-Chloranilin-6-sulfonsäure, 4-Chloranilin-2-sulfonsäure, 1-Amino-3,4-dichlorbenzol-6-sulfonsäure, 1-Amino-2,5-dichlorbenzol4-sulfonsäure, 1-Amino-4-methyl-5-chlorbenzol-2-sulfonsäure, 1-Amino-3-methyl-4-chlorbenzol-6-sulfonsäure, 2-Amino-4-sulfobenzoesäure, 1-Amino-4-acetaminobenzol-2-sulfonsäure, 1-Amino-5-acetaminobenzol-2-sulfonsäure, 1-Amino-2-methoxy-4-nitrobenzol-5-sulfonsäure, 1-Aminonaphthalin-2- oder 4-sulfonsäure, 2-Aminonaphthalin-1-sulfonsäure, sowie die Diazokomponenten der Formeln

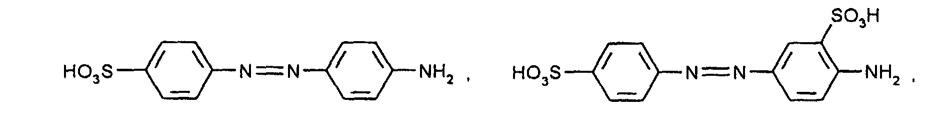

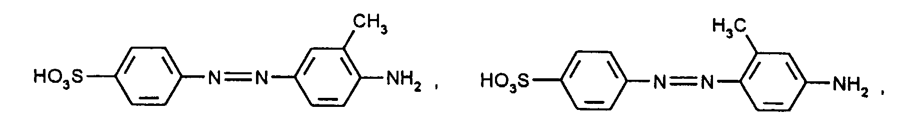

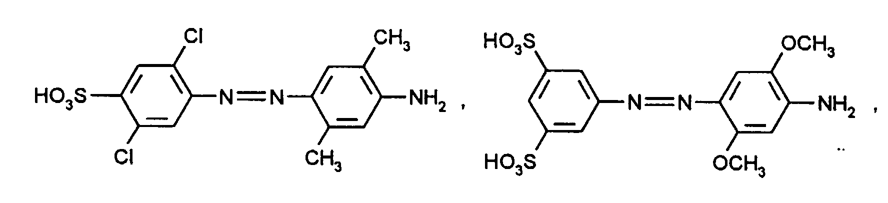

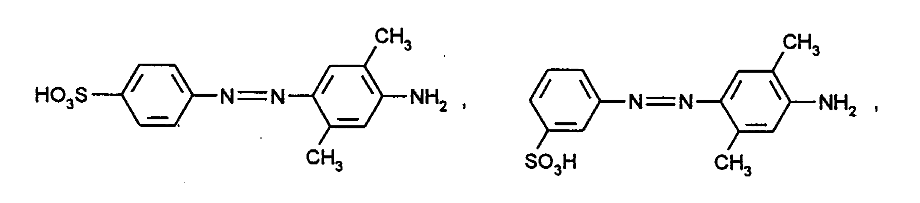

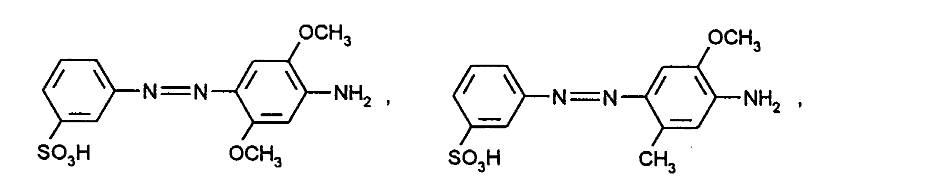

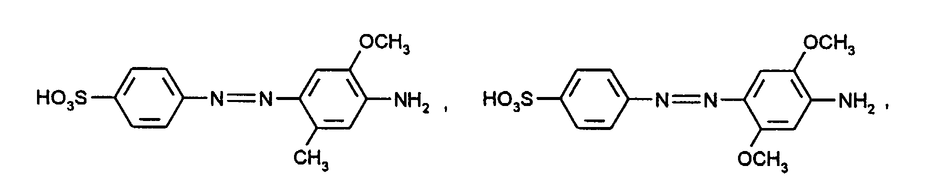

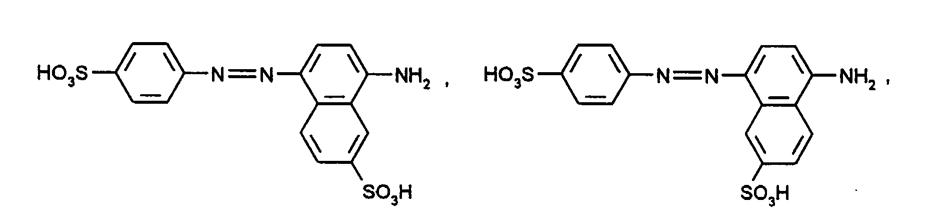

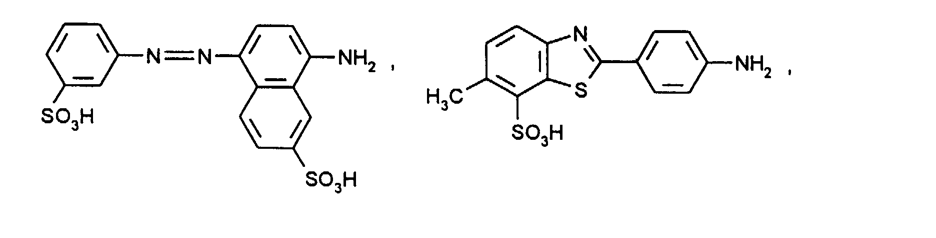

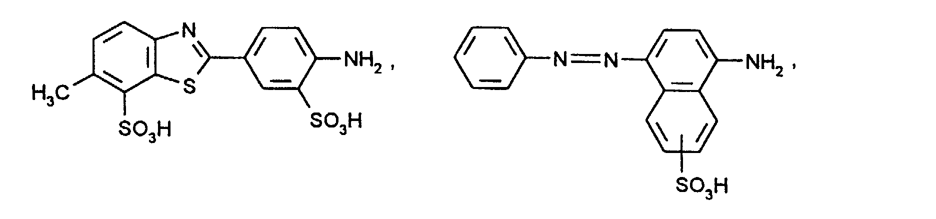

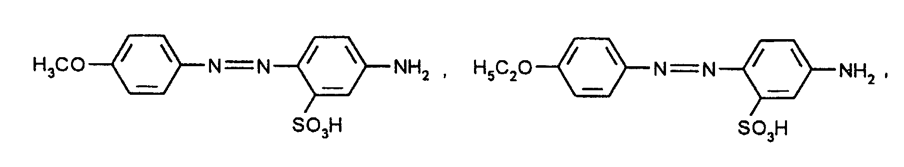

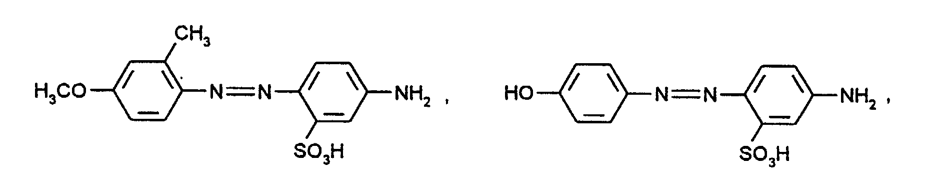

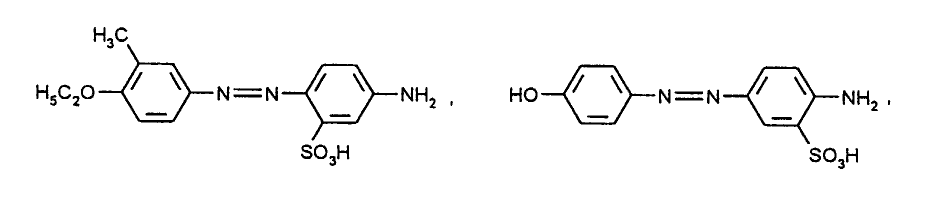

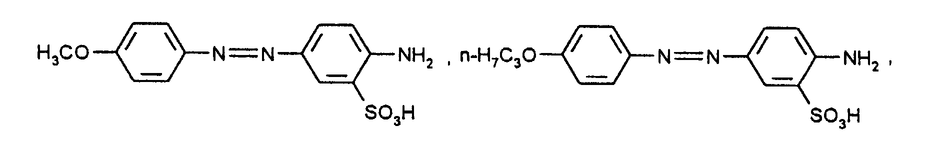

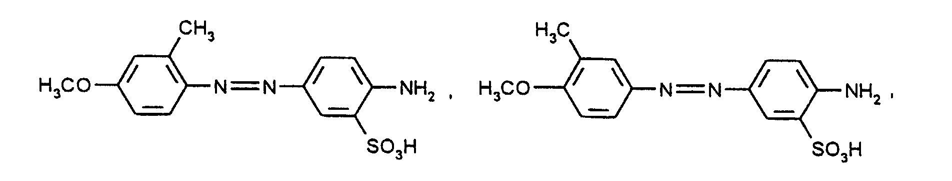

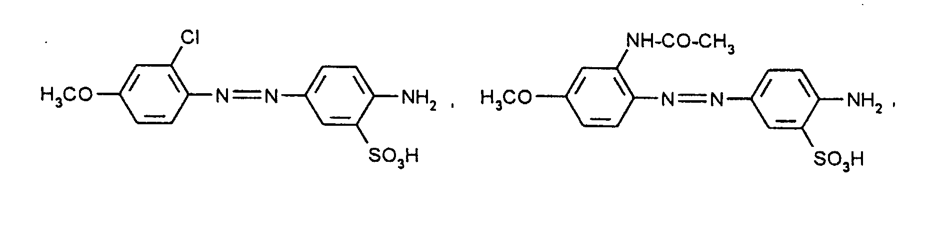

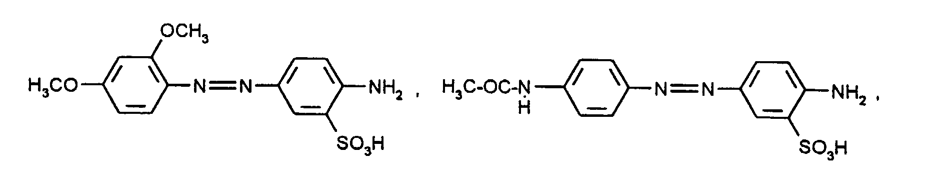

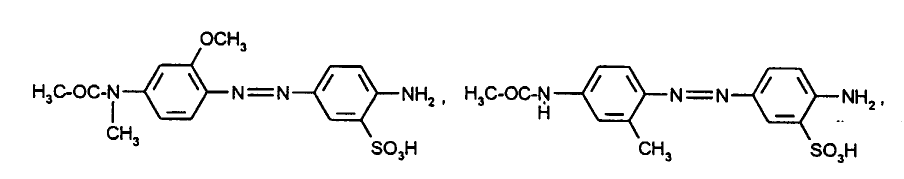

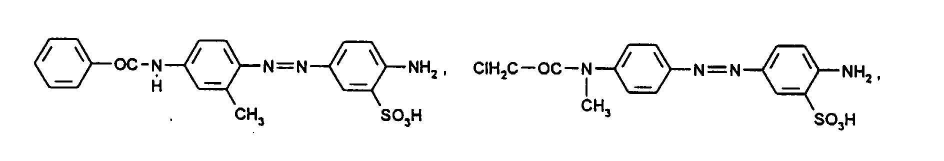

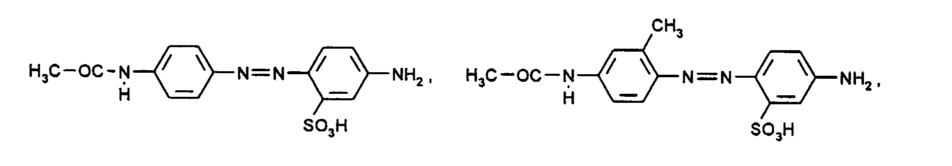

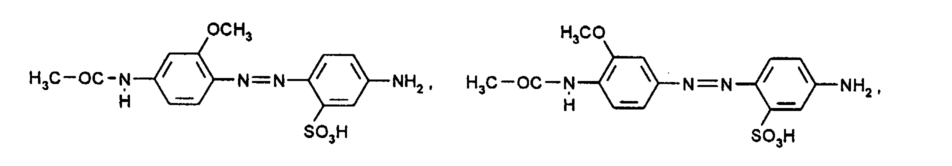

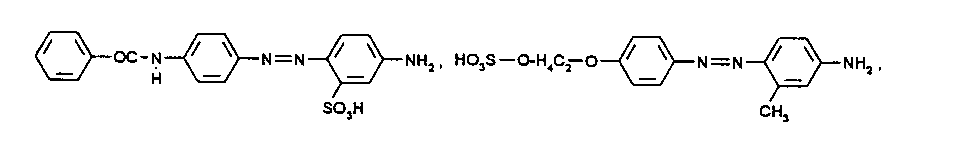

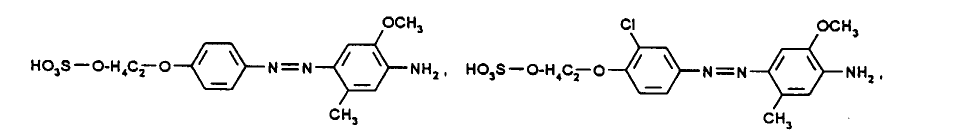

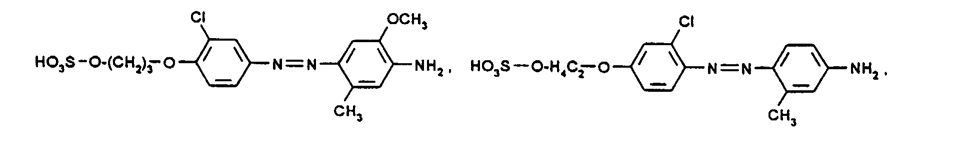

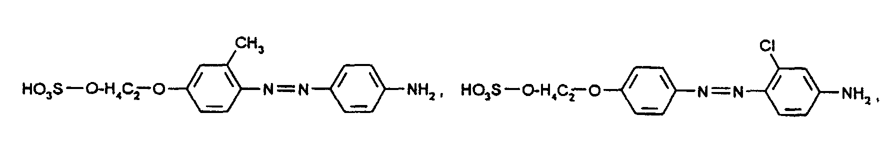

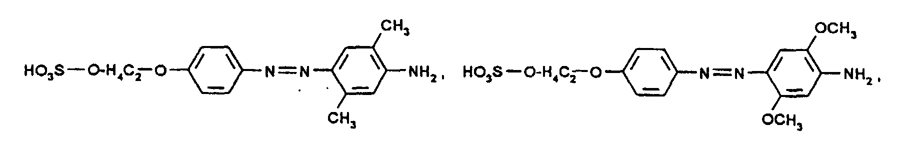

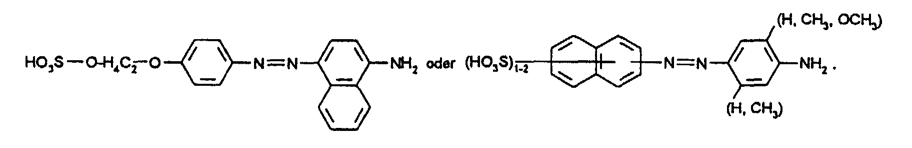

### Reaktivfarbstoffe

Unter den Reaktivfarbstoffen der Formel (I) sind solche bevorzugt, worin D, A¹ bis A⁵ und X die für die Dispersionsfarbstoffen der Formel (I) genannten Bedeutungen besitzen, wobei vorzugsweise an mindestens einem dieser Reste eine SO₃H-Gruppe und/oder eine COOH-Gruppe gebunden ist, und worin ein oder mehrere gleiche oder verschiedene faserreaktive Reste Z an mindestens einem der genannten Reste gebunden sind.

Bevorzugt sind Reaktivfarbstoffe der Formel (I) mit n = 1 bis 3.

Bevorzugte faserreaktive Reste sind beispielsweise solche der Formel

-CH=CH₂ oder -CH₂CH₂-V,

worin
- V =: OH oder alkalisch abspaltbarer Rest, z.B. OSO₃H, SSO₃H, OCOCH₃, OPO₃H₂, OSO₂CH₃, SCN, NHSO₂CH₃, Cl, Br, F, OCOC₆H₅, OSO₂-C₆H₄, [N(CH₃)₃]^{⊕}Anion^{⊖} oder gegebenenfalls substituierter Pyridinium-Rest (Substituenten am Pyridinium-Rest sind insbesondere gegebenenfalls substituiertes C₁-C₄-Alkyl, COOH, SO₃H, CN, Carbonamid), (Anion = z.B. Cl , HSO₄^{⊖}, HCO₃^{⊖} usw),
oder heterocyclische faserreaktive Reste.

Geeignete faserreaktive Reste Z, d.h. solche die mit den OH- oder NH-Gruppen der Faser unter Färbebedingungen unter Ausbildung kovalenter Bindungen reagieren, sind vorzugsweise solche, die mindestens einen reaktiven Substituenten an einen 5- oder 6-gliedrigen aromatisch-heterocyclischen Ring gebunden enthalten, bspw. an einen Monoazin-, Diazin- oder Triazinring, insbesondere einen Pyridin-, Pyrimidin-, Pyridazin-, Pyrazin-, Thiazin-, Oxazin- oder asymmetrischen oder symmetrischen Triazinring, oder an ein derartiges Ringsystem, welches einen oder mehrere ankondensierte aromatisch-carbocyclische Ringe aufweist, bspw. ein Chinolin-, Phthalazin-, Cinnolin-, Chinazolin-, Chinoxalin-, Acridin-, Phenazin-oder Phenanthridin-Ring-System, und die an kein weiteres Chromophor gebunden sind.

Unter den reaktiven Substituenten am Heterocyclus sind beispielsweise zu erwähnen Halogen (Cl, Br oder F), Ammonium einschließlich Hydrazinium, Pyridinium, Picolinium, Carboxypyridinium, Sulfonium, Sulfonyl, Azido-(N₃), Rhodanido, Thiolether, Oxyether, Sulfinsäure und Sulfonsäure.

Im einzelnen sind beispielsweise folgende Reste für Z zu nennen:
2,4-Difluortriazinyl-6-, 2,4-Dichlortriazinyl-6-, Monohalogen-sym.-triazinylreste, insbesondere Monochlor- und Monofluortriazinylreste, die durch Alkyl, Aryl, Amino, Monoalkylamino, Dialkylamino, Aralkylamino, Arylamino, Morpholino, Piperidino, Pyrrolidino, Piperazino, Alkoxy, Aryloxy, Alkylthio, Arylthio substituiert sind, wobei Alkyl vorzugsweise gegebenenfalls substituiertes C₁-C₄-Alkyl, Aralkyl vorzugsweise gegebenenfalls substituiertes Phenyl-C₁-C₄-alkyl und Aryl vorzugsweise gegebenenfalls substituiertes Phenyl oder Naphthyl bedeutet und wobei bevorzugte Substituenten für Alkyl Halogen, Hydroxy, Cyan, Dialkylamino, Morpholino, C₁-C₄-Alkoxy, Carboxy, Sulfo oder Sulfato sind und für Phenyl und Naphthyl, Sulfo, C₁-C₄-Alkyl, C₁-C₄-Alkoxy, Carboxy, Halogen, Acylamino, Hydroxy und Amino. Weiter zu nennen sind 2-Amino-4-fluor-triazinyl-6, 2-Methylamino-4-fluortriazinyl-6, 2-Ethylamino-4-fluortriazinyl-6, 2-Isopropylamino-4-fluor-triazinyl-6, 2-Dimethylamino-4-fluortriazinyl-6, 2-Diethylamino-4-fluor-triazinyl-6, 2-β-Methoxy-ethylamino-4-fluor-triazinyl-6, 2-β-Hydroxyethylamino-4-fluor-triazinyl-6, 2-Di-(β-hydroxyethylamino)-4-fluor-triazinyl-6, 2 2-Carboxymethylamino-4-fluor-triazinyl-6, 2-Di-(carboxymethylamino)-4-fluortriazinyl-6, 2-Sulfomethyl-methylamino-4-fluor-triazinyl-6, 2-β-Cyanethylamino-4-fluor-triazinyl-6, 2-Benzylamino-4-fluor-triazinyl-6, 2-β-Phenylethylamino-4-fluor-triazinyl-6, 2-Benzyl-methylamino-4-fluor-triazinyl-6, 2-(4'-Sulfobenzyl)-amino-4-fluor-triazinyl-6, 2-Cydohexylamino-4-fluor-triazinyl-6, 2-(o-, m-, p-Methylphenyl)-amino-4-fluor-triazinyl-6, 2-(o-, m-, p-Sulfophenyl)-amino-4-fluor-triazinyl-6, 2-(2',5'-Disulfophenyl)-amino-4-fluor-triazinyl-6, 2-(o-, m-, p-Chlorphenyl)-amino-4-fluor-triazinyl-6, 2-(o-, m-, p-Methoxyphenyl)-amino-4-fluor-triazinyl-6, 2-(2'-Methyl-4'-sulfophenyl)-amino-4-fluor-triazinyl-6, 2-(2'-Methyl-5'-sulfophenyl)-amino-4-fluor-triazinyl-6, 2-(2'-Chlor-4'-sulfophenyl)-amino-4-fluor-triazinyl-6, 2-(2'-Chlor-5'-sulfophenyl)-amino-4-fluor-triazinyl-6, 2-(2'-Methoxy-4'-sulfophenyl)-amino-4-fluor-triazinyl-6, 2-(o-, m-, p-Carboxyphenyl)-amino4-fluor-triazinyl-6, 2-(2',4'-Disulfophenyl)-amino-4-fluor-triazinyl-6, 2-(3',5'-Disulfophenyl)-amino-4-fluor-triazinyl-6, 2-(2'-Carboxy-4'-sulfophenyl)-amino-4-fluor-triazinyl-6, 2-(2'-Carboxy-5'-sulfophenyl)-amino-4-fluor-triazinyl-6, 2-(6'-Sulfonaphthyl-(2'))-amino-4-fluor-triazinyl-6, 2-(4',8'-Disulfonaphthyl-(2'))-amino-4-fluor-triazinyl-6, 2-(6',8'-Disulfonaphthyl-(2'))-amino-4-fluor-triazinyl-6, 2-(N-Methyl-N-phenyl)-amino-4-fluor-triazinyl-6, 2-(N-Ethyl-N-phenyl)-amino-4-fluor-triazinyl-6, 2-(N-β-Hydroxyethyl-N-phenyl)-amino-4-fluor-triazinyl-6, 2-(N-iso-Propyl-N-phenyl)-amino-4-fluor-triazinyl-6, 2-Morpholino-4-fluor-triazinyl-6, 2-Piperidino-4-fluor-triazinyl-6, 2-(4',6', 8'-Trisulfonaphthyl-(2'))-amino-4-fluor-triazinyl-6, 2-(3',6',8'-Trisulfonaphthyl-(2'))-amino-4-fluor-triazinyl-6, 2-(3',6'-Disulfonaphthyl-(1'))-amino-4-fluor-triazinyl-6, N-Methyl-N-(2,4-dichlortriazinyl-6)-carbamyl-, N-Methyl-N-(2-methylamino-4-chlortriazinyl-6)-carbamyl-, N-Methyl-N-(2-dimethylamino-4-chlortriazinyl-6)-carbamyl-, N-Methyl-bzw. N-Ethyl-N-(2,4-dichlortriazinyl-6)-aminoacetyl-, 2-Methoxy-4-fluor-triazinyl-6, 2-Ethoxy-4-fluor-triazinyl-6, 2-Phenoxy-4-fluor-triazinyl-6, 2-(o-, m- oder p-Sulfophenoxy)-4-fluor-triazinyl-6, 2-(o-, m-oder p-Methyl- oder -Methoxy-phenoxy)-4-fluor-triazinyl-6, 2-β-Hydroxyethylmercapto-4-fluor-triazinyl-6, 2-Phenylmercapto-4-fluor-triazinyl-6, 2-(4'-Methylphenyl)-mercapto-4-fluortriafinyl-6, 2-(2',4'-Dinitrophenyl)-mercapto-2-Methyl-4-fluor-triazinyl-6, 2-Methyl-4-fluor-triazinyl-6, 2-Phenyl-4-fluor-triazinyl-6 sowie die entsprechenden 4-Chlor- bzw. 4-Brom-triazinyl-Reste und die entsprechenden durch Halogenaustausch mit tertiären Basen wie Trimethylamin, Triethylamin, Dimethyl-β-hydroxyethylamin, Triethanolamin, N,N-Dimethylhydrazin, Pyridin, α-, β- oder γ-Picolin, Nicotinsäure oder Isonicotinsäure, Sulfinaten insbesondere Benzolsulfinsäure oder Hydrogensulfit erhältlichen Reste sowie Di- oder Trihalogenpyrimidinylreste, wie 2,4-Dichlorpyrimidinyl-6, 2,4,5-Trichlor-pyrimidinyl-6, 4,5-Dichlorpyrimidinyl-6-, 2,4-Difluorpyrimidinyl-6-, 4,5-Difluor-pyrimidinyl-6-, 4-Fluor-5-chlorpyrimidinyl-6-, 2,4-Difluor-5-chlorpyrimidinyl, sowie 2,3-Dichlorchinoxalin-5-carbonyl und 2,3-Dichlorchinoxalin-6-carbonyl.

Geeignete Diazokomponenten für die Reaktivfarbstoffe der Formel (I) sind unter anderem solche, worin D die oben angegebene Bedeutung besitzt, sowie für D² mit der dort genannten Bedeutung steht und ferner für reaktive Diazokomponenten D³ steht, worin D³ 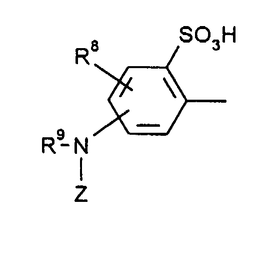 bedeutet, worin
- R⁸: die für R¹ genannten Bedeutungen annehmen kann und zusätzlich SO₃H, OSO₃H oder COOH bedeuten kann und
- R⁹: für H, T¹ oder T⁴ steht.

Die erfindungsgemäßen Farbstoffe, insbesondere Dispersionsfarbstoffe der Formel (I) können durch Kuppeln diazotierter Amine die als Amin der Formel (IV) entsprechen,

D - NH₂ (IV)

auf Kupplungskomponenten der Formel (V) 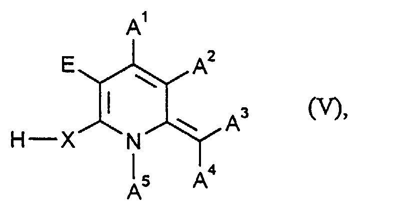 worin A¹ bis A⁵, D und X die vorgenannte Bedeutung haben und
- E: für einen durch elektrophile Substitution verdrängbaren Substituenten, wie z.B. H, -CO₂H, -CH₂OH, -SO₃H, -CH=O, -COT, 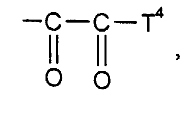 -CONH₂, -CONHT steht, hergestellt werden.

Ein derartiges Kupplungsverfahren unter Verdrängung eines Substituenten ist z.B. in GB 2 036 775 und JP 58 157 863 beschrieben.

Die Kupplung kann in wäßrigen und nichtwäßrigen Lösungsmitteln erfolgen. Unter den nichtwäßrigen Lösungsmitteln seien Alkohole wie Methanol, Ethanol, Propanol, Butanol, Pentanol etc., dipolare aprotische Lösungsmittel wie DMF, DMSO, NMP und mit Wasser nicht mischbare Lösungsmittel wie Toluol, Chlorbenzol genannt.

Die Kupplung wird vorzugsweise im stöchiometrischen Verhältnis durchgeführt, wobei es vorteilhaft und z.T. aus ökonomischen Gründen sinnvoll sein kann, die billigere Komponente in bis zu 30 % Überschuß einzusetzen.

Die Kupplung erfolgt bei Temperaturen zwischen -30 bis 100°C, bevorzugt sind Temperaturen von -10 bis 30°C, besonders bevorzugt Temperaturen von -5 bis 10°C.

Die Kupplung kann im sauren wie auch im alkalischen Milieu ausgeführt werden. Bevorzugt werden pH-Werte >0,5 und <14, besonders bevorzugt >1,0 und <12 und ganz besonders bevorzugt >3,0 und ≤ 11,0.

Weiterhin ist auch eine oxidative Kupplung von (het-)aromatischen Hydrazinen der Formel (VI)

D-NH-NH₂ (VI)

auf Kupplungskomponenten der Formel (V) möglich, dadurch gekennzeichnet, daß man VI in Gegenwart von V oxidativ kuppelt. Methoden der oxidativen Kupplung sind z.B. in Houben-Weyl "Methoden der organischen Chemie", Band 10/3, S. 360 f, sowie auch in EP 201 892 oder Chem. Express 1988, 3(7), 423-6, beschrieben.

Weiterhin bevorzugt ist ein Verfahren zur Herstellung von Farbstoffen der Formel (I), worin X gleich O bedeutet, dadurch gekennzeichnet, daß Verbindungen der Formel (VII), die in den tautomeren Formen der Formel (VIIa) und (VIIb) vorliegen können, 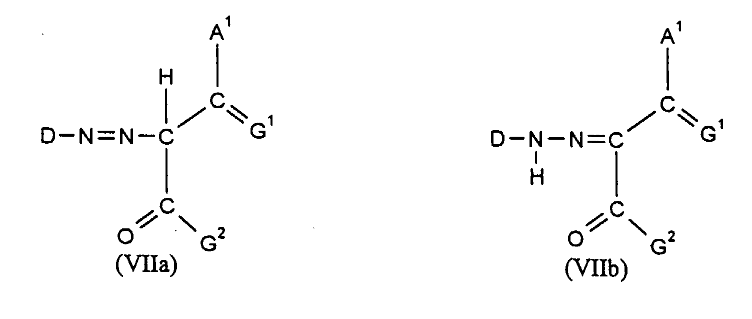 worin
- G¹: O, NH oder NT, vorzugsweise O bedeutet,
- G²: für -OT, -NH₂, oder -NHT, vorzugsweise für OT¹ steht,
- D und A¹: die vorgenannten Bedeutungen haben
mit Enaminen der Formel (VIII) 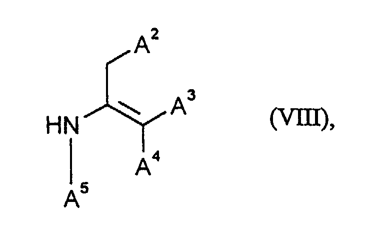 worin A² bis A⁵ die vorgenannten Bedeutungen haben, kondensiert werden.

Diese Kondensation wird vorzugsweise bei den Dispersionsfarbstoffe der Formel (I) vorzugsweise in organischen Lösungsmitteln, insbesondere dipolaren aprotischen Lösungsmitteln durchgeführt. Beispielsweise seien Alkohole wie Methanol, Ethanol und andererseits DMF, DMSO, NMP genannt.

Man führt die Kondensation bei Temperaturen von -10 bis 200°C, vorzugsweise von 0 bis 150°C, insbesondere bei 20 bis 130°C durch.

Vorzugsweise wird die Kondensation in Gegenwart von Basen wie z.B. sekundären und tertiären Aminen und Alkalialkoholaten durchgeführt. Hierbei variiert die Basenmenge zwischen katalytischen und fünffach-molaren Mengen. Vorzugsweise werden 1 bis 3 Mol Base eingesetzt. Geeignete Basen sind z.B. Triethylamin, Natriummethylat, Natriumethylat, Natriumbutylat, Natriumamylat, K₂CO₃, Na₂CO₃, DBU (Diazabicycloundecen), DBN (Diazabicyclononen).

Die Verbindungen der Formel (VII) sind teilweise aus DE-A-2.015.172 bekannt oder können nach bekannten Methoden hergestellt werden.

Die Enamine der Formel (VIII) sind größtenteils bekannt und/oder durch "Dimerisation" oder "Codimerisation" von Cyanessigsäurederivaten nach dem Schema 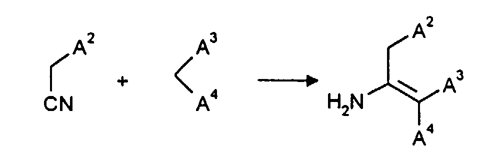 zugänglich; vergl. z.B. Liebigs Ann. Chem. 1987, 1131 bis 1132 und dort zitierte Literatur.

Bevorzugte Enamine der Formel (VIII) entsprechen den Formeln (VIIIa) bis (VIIIc).

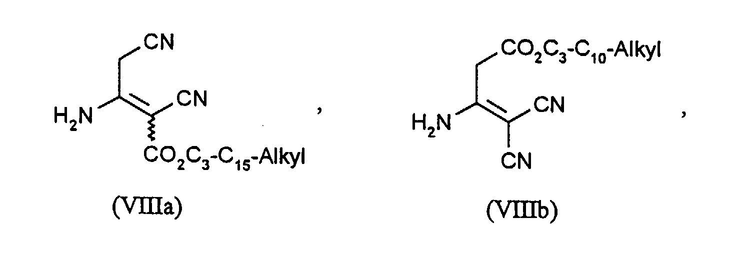

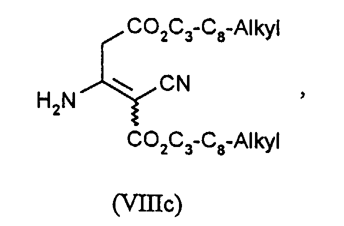

Eine weitere Darstellungsmethode für Enamine der Formel (VIII) ist die Umsetzung von Iminoestern mit methylenaktiven Verbindungen gemäß Liebigs Ann. Chem. 1986, 533 bis 544 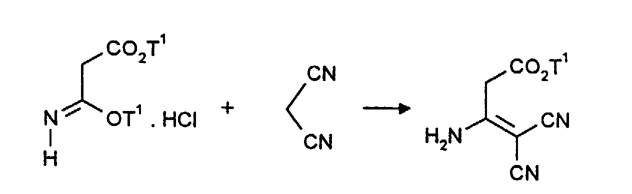 wobei T¹ die vorgenannte Bedeutung hat.

Verbindungen der Formel (V) sind teilweise aus AU 491 554, Aust. J. Chem. 29 (1976), 1039-50, und J. Am. Chem. Soc. 81, 2452 bekannt.

Die Erfindung betrifft weiterhin Verbindungen der Formel (V) 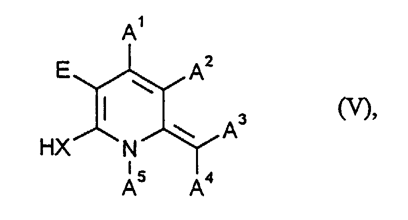 worin
- E und X: die oben genannten breitesten Bedeutungen haben,
- A¹ und A²: unabhängig voneinander H oder einen Rest der Formel T, -COH, -CO-T, -CO₂T, -CN, -CONH₂, -CONHT, -CONT₂, CF₃, -NO₂, -NO,-SO₂T, -OH, -OT, -OCOT, -OCO₂T, -OSO₂T, Cl, Br, J
bedeuten, wobei
- T: die Bedeutung von T¹, T², T³, T⁴ oder T⁵ annehmen kann, mit
- T¹ =: Alkyl, Cycloalkyl oder Aralkyl,
- T² =: Alkenyl,
- T³ =: Alkinyl,
- T⁴ =: Aryl,
- T⁵ =: Hetaryl,
A³ und A⁴ unabhängig voneinander einen elektronenziehenden Rest bedeuten, oder zusammen mit dem gemeinsamen C-Atom eine cyclische methylenaktive Verbindung bilden,
- A⁵: für H, einen Rest der Formel T, -OT¹, -NH₂, -NHT, -NT₂, -NHCOH, -NHCOT, -N=CH-T, -N=CT₂ oder NHSO₂T steht oder
- A¹ und A²: oder
- A² und A³: zusammen mit den jeweils dazwischenliegenden Atomen einen ungesättigten, gegebenenfalls substituierten 5- oder 6-gliedrigen Carbo- oder Heterocyclus bilden, wobei bei einer Ringbildung unter Beteiligung eines der Reste A³ oder A⁴ der jeweils andere einen elektronenziehenden Rest bedeutet und wobei die Reste A¹ bis A⁵ gegebenenfalls ein oder mehrere SO₃H, COOH oder K^{⊕}B^{⊖}-Gruppen tragen, worin K^{⊕} und B^{⊖} die obige Bedeutung haben.

Bevorzugte Verbindungen der Formel (V) sind solche, worin
- A¹: H, T¹, T⁴, CF₃,
- A²: H, -CO₂T¹, -CN, -CONH₂, -CONHT, -CO₂T², -CONT¹₂,
- A³: -CO₂T¹, -CN, -CONH₂, -CONHT, -CONT¹₂, -CO₂T²,
- A⁴: -CO₂T¹, -CN, -CONH₂, -CONHT, -CONT¹₂, -CO₂T²,
- A⁵: H, T¹ oder T² oder
- A³ und A⁴: zusammen mit dem gemeinsamen C-Atom eine cyclische methylenaktive Verbindung der Formel (IIa) oder (IId) und
- E: H bedeuten.

Besonders bevorzugt sind Verbindungen der Formel (V), worin
- A¹: H, CH₃, C₆H₅, CF₃,
- A²: H, CO₂C₁-C₁₀-Alkyl, -CO₂C₂-C₁₀-Alkenyl,
- A³ und A⁴: unabhängig voneinander -CO₂C₁-C₁₀-Alkyl, -CO₂C₂-C₁₀-Alkenyl, -CN,
- A⁵: H, C₁-C₁₀-Alkyl und
- E: H bedeuten.

Die Erfindung betrifft weiterhin ein Verfahren zur Herstellung der Verbindungen der Formel (V) in Form der Verbindung Va bzw. Vb, worin X für O oder NH steht, dadurch gekennzeichnet, daß man Essigsäurederivate der Formel IXa bzw. IXb mit Enaminen der Formel (VIII): 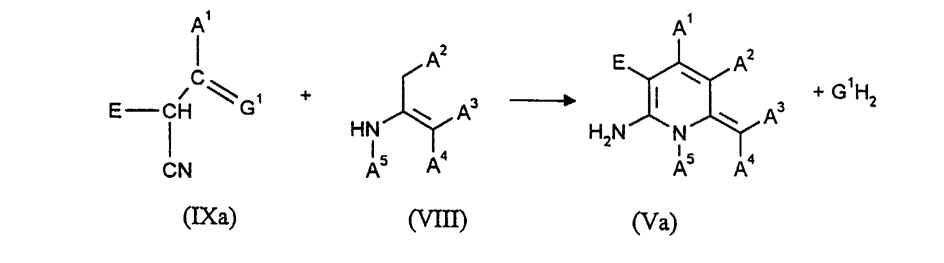 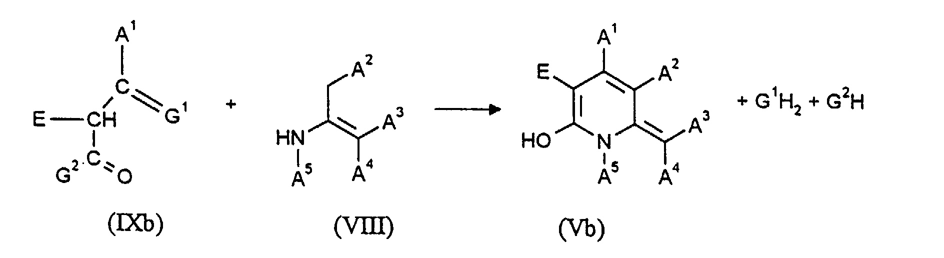 umsetzt,
worin
- A¹ bis A⁵, E, G¹ und X: die vorgenannte breiteste Bedeutung haben ,
worin
- A⁵: vorzugsweise für H steht, und
- G²: die vorgenannte Bedeutung hat.

Vorzugsweise steht
- G¹: für O,
- E: für H und
- G²: für OT¹.

Die Umsetzung wird vorzugsweise in organischen Lösungsmitteln durchgeführt. Geeignet sind Alkohole, dipolare aprotische Lösungsmittel wie DMF, mit Wasser nicht mischbare Lösungsmittel und Ester, vor allem Acylessigester wie z.B. Acetessigsäuremethyl- oder -ethylester.

Mit Wasser nicht mischbare Lösungsmittel bieten den Vorteil, daß das bei der Reaktion im Falle von G¹ = O entstehende Wasser azeotrop mit ihnen aus dem Reaktionsgemisch entfernt werden kann.

Eine besonders bevorzugte Ausführungsform, speziell bei weniger reaktiven Enaminen der Formel (VIII), d.h. worin A² für -CO₂T steht, besteht darin, mit einem Überschuß an (IX) als Lösungsmittel - ohne die Verwendung weiterer Cosolventien - zu arbeiten. Dieser Überschuß an (IX) gegenüber (VIII) beträgt vorzugsweise 5 bis 500 Gew.-%.

Die Reaktion wird vorzugsweise bei Temperaturen von 0 bis 200°C, vorzugsweise 20 bis 150°C, besonders bevorzugt bei 70 bis 120°C, durchgeführt.

Durch Katalysatoren läßt sich die Reaktion beschleunigen. Geeignete Katalysatoren sind Säuren, wie z.B. Eisessig und Säure-Base-Paare, wie β-Alanin-Eisessig, Ammoniumacetat, Piperidin-Eisessig, Morpholin-Eisessig.

Ferner eignen sich anorganische und organische Basen als Katalysatoren oder Reaktionspartner. Genannt seien Natriumhydroxid, Natriumethylat, Kaliumethylat, Triethylamin, Piperidin, Morpholin, Ethanolamin, wie sie auch bei Kondensationen von CH-aciden Verbindungen mit Acetylaceton und ähnlichen eingesetzt werden.

(Vergleiche: Monatshefte für Chemie 95, 1201, 1473 (1964) und J. Chem. Eng. Data 29, 101).

Besonders geeignet, speziell bei weniger reaktiven Enaminen der Formel (VIII), worin A² für -CO₂T steht, sind sterisch gehinderte starke Basen wie z.B. Kalium-tert.butylat und sterisch gehinderte N-Basen wie z.B. Diazabicycloundecen (DBU) und Diazabicyclononen (DBN).

Die Reaktion kann bei Normaldruck wie auch unter erhöhtem oder vermindertem Druck ausgeführt werden. Bevorzugt speziell bei weniger reaktiven Enaminen der Formel (VIII) ist das Arbeiten unter vermindertem Druck bei Drücken < 200 mbar, insbesondere <100 mbar um die aus der Kondensation mit dem bevorzugten Essigsäurederivat der Formel (IX), dem Acetessigsäurealkylester, entstehenden Spaltprodukte Wasser und Alkohol rasch aus der Reaktionsmischung zu entfernen.

Im Gegensatz zu den sauren Katalysatoren und Säure-Base-Paaren werden Basen vorzugsweise im stöchiometrischen Verhältnis, bzw. im Überschuß eingesetzt. Der Überschuß kann 1 bis 500 % betragen.

Ebenfalls bevorzugt ist ein Verfahren zur Herstellung von Verbindungen der Formel V, die der Formel X entsprechen, das dadurch gekennzeichnet ist, daß man Trifluoracetessigester der Formel (XI), der eine besonderen Ausführungsform der Essigsäurederivate der Formel (IX) darstellt, mit dimeren Cyanessigsäureestern der Formel (XII), die eine besondere Ausführungsform der Enamine der Formel (VIII) darstellen, kondensiert, 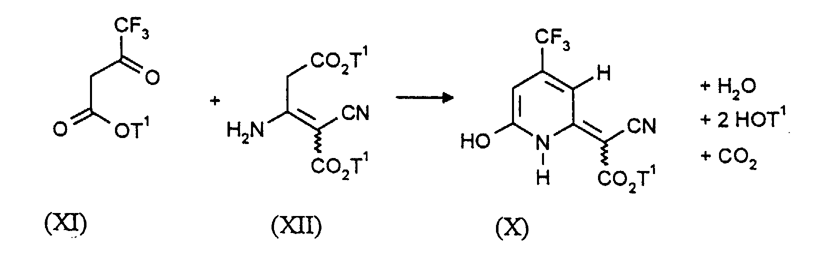 wobei T¹ die vorgenannte breiteste Bedeutung hat. Die Reaktionsbedingungen sind die gleichen, wie vorstehend bei weniger reaktiven Enaminen beschrieben.

Die Erfindung betrifft ebenfalls ein Verfahren zur Herstellung von Verbindungen der Formel (V), worin A⁵ für T¹, T² oder T³ steht, das dadurch gekennzeichnet ist, daß man Pyridiniumsalze der Formel (XIII) 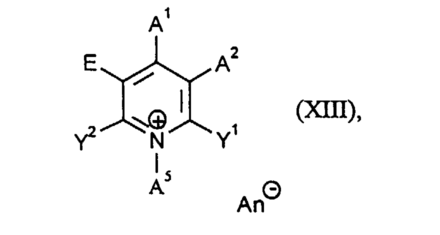 worin
- E, A¹ und A²: die oben genannte breiteste Bedeutung haben,
- An^{⊖}: ein Anion ist, das bspw, die für B^{⊖} angegebenen Bedeutungen annehmen kann, insbesondere für Cl^{⊖}, Br^{⊖}, J^{⊖}, T¹OSO₃^{⊖} oder TSO₃^{⊖} steht,
- A⁵: für T¹, T² oder T³ steht,
- Y¹ und Y²: unabhängig voneinander für eine Abgangsgruppe wie F, Cl, Br, J, -OSO₂T, -OT, -ST oder -SO₂T stehen,
mit Verbindungen der Formel (II) 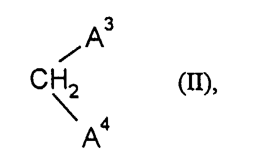 worin
- A³ und A⁴: die oben genannte breiteste Bedeutung besitzen
zu Verbindungen der Formel (XIV) 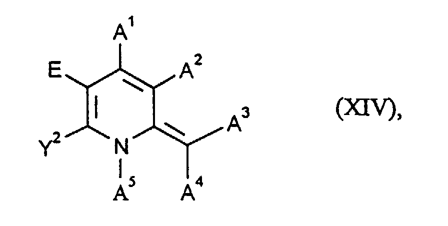 umsetzt, und diese mit Verbindungen der Formel (XV)

XH₂ (XV),

worin
- X: für O, NH, NT¹, NCOT, NCO₂T oder NSO₂T steht,
zu Verbindungen der Formel (V) umsetzt,
worin A⁵ für T¹, T² oder T³ steht.

In einer bevorzugten Ausführungsform dieses Verfahrens
steht E für H,
- A¹: für H oder T¹,
- A²: für H,
- Y¹: für Cl, Br oder J,
- Y²: für Cl, Br, J oder -OT¹ und
- H₂X: für H₂O, NH₃, H₂NT¹ oder H₂NSO₂T, insbesondere für H₂O.

In einer besonderen Ausführungsform des Verfahrens steht A⁵ für T¹ oder T², besonders bevorzugt für T¹, ganz besonders bevorzugt für Methyl oder Ethyl.

Die Umsetzung der Verbindungen der Formeln (XIII) und (II) kann in Wasser, in wäßrigen oder nichtwäßrigen Lösungsmitteln, wie z.B. Alkoholen oder dipolaren aprotischen Lösungsmitteln durchgeführt werden.

Geeignete Temperaturen sind -20 bis 150°C, bevorzugt 0 bis 100°C, insbesondere 15 bis 80°C.

Vorzugsweise führt man diese Reaktion in Gegenwart von anorganischen oder organischen Basen aus. Geeignet sind Alkalihydroxide, Alkalialkoholate, Alkalihydride, Alkalioxide, Erdalkalioxide, Alkalicarbonate, Alkalihydrogencarbonate, organische N-Basen wie tertiäre Amine (Triethylamin, DBU, DBN etc.). Diese Basen werden vorzugswseise in stöchiometrischen Mengen, d.h. doppelt molare Mengen in (XIII) eingesetzt.

Anstelle von Verbindung (II) kann für die Umsetzung mit Pyridiniumsalzen (XIII) auch das korrespondierende Anion von (II) 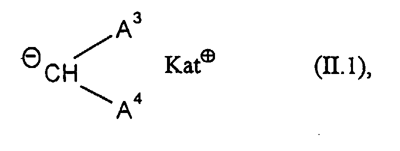 worin A³ und A⁴ die oben genannte breiteste Bedeutung besitzen und Kat^{⊕} für ein Kation wie z.B. diejenigen von Lithium, Natrium und Kalium steht, eingesetzt werden.

Auch die zweite Umsetzungsstufe - Überführung von (XIV) in (V) - wird vorzugsweise in Gegenwart von Basen durchgeführt.

Im Falle, daß die Verbindungen der Formel (XV) den Aminen -NH₃ oder H₂NT¹ entsprechen, werden diese Reaktanden bevorzugt auch gleichzeitig als Base und gegebenenfalls auch als Lösungsmittel eingesetzt.

Steht X für O, so werden als Basen bevorzugt Alkali-, Erdalkalihydroxide und/oder Alkalicarbonate eingesetzt.

Wenn X für -NCOT, -NCO₂T oder -NSO₂T steht, so wird als Base und Reaktant bevorzugt das korrespondierendes Anion z.B. C₆H₅SO₂NH^{⊖} eingesetzt.

Weitere bevorzugte Basen sind auch hier inerte Basen und sterisch gehinderte Basen. Geeignet sind z.B. tertiäre Amine wie Triethylamin, DBU, DBN. Bevorzugt werden mindestens 2 Mol Äquivalente Base eingesetzt.

Als Lösungsmittel sind vorzugsweise organische Lösungsmittel, die unter diesen Bedingungen inert sind, geeignet wie z.B. dipolare aprotische Lösungsmittel, aromatische Kohlenwasserstoffe wie Toluol, chlorierte Kohlenwasserstoffe wie Chlorbenzol.

Je nach Nukleophilie des Edukts H₂X bzw. der korrespondierenden Base findet die Reaktion bei Temperaturen zwischen -20°C bis 200°C, bevorzugt 0°C bis 150°C statt.

Die benötigten Pyridiniumsalze der Formel (XIII) sind in einfacher Weise durch Quarternierung entsprechenden Pyridine (XVI) mit Alkylierungsmitteln A⁵-An wie z.B. Dimethylsulfat, gemäß der Reaktion 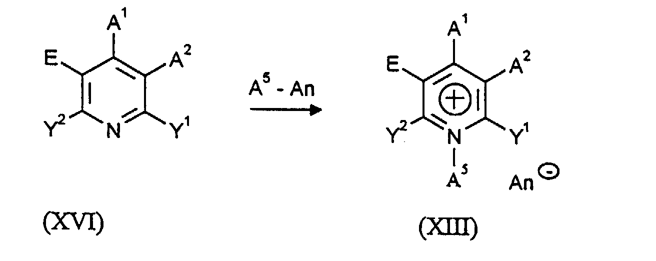 zugänglich.

Aus ökologischen und ökonomischen Gründen werden vorzugsweise die Quarternierung als auch die Umsetzung mit Verbindungen (II) und gegebenenfalls auch die Umsetzung mit XH₂ ohne Zwischenisolierung durchgeführt.

Ein weiteres Verfahren zur Herstellung der Kupplungskomponenten der Formel (V), worin A⁵ für T steht, ist dadurch gekennzeichnet, daß man Pyridinderivate der Formel (XVII) mit Verbindungen der Formel (II) umsetzt: 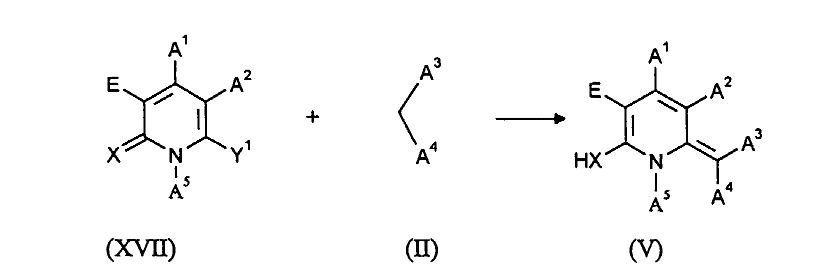 worin X=O, A¹ bis A⁴ und E die vorgenannte Bedeutung haben und A⁵ für T steht.

In einer bevorzugten Ausführungsform dieses Verfahrens steht E für H,
- A¹: für H oder T¹, ganz besonders bevorzugt für H,
- A²: für H,
- Y¹: für F, Cl, Br, J, OSO₂T, SO₂T, -ST, OT, besonders bevorzugt für Cl, Br, -SO₂T, ganz besonders bevorzugt für Cl und
- A⁵: für T¹, T², besonders bevorzugt T¹, ganz besonders bevorzugt für Methyl oder Ethyl,
einer der beiden Reste A³ und A⁴ steht bevorzugt für -CN, -CO₂T, -CONH₂, -CONHT, -CONT², -CHO, -COT, -SO₂T, -SO₂NH₂, -SO₂NHT, -SO₂NT₂, -NO₂, T⁴, T⁵,
während der andere bevorzugt für -CN, -CO₂T, -CONH₂, -CONHT oder -CONT² steht oder
- A³ und A⁴: bilden zusammen mit dem C-Atom, an das sie gebunden sind, eine cyclische methylenaktive Verbindung der Formeln (IIa bis IIv), bevorzugt (IIa bis IIh), insbesondere (IIa und IId).

Geeignete Lösungsmittel für diese Reaktion sind vorzugsweise organische Lösungsmittel. Hierbei kommen sowohl wassermischbare wie mit Wasser nicht mischbare Lösungsmittel in Frage.

Die Reaktionstemperaturen liegen vorzugsweise im Bereich von 20 bis 250°C, vorzugsweise bei 50 bis 150°C.

Vorzugsweise werden Basen zugesetzt, insbesondere mehr als 2 Mol-Äquivalente, bezogen auf das Edukt der Formel (XVII), Base eingesetzt.

Geeignete Basen sind anorganischer oder organischer Natur. Geeignet sind z.B. tertiäre Amine wie Triethylamin, DBU und DBN.

Ein weiteres Verfahren zur Herstellung von Kupplungskomponenten der Formel (V), die der Formel (XX) entsprechen, worin A⁵ für T¹, T² oder T³ steht, ist dadurch gekennzeichnet, daß man 2-Methylpyridone der Formel (XVIII) mit Phthalsäureestern der Formel (XIX) in Gegenwart von Basen umsetzt, 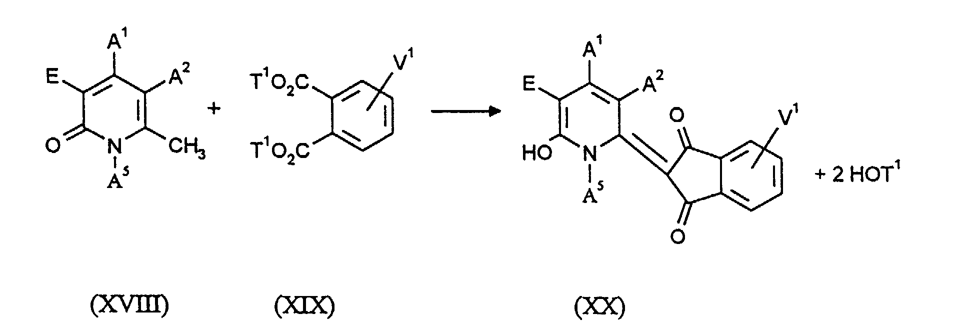 wobei A¹, A², E, T¹, V¹ die vorgenannte breiteste Bedeutung haben.

In einer bevorzugten Ausführungsform dieses Verfahrens
steht E für H,
A⁵ für T¹, besonders bevorzugt für Methyl oder Ethyl,
A¹ für H, T¹ oder T⁴, besonders bevorzugt für T¹, ganz besonders bevorzugt für Methyl und
V¹ für H.

Bei dieser Reaktion dient der Phthalsäureester der Formel (XIX) gleichzeitig als Lösungsmittel.

Geeignete Basen sind vorzugsweise Alkalialkoholate, die vorzugsweise in mindestens äquimolaren Mengen, bezogen auf (XVIII), eingesetzt werden.

Die Reaktionstemperatur liegt im allgemeinen bei 50 bis 200°C, vorzugsweise 100 bis 160°C, wobei der entstehende Alkohol T¹OH, vorzugsweise aus der Reaktion abdestilliert.

Durch Arbeiten unter vermindertem Druck kann die Abtrennung des Alkohols gefördert werden.

Durch Austragen auf Wasser und Ansäuern der wäßrigen Phase kann das Produkt kristallin isoliert werden.

Die erfindungsgemäßen Farbstoffe der Formel (I) eignen sich je nach Vorhandensein substratspezifischer Substituenten zum Färben und Bedrucken von natürlichen und synthetischen Materialien wie beispielsweise Cellulosefasern, Baumwolle, Wolle, Seide, Polyamid, Polyacrylnitril, Polyester oder Polyolefinen.

So sind bspw. die als Dispersionsfarbstoffe der Formel (I) bezeichneten Farbstoffe besonders geeignet entweder als solche oder auch im Gemisch mit anderen Dispersionsfarbstoffen zum Färben und Bedrucken von hydrophoben synthetischen Fasermaterialien und deren Mischungen mit natürlichen Faserstoffen.

Als hydrophobe, synthetische Materialien kommen z.B. in Betracht: Cellulose-2½-Acetat, Cellulosetriacetat, Polyamide und besonders Polyester, wie z.B. Polyethylenglykolterephthalat. Deren Mischungen mit natürlichen Faserstoffen sind z.B. Baumwolle, regenerierte Cellulosefasern oder Wolle.

Weiterhin sind sie geeignet zum Färben und gegebenenfalls Bedrucken von Wachsen, Ölen und Kunststoffen wie Polymethacrylat, PVC, Polystyrol oder ABS.

Ferner sind sie geeignet für textilen und nichttextilen Thermotransferdruck z.B. mittels eines Thermokopfes oder auch mittels Ink-jet-Verfahren.

Die im Rahmen dieser Anmeldung als kationische Farbstoffe der Formel (I) bezeichneten Verbindungen werden vorzugsweise zum Färben oder Bedrucken von sauer modifiziertem Polyester bzw. Polyamid, vorzugsweise aber zum Färben und Bedrucken von Polyacrylnitril verwendet.

Darüber hinaus können sie auch zum Färben von Papier eingesetzt werden.

Die im Rahmen dieser Anmeldung als Reaktivfarbstoffe der Formel (I) bezeichneten Verbindungen werden vorzugsweise zum Färben und Bedrucken von hydroxylgruppenhaltigen Materialien wie z.B. Cellulosefasern, insbesondere Baumwolle und zum Färben und Bedrucken amidgruppenhaltiger Materialien, wie z.B. Wolle, Seide, Polyamid, verwendet.

Die im Rahmen dieser Anmeldung als Säurefarbstoffe der Formel (I) bezeichneten Verbindungen werden vorzugsweise zum Färben und Bedrucken von natürlichen oder synthetischen Polyamid- sowie basisch modifizierter Polyacrylnitrilfasern. Darüberhinaus können sie zum Färben von Papier eingesetzt werden.

Die Farbstoffe können dabei aus wäßrigen oder nichtwäßrigen Flotten oder aber auch in Druckverfahren angewandt werden.

Sie ergeben farbstarke Färbungen mit guten Allgemeinechtheiten.

Neutrale Farbstoffe ergeben auf Polyester besonders brillante Färbungen mit hoher Lichtechtheit. Die Nuancen reichen hierbei von Gelb bis Blau, wobei die besondere Stärke in brillanten roten Nuancen liegt.

Sofern A³ und A⁴ in den in den Beispielen genannten Formeln unterschiedliche Bedeutungen besitzen, so soll die Konfiguration an dem Kohlenstoffatom, an das sie gebunden sind, nicht durch die Formeln festgelegt sein. Die in den Beispielen verwendete Enamine 3-Amino-2-cyano-penten-disäurediester (Δ 2-Amino-1-cyan-glutaconsäurediester) wurden analog dem in Liebigs Anm. Chem. 1987, 1131 beschriebenen Verfahren hergestellt, worin derartige Enamine als Z-konfiguriert beschrieben sind.

### Beispiele

### Beispiel 1

Herstellung des Farbstoffs der Formel 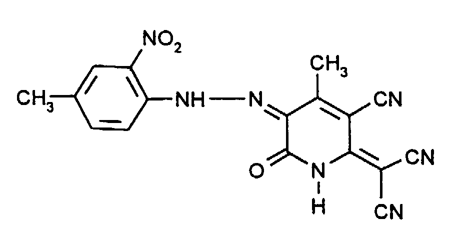 6,1 g 4-Methyl-2-nitroanilin, gelöst in 20 ml Propionsäure und 40 ml Eisessig wurden bei 0-5°C innerhalb von 10 Min mit 5,5 ml 40-%iger Nitrosylschwefelsäure versetzt. Man rührte weitere 2 h bei 0-5°C. Zu einer mittels weniger ml konz. Natronlauge auf pH 7 gestellten Lösung von 5,8 g [6-Hydroxy-3-cyano-4-methyl-2(1H)-pyridinyliden]-malonitril in 400 ml Wasser fügte man 20 ml 10 %ige wäßrige Amidosulfonsäurelösung und anschließend bei 5°C die obige Diazotierungsreaktionsmischung. Man ließ innerhalb von 16 h auf Raumtemperatur erwärmen, saugte den Niederschlag ab und wusch den Filterkuchen mit Wasser neutral.
Ausbeute: 9,3 g
MS, m/z (%): 361 (72) [M^{+.}], 315 (18) [M^{+.}-NO₂] 152 (100)
UV (DMF): λₘₐₓ = 537 nm

Der Farbstoff färbt Polyester in einem blaustichigen Rot mit guten Echtheiten.

Analog Beispiel 1 wurden die nachfolgend aufgeführten Farbstofffe der Formel 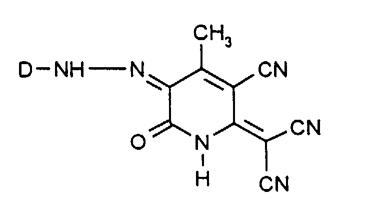 erhalten, die Polyester ebenfalls mit guten Echtheiten färben.

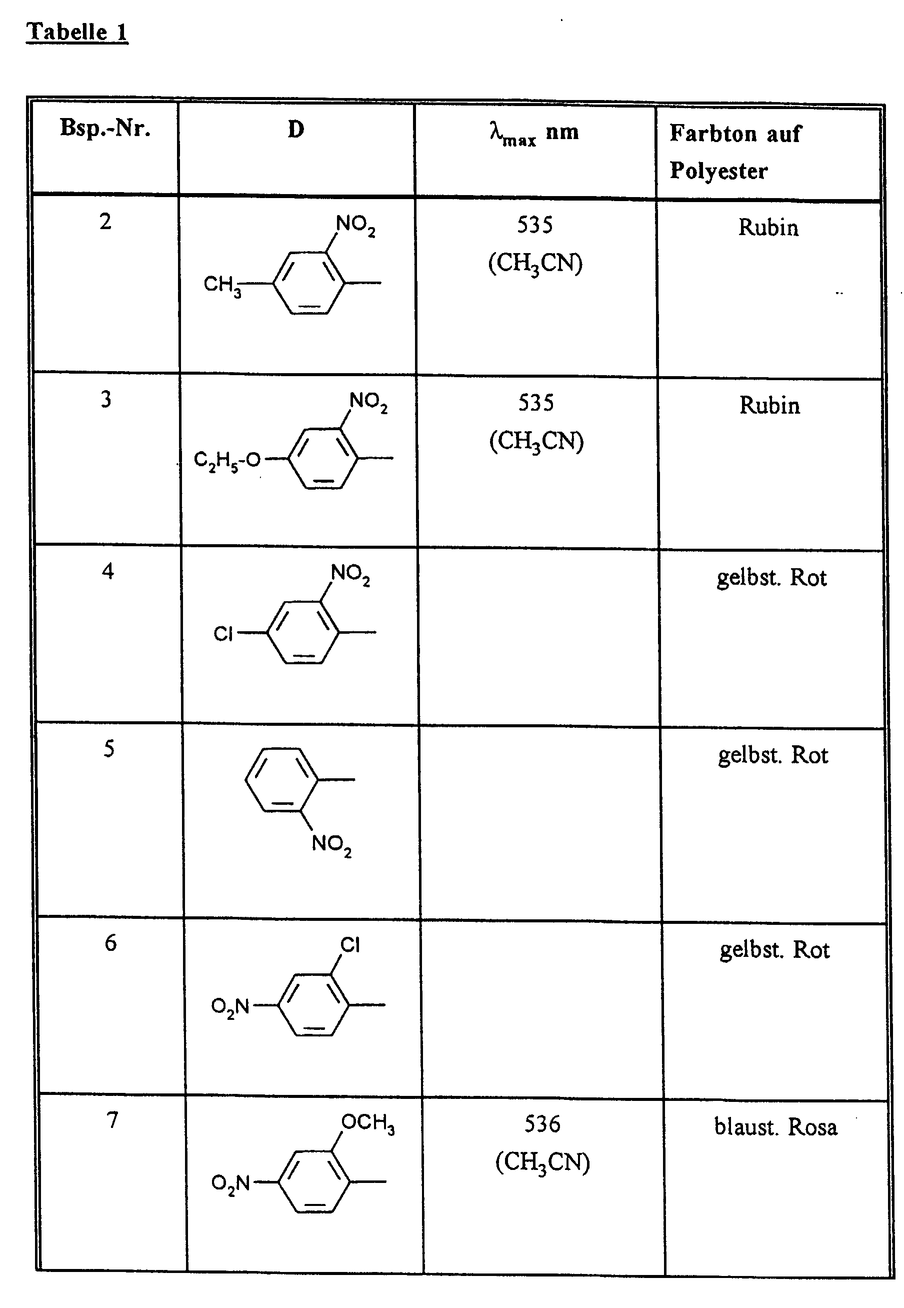

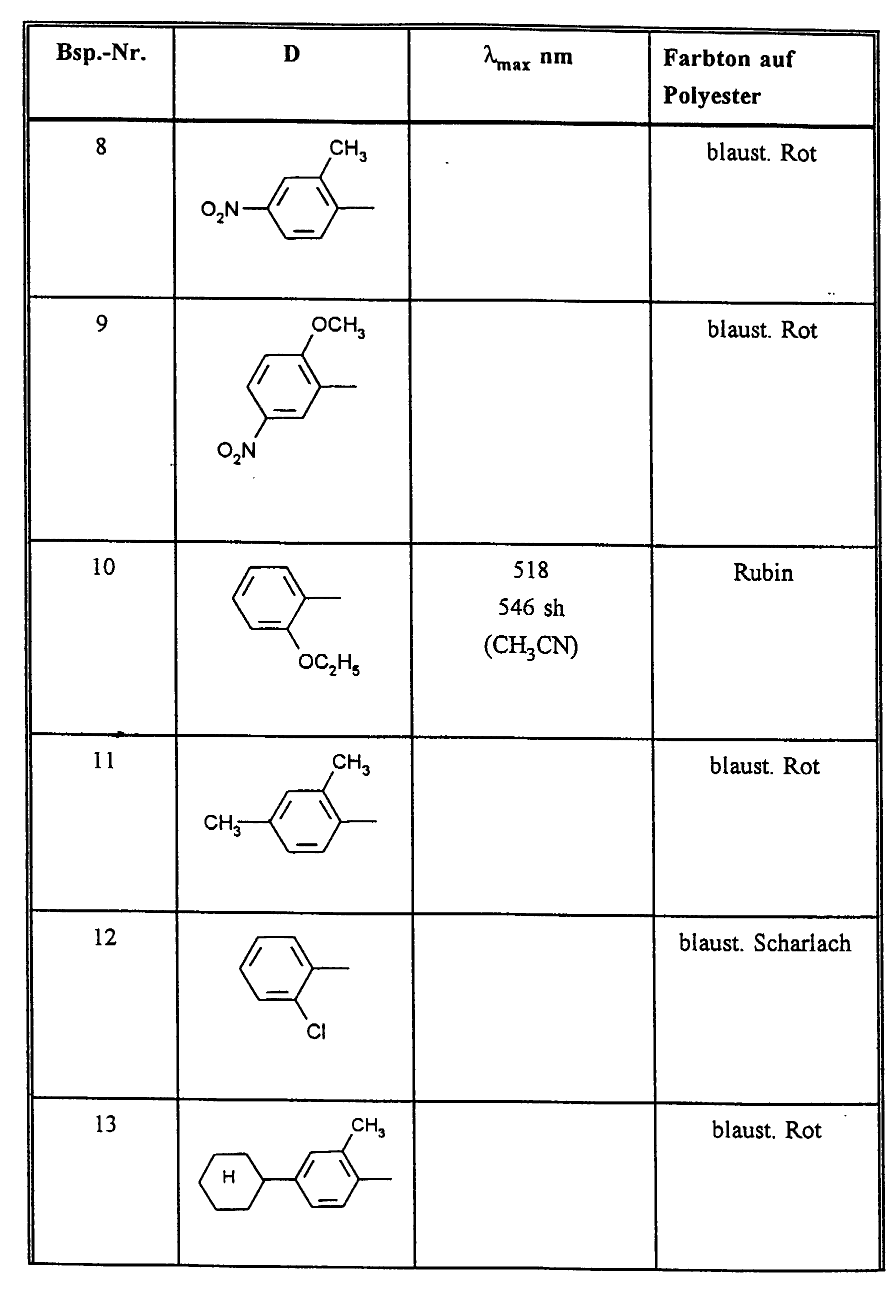

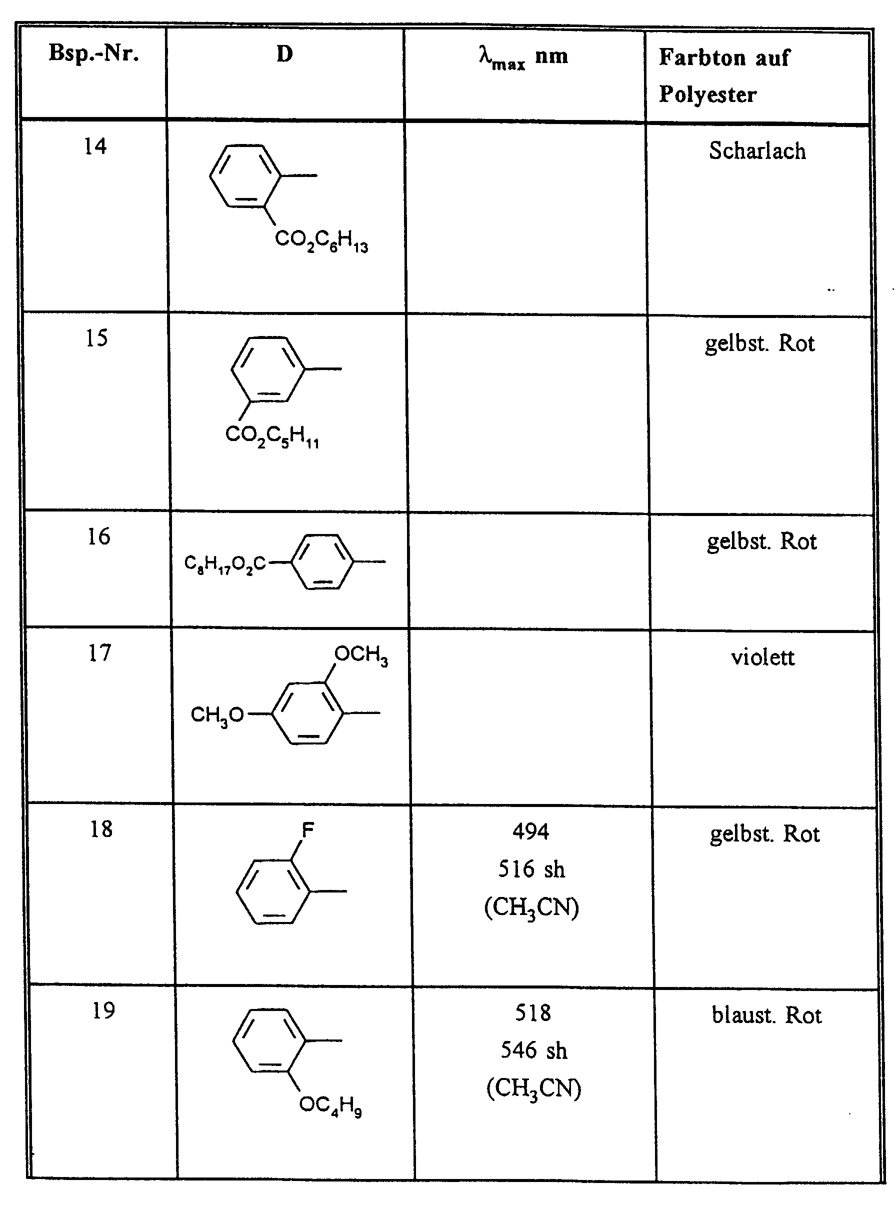

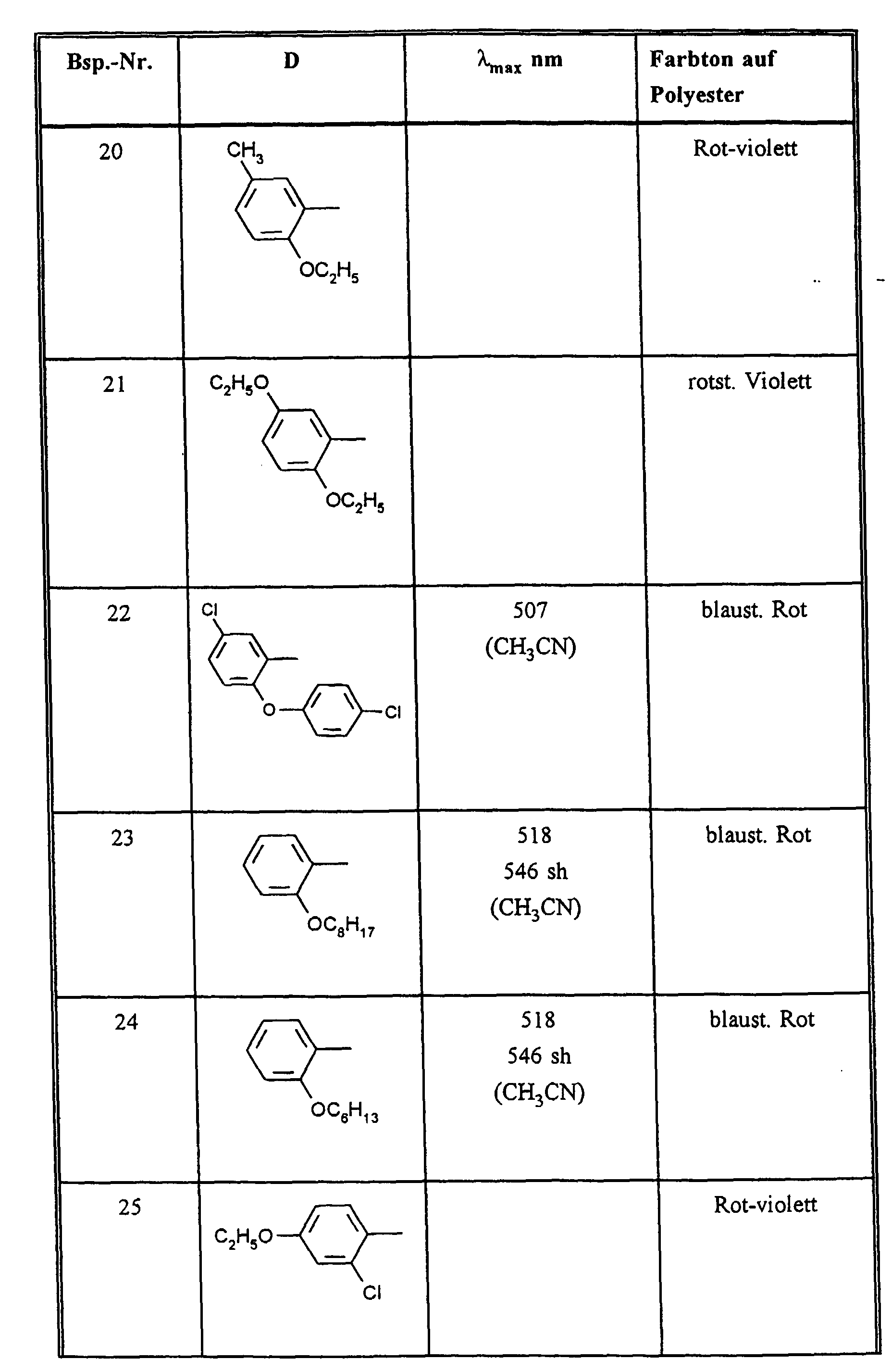

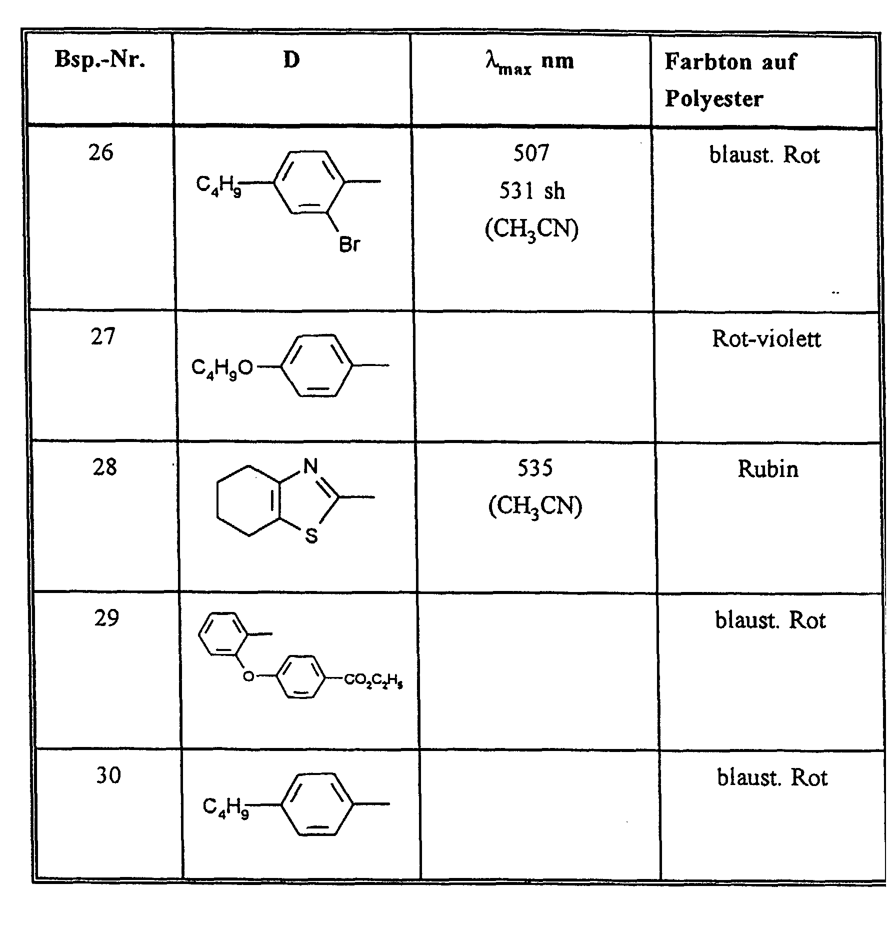

### Beispiel 31

Herstellung von [6-Hydroxy-3-cyano-4-methyl-2(1H)-pyridinyliden]-malonitril 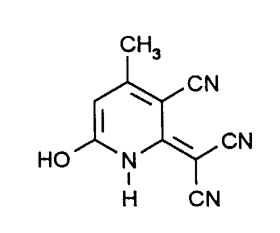 15,4 g Natriumsalz des 2-Amino-1,2,3-tricyan-propen ("Dimers Malonitril") wurden mit 13 g Acetessigsäureethylester, 1 ml Piperidin und 6 g Eisessig in 100 ml Ethanol 16 h zum Sieden unter Rückfluß erhitzt. Nach Abkühlen saugte man ein beiges Pulver ab und wusch mit Ethanol.
Ausbeute: 14,5 g
IR ν (cm⁻¹): 3430, 2185 (C≡N), 2205 (C≡N), 2225 (C≡N) 1668, 1566, 1356, 832
¹H-NMR (d₆-DMSO, 300 MHz, ppm): δ = 2,15 (s, 3H, CH₃), 5,70 (s, 1H), 7,30 (s, 2H, OH, NH)
MS, m/z (%): 198 (42) [M^{+.}], 170 (13) [M^{+`}-H₂O] 155 (100)

### Beispiel 32

Herstellung des Farbstoffs der Formel 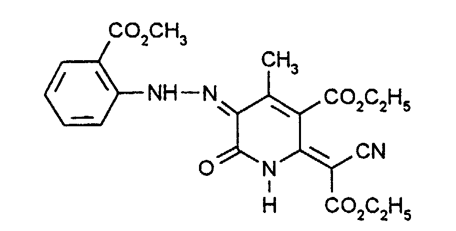 6,6 g 2-Aminobenzoesäuremethylester, gelöst in 15 ml Propionsäure und 30 ml Eisessig wurden bei 0-5°C mit 8,3 ml 40 %ige Nitrosylschwefelsäure innerhalb von 10 Min versetzt. Man rührte weitere 2 Stunden bei 0°C.

70 ml der im nachfolgenden Beispiel Nr. 33 beschriebenen Lösung der Kupplungskomponente[6-Hydroxy-4-methyl-3-(ethoxycarbonyl)-2(1H)-pyridinyliden]-cyanessigsäureethylester (ca. 0,5 Mol) in 550 ml DMF wurden mit Ethanol auf 200 ml aufgefüllt, mit 2 g Amidosulfonsäure versetzt und anschließend bei 0°C mit obiger Diazotierung unter Zusatz von 50 g Eis gekuppelt. Man ließ 15 Stunden nachrühren, saugte ab und wusch mit Wasser.
Ausbeute: 15,3 g. Nach Umkristallisieren aus DMF verblieben 6,3 g.

Der Farbstoff färbte Polyester in brillantem Orange mit guten Echtheiten.
- MS, m/z (%):: 454 (100) [M^{+.}], 422 (8) 409 (8), 350 (25), 349 (16), 322 (25) 293 (18)
- λₘₐₓ:: 483 nm (CH₃CN)

### Beispiel 33

Herstellung der Kupplungskomponente der Formel 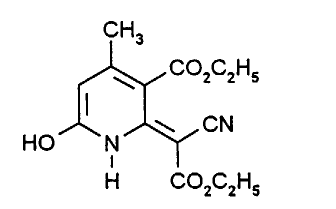 Eine Lösung von 113 g 3-Amino-2-cyano-pentendisäurediethylester ("Dimerer Cyanessigsäureethylester", 0,5 mol), 90 ml Acetessigsäureethylester und 76 g Diazabicycloundecen (DBU) in 500 ml DMF wurde 11 h bei 120°C gerührt. Man setzte nochmals 25 ml Acetessigsäureethylester zu und rührte weitere 9 h bei 120°C unter Abdestillieren der leichtflüchtigen Spaltprodukte. Man füllte mit 50 ml DMF auf ein Volumen von 800 ml auf und setzte die braune Lösung direkt zur Kupplung ein. Eine Probe wurde bei 20 mbar/100°C vom Lösungsmittel befreit. Das zurückbleibende braune Öl zeigte folgendes Massenspektrum:
MS, m/z (%): 292 (27) [M^{+.}], 247 (14), 218 (27), 192 (42), 174 (58), 164 (35), 152 (65), 151 (51), 148 (43), 96 (33), 42 (64), 29 (100)

Analog Beispiel 32 wurden die nachfolgend (Tabelle 2) aufgeführten Farbstoffe 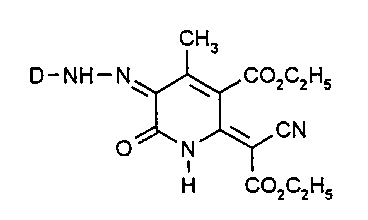 erhalten, die Polyester ebenfalls mit guten Echtheiten färben.

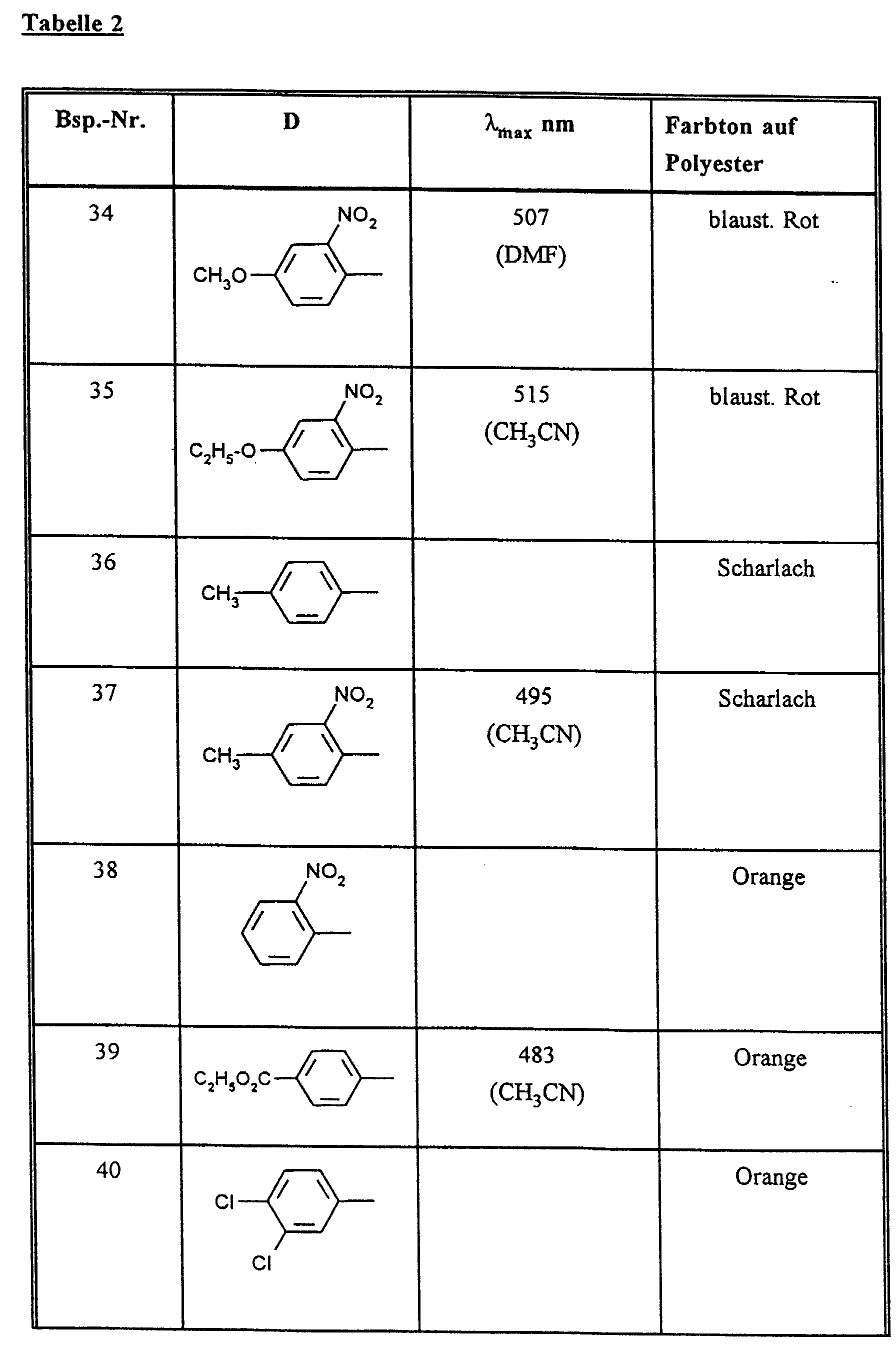

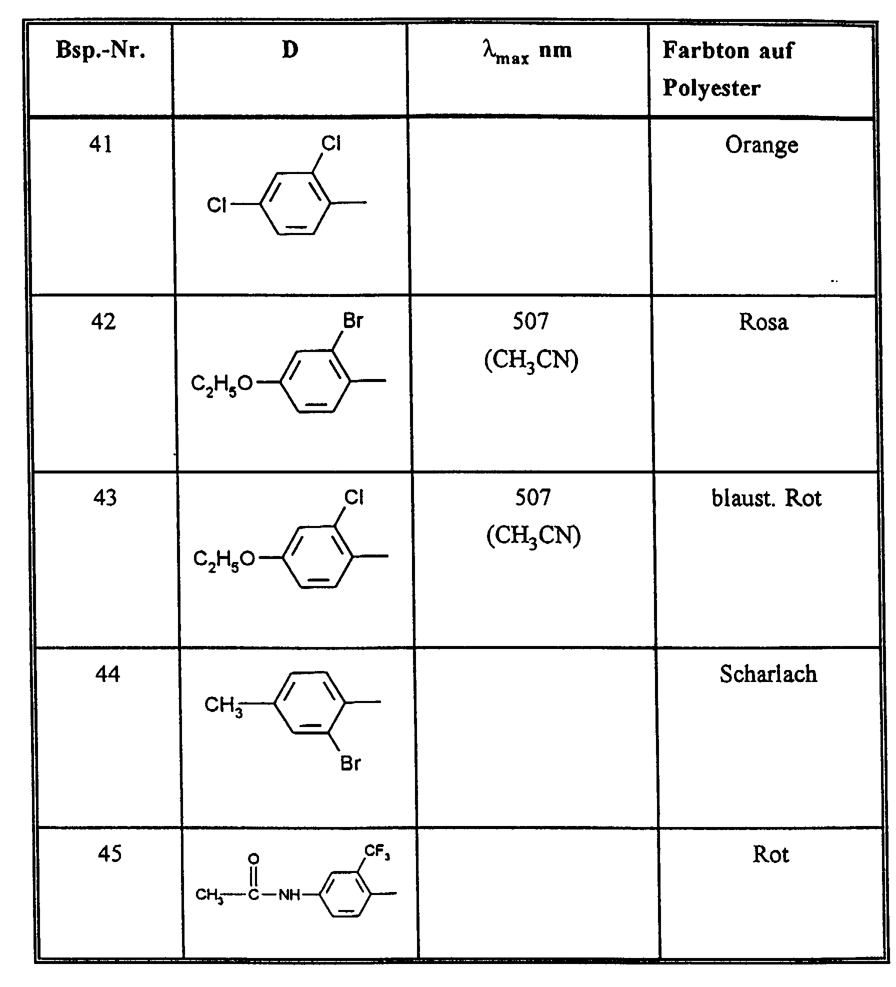

### Beispiel 46

Herstellung des Farbstoffgemisches der Formel 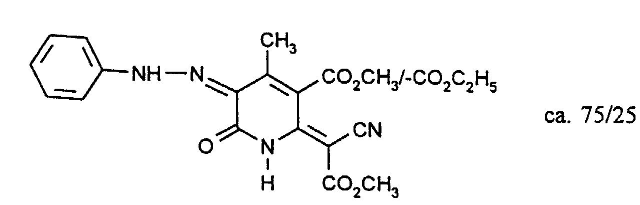 11,7 g 2-Phenylazo-acetessigsäureethylester und 11 g Natriumsalz von 3-Amino-2-cyano-pentendisäuredimethylester ("Dimerer Cyanessigsäuremethylester") wurden in 50 ml DMF 10 h bei 90°C gerührt. Nach Zusatz von weiteren 2,2 g dimerem Cyanessigsäuremethylester erhitzt man nochmals 6 h auf 100°C. Nach Abkühlen auf Raumtemperatur wurde mit 100 ml Methanol, 6 g 30 %iger Salzsäure und 15 ml Wasser versetzt. Man saugte ab und wusch mit Methanol.
Ausbeute: 4,1 g
Der Farbstoff färbte Polyester in brillantem Scharlach mit guten Echtheiten.
- MS, m/z (%): 382 (22) [M^{+.} Ethylester] 369 (20), 368 (89) [M^{+.} Methylester], 337 (9) [M^{+.} Methylester - OCH₃], 310 (9), 276 (7), 231 (19), 199 (11), 105 (19), 93 (30), 92 (28), 77 (100)
- λₘₐₓ:: 486 nm (CH₃CN)

Analog Beispiel 46 wurden die folgenden Farbstoffe der Formel 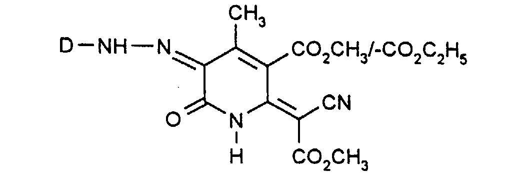 erhalten, die ebenfalls Polyester mit guten Echtheiten färben.

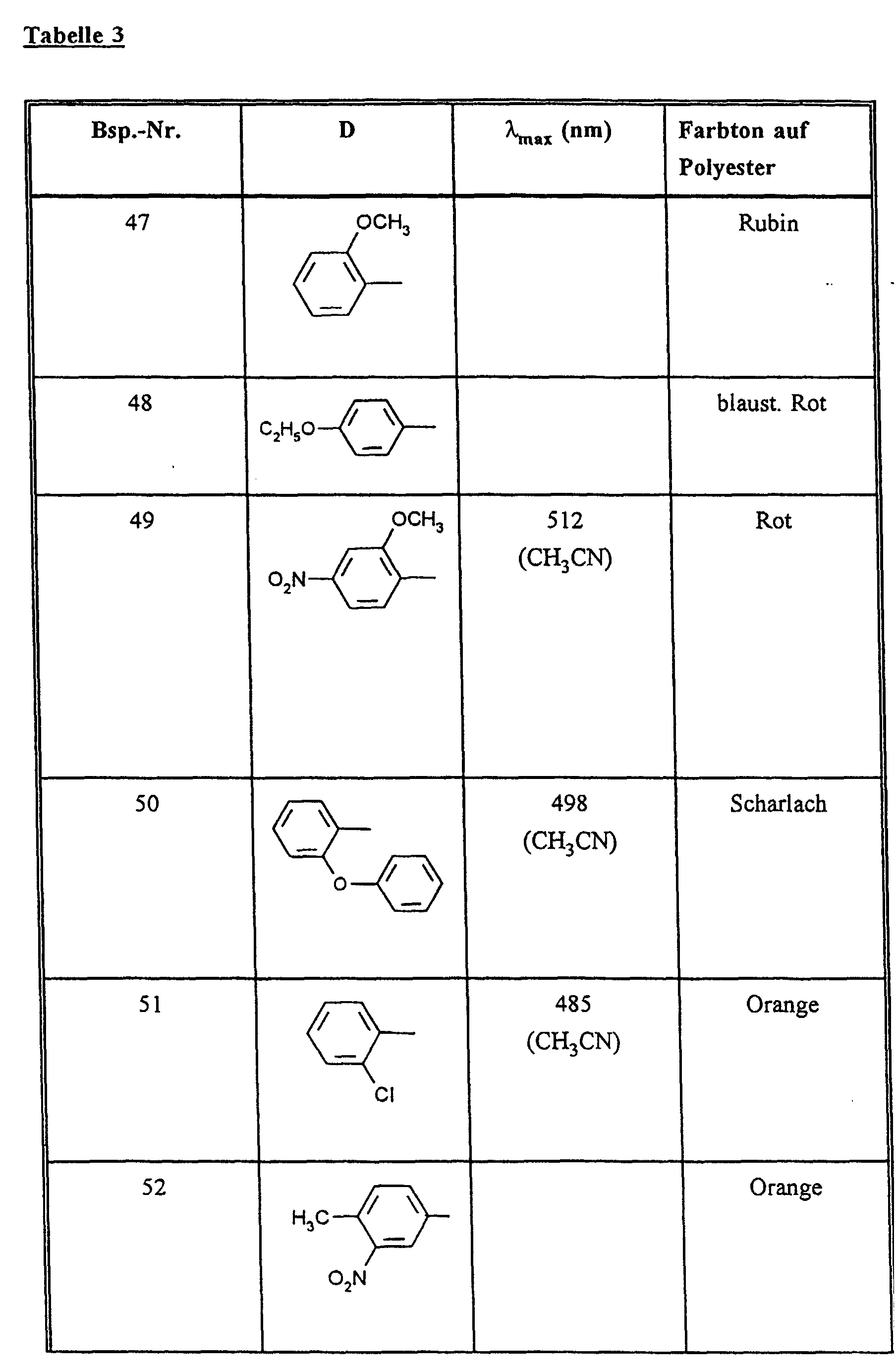

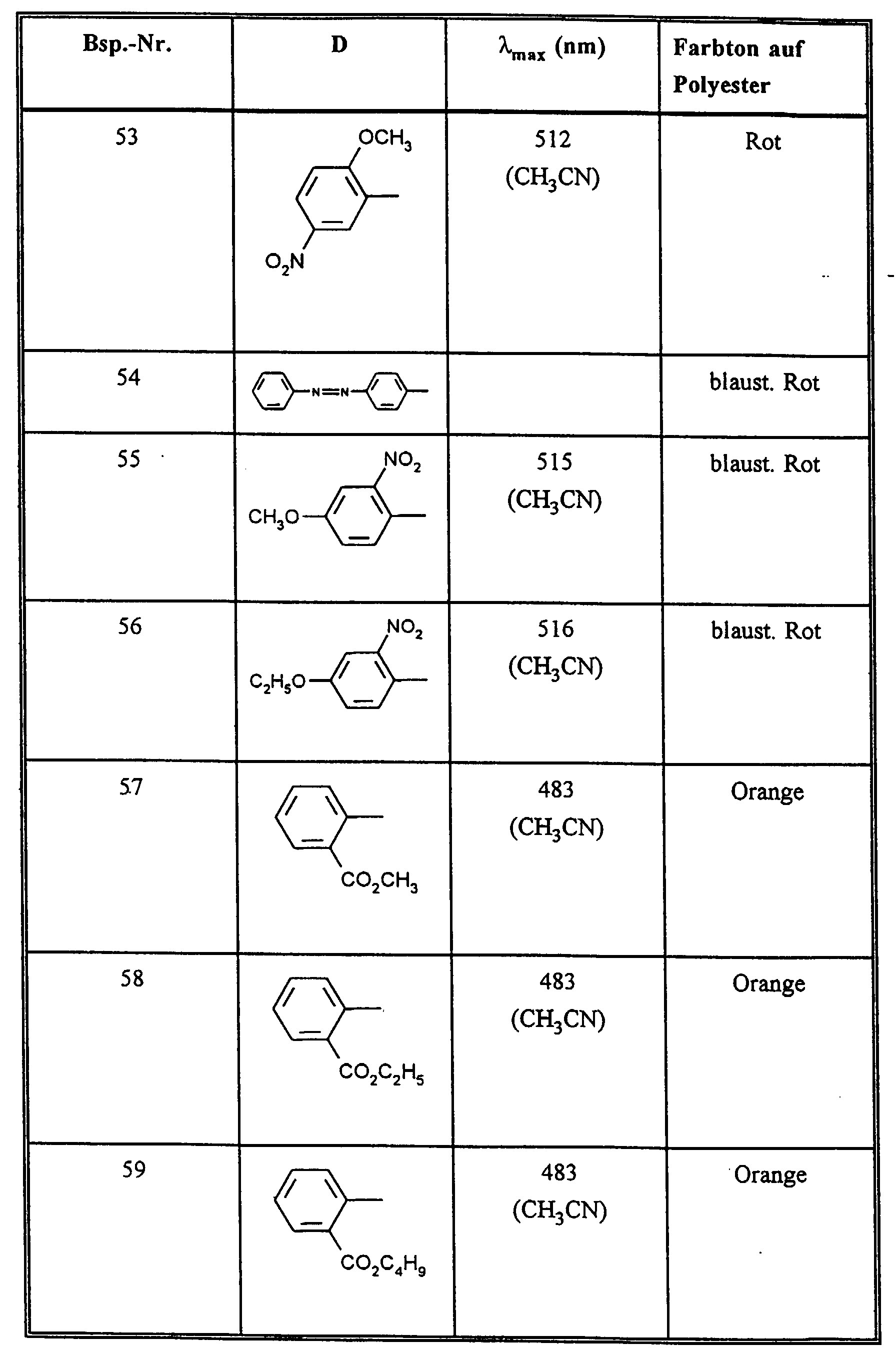

### Beispiel 60

Herstellung der Kupplungskomponente der Formel 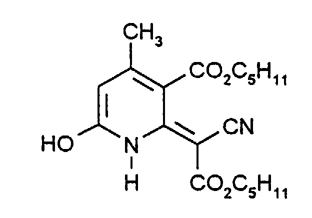 3-Amino-2-cyano-pentendisäurediamylester ("Dimerer Cyanessigsäureamylester") wurde analog Junek, Monatshefte für Chemie, 101 [1979] 1208, dargestellt.

31 g 3-Amino-2-cyano-pentendisäurediamylester wurden mit 12,2 ml Diazabicyclononen (DBN) und 34,4 g Acetessigsäureamylester versetzt und 4 h auf 80°C erhitzt. Man setzte nochmals 8,6 g Acetessigsäureamylester zu und erhitzte weiter 2 h auf 80°C. Man erhielte ein braunes Öl, das in 200 ml NMP aufgenommen ein Gewicht von 276 g ergab und direkt zur Kupplung eingesetzt wurde.
Eine Probe, die bei 0,5 mbar/100°C eingeengt wurde, ergab folgendes Massenspektrum:
MS, m/z (%) 376 (12) [M^{+.} ], 262 (8), 236 (11), 219 (18) 218 (21), 193 (11), 192 (45), 151 (30), 55 (35), 43 (100)

Durch Kupplung analog Beispiel 32 wurden die nachfolgende aufgeführten Farbstoffe erhalten, die Polyester ebenfalls mit guten Echtheiten färbten.

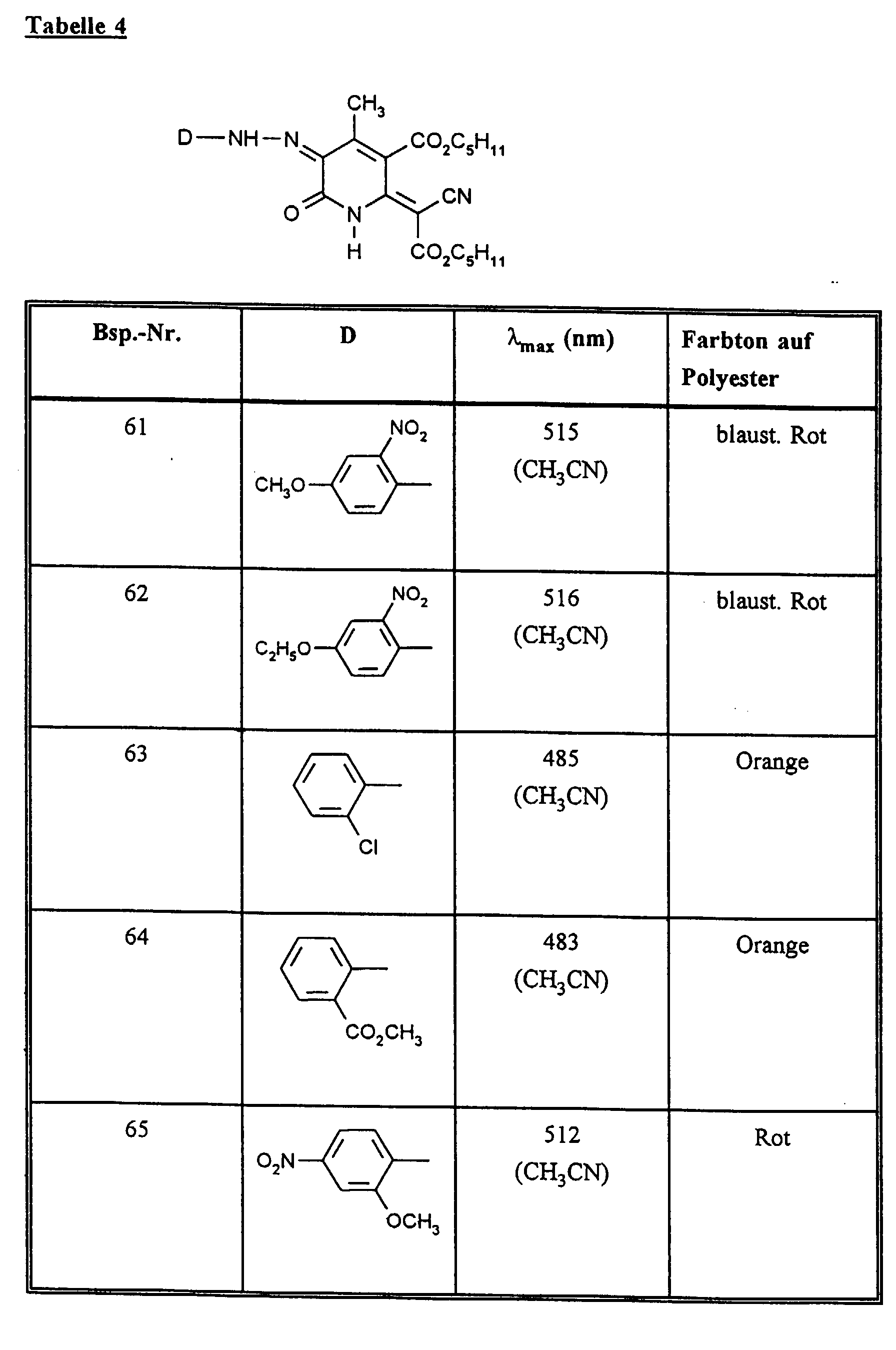

### Beispiel 66

Herstellung der Kupplerkomponente der Formel

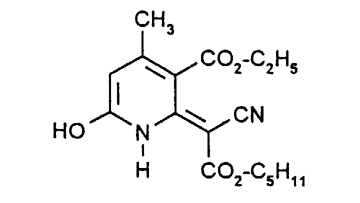

Analog Ivanov J.C. et al., Liebigs Ann. Chem. 1983, 753-60 sowie Mittelbach M. und Junek H., Liebigs Ann. Chem. 1986, 533-544 stellte man durch Umsetzung von 3-Amino-ethoxy-2-propensäure-ethylester-hydrochlorid mit Cyanessigsäureamylester in Chloroform in Gegenwart von Triethylamin 3-Amino-2-cyano-pentendisäure-1-amylester-5-ethylester 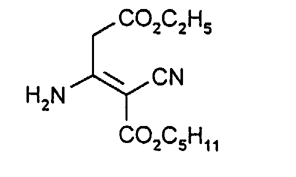 dar. Die weitere Umsetzung mit Acetessigsäureethylester analog Beispiel 33 lieferte die Kupplerkomponente der obigen Formel.

### Beispiel 67

Herstellung des Farbstoffs der Formel

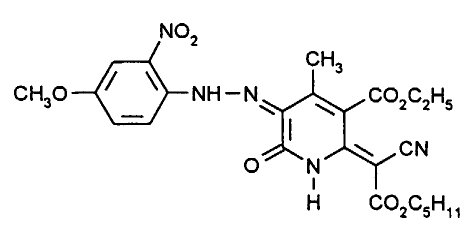

Durch Diazotierung von 2-Nitro-4-methoxy-anilin in Salzsäure mit 30 %iger Nitritlösung bei 0°C und anschließende Kupplung mit der Kupplungskomponente gemäß Beispiel 66 wurde der Farbstoff obiger Formel erhalten. Er färbte Polyester in brillantem Rot mit guten Echtheiten.
MS, m/z (%): 514 (28), [M^{+.} + H], 513 (100) [M^{+.}] 399 (12), 370 (26), 168 (29), 43 (36)
λₘₐₓ: 515 nm (CH₃CN)

### Beispiel 68

Herstellung des Gemisches der Kuppler der Formel

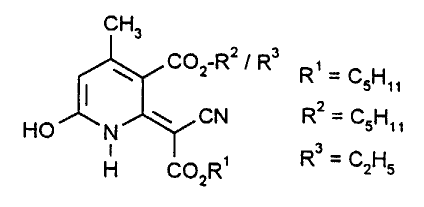

394 g 3-Amino-2-cyano-pentendisäurediamylester (78,7 % nach GC) wurden zusammen mit 258 ml Acetessigsäureethylester und 154 ml Diazabicyloundecen 8 h bei 80°C/60 mbar unter Abdestillieren leichtflüchtiger Komponenten erhitzt. Der Überschuß Acetessigester wurde bei 0,5 mbar/100°C abdestilliert. Das zurückbleibende braune Öl (644 g) wurde direkt zur Kupplung eingesetzt.
MS, m/z (%): 376 (14) [M^{+.}], 334 (5) [M^{+.}], 307 (5), 262 (12), 219 (23), 218 (22), 193 (15), 192 (67), 151 (48), 137 (20), 126 (27), 123 (27), 98 (19), 96 (17), 71 (20), 55 (33), 43 (100)

Durch Kupplung analog Beispiel 32 mit Kupplungskomponente, die analog den Beispielen 33, 60, 66 oder 68 zugänglich sind, erhielt man die in nachfolgender Tabelle 5 aufgeführten Farbstoffmischungen der Formel

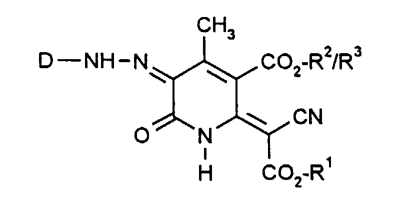

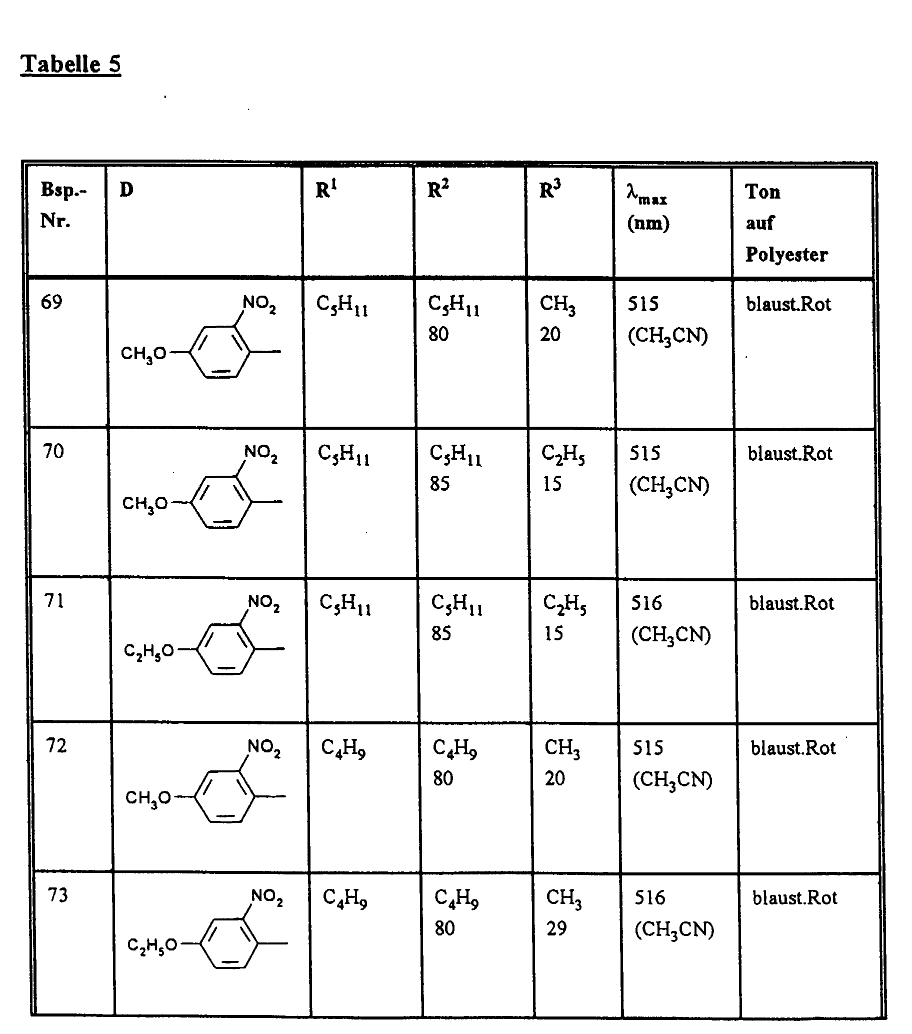

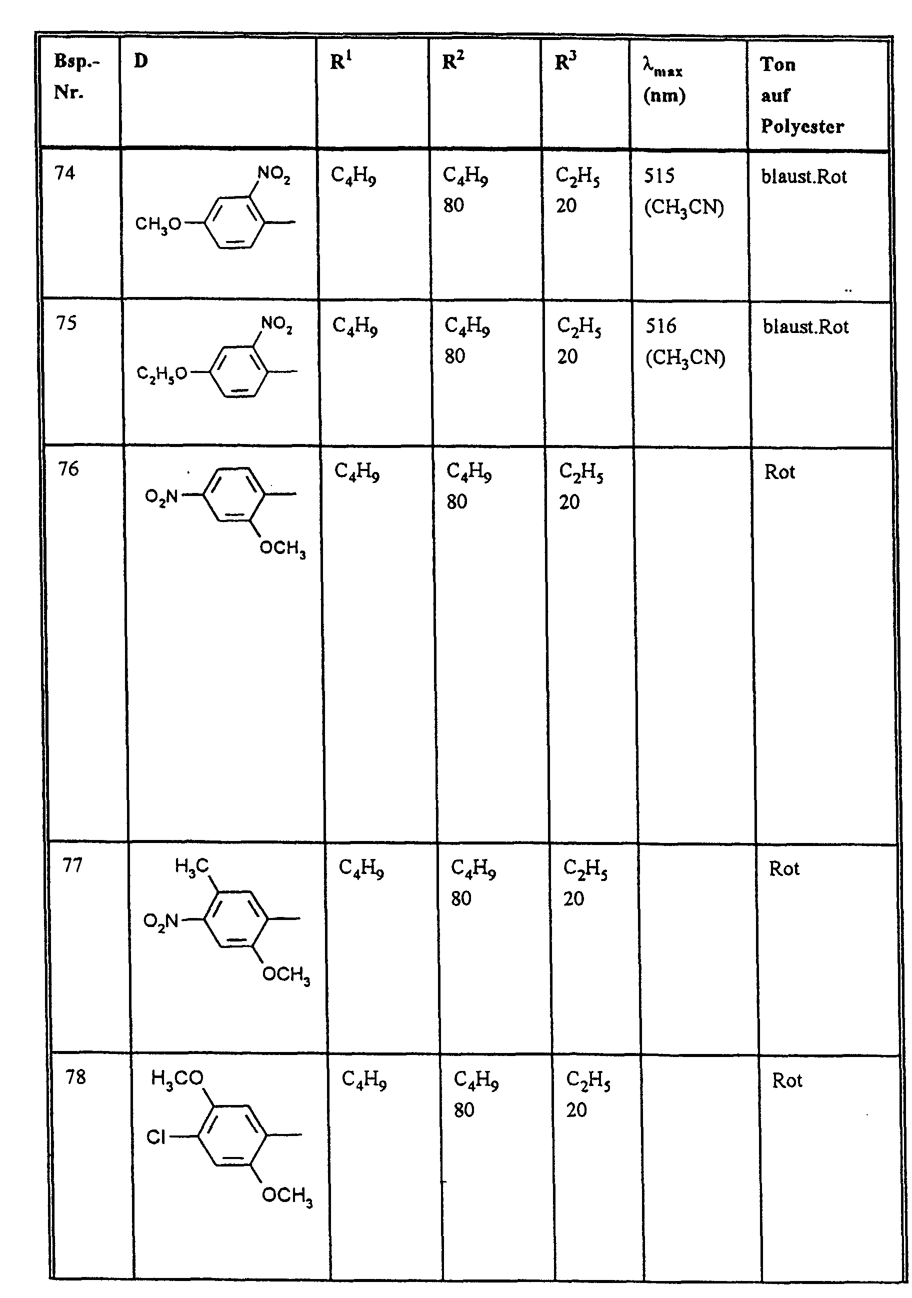

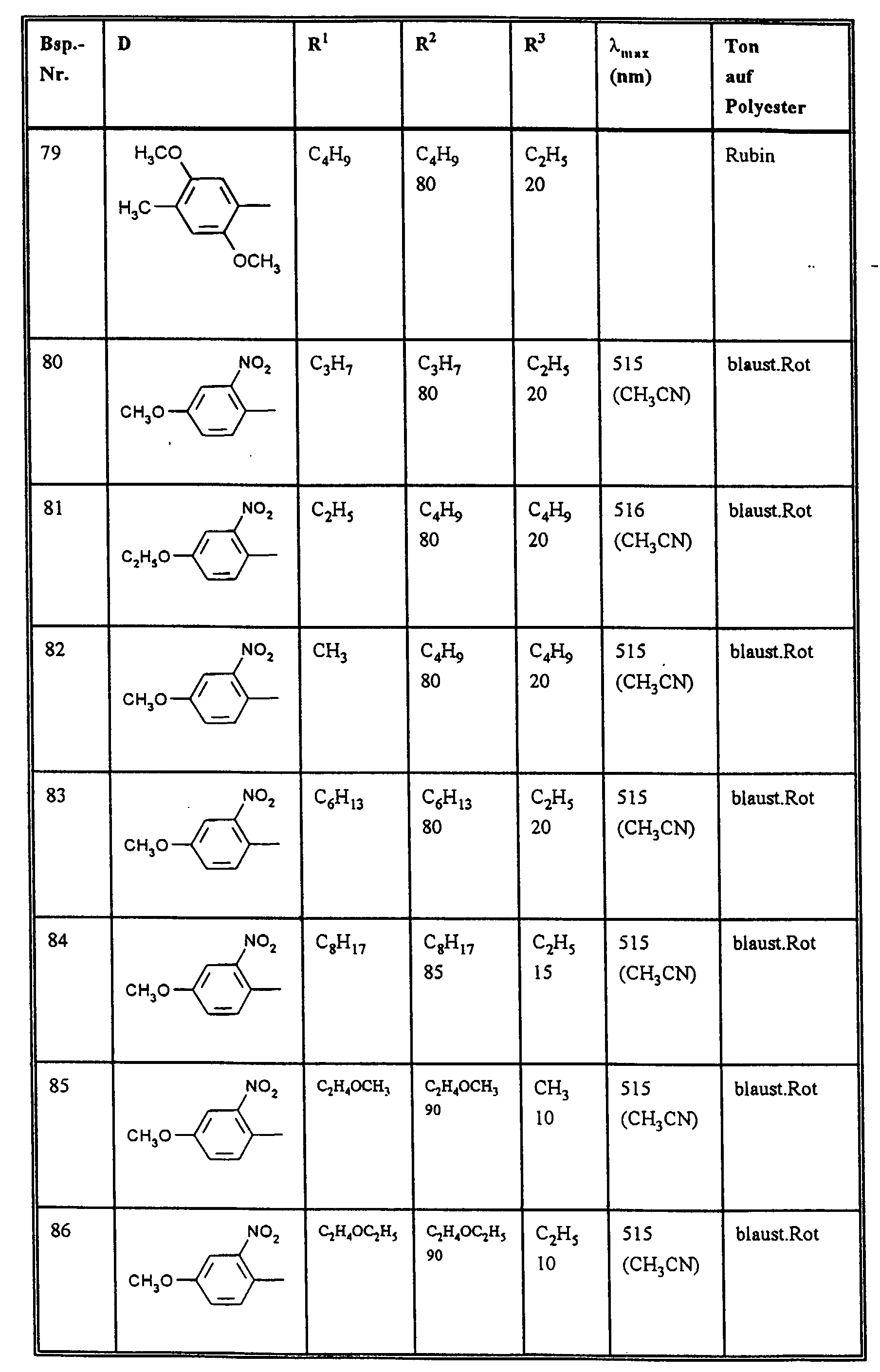

### Beispiel 87

Herstellung des Farbstoffs der Formel

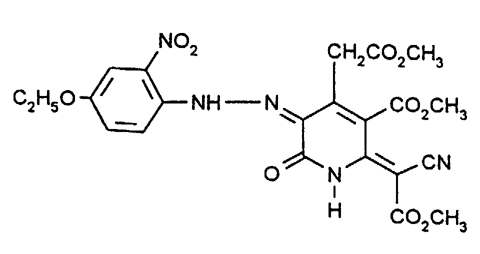

Die Herstellung der Kupplungskomponente erfolgte durch Umsetzung von Acetondicarbonsäuredimethylester mit 3-Amino-2-cyano-pentendisäuredimethylester in Gegenwart von DBU bei 100°C analog Beispiel 68. Kupplung mit diazotiertem 2-Nitro-4-ethoxyanilin ergab den Farbstoff obiger Formel.
- MS, m/z (%):: 515 (100) [M⁺], 516 (27), 457 (20), 456 (19), 425 (8), 424 (36), 153 (12)

Der Farbstoff färbte Polyester in blaustichigem Rot mit guten Echtheiten.

### Beispiel 88

Herstellung des Farbstoffs der Formel

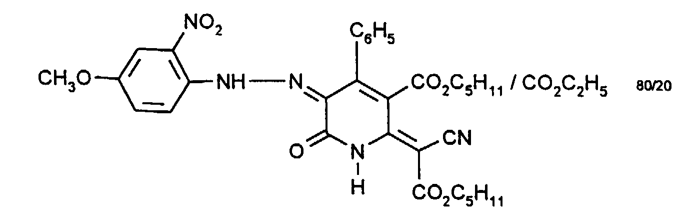

Die Herstellung der Kupplungskomponente erfolgte analog Beispiel 68 durch Umsetzung von Benzoylessigsäureethylester mit 3-Amino-2-cyano-pentendisäurediamylester. Die anschließende Kupplung mit diazotiertem 2-Nitro-4-methoxyanilin in Methanol lieferte den Farbstoff obiger Formel in kristalliner Form.

Der Farbstoff färbte Polyester in blaustichigem Rot mit guten Echtheiten.
MS, m/z (%): 514 (28) [M^{+.} + H], 513 (100) [M^{+.}], 471 (6), 443 (6), 399 (12), 370 (26), 168 (29), 43 (36)
λₘₐₓ: 497 nm (CH₃CN)

Analog Beispiel 88 wurden die folgenden Farbstoffe der Formel 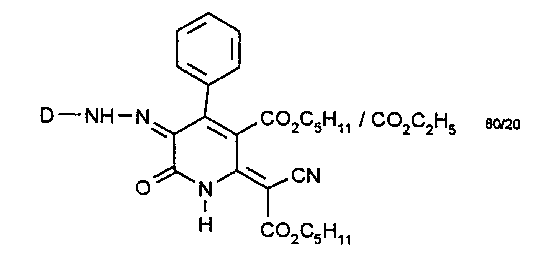 erhalten.

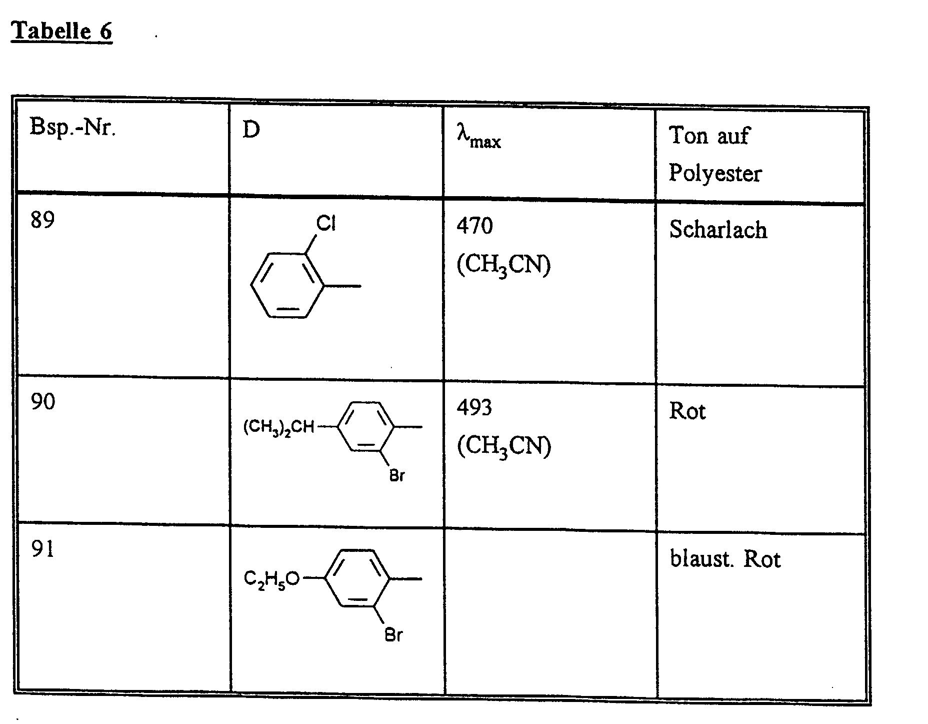

### Beispiel 92

Herstellung des Kupplers der Formel

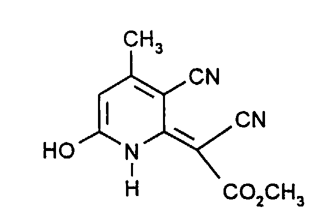

49,5 g 3-Amino-2-methoxycarbonyl-2-pentendinitril (H. Junek et al., Synthesis 1977, 560) wurden mit 47 g Acetessigsäureethylester und 30,6 g Piperidin in 300 ml Ethanol 13 Stunden zum Sieden unter Rückfluß erhitzt. Nach Abkühlen verdünnte man mit Eiswasser auf ein Volumen von 1,8 l, stellte mit konz. Salzsäure auf pH = 2, saugte ab und wusch mit Wasser. Ausbeute 59 g.
MS, m/z (%): 232 (5), 231 (24) [M^{+.}], 187 (42), 172 (36), 160 (14), 144 (21), 132 (13), 92 (26), 91 (29), 84 (23), 59 (100)
¹H-NMR (d₆-DMSO, 300 MHz, ppm): δ = 2,15 (s, 3H, CH₃), 3,60 (s, 3H, OCH₃), 5,70 (s, 1H, 5-H), 8,25 (s, (br), 1H, N-H), 12,65 (s, 1H, O-H)

Die nachfolgenden Farbstoffe der Formel 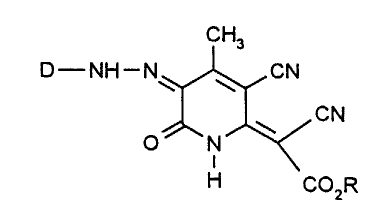 wurden analog Beispiel 32 unter Verwendung von Kupplungskomponenten des Beispiels 92 oder homologer Kupplungskomponenten hergestellt.

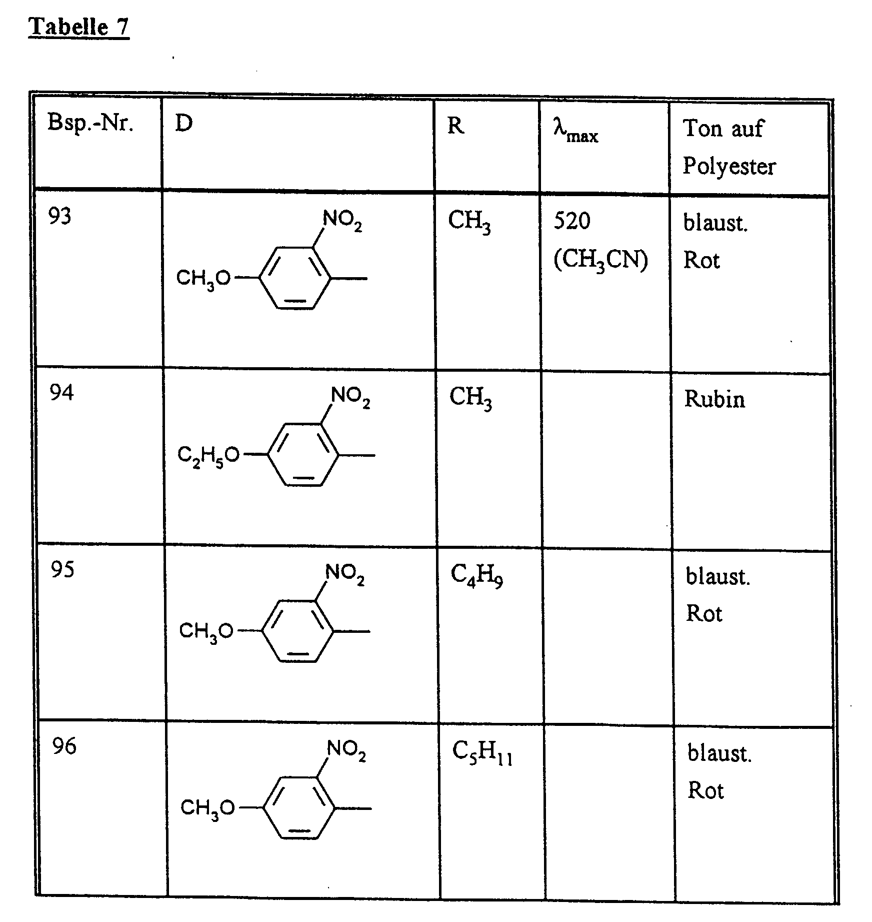

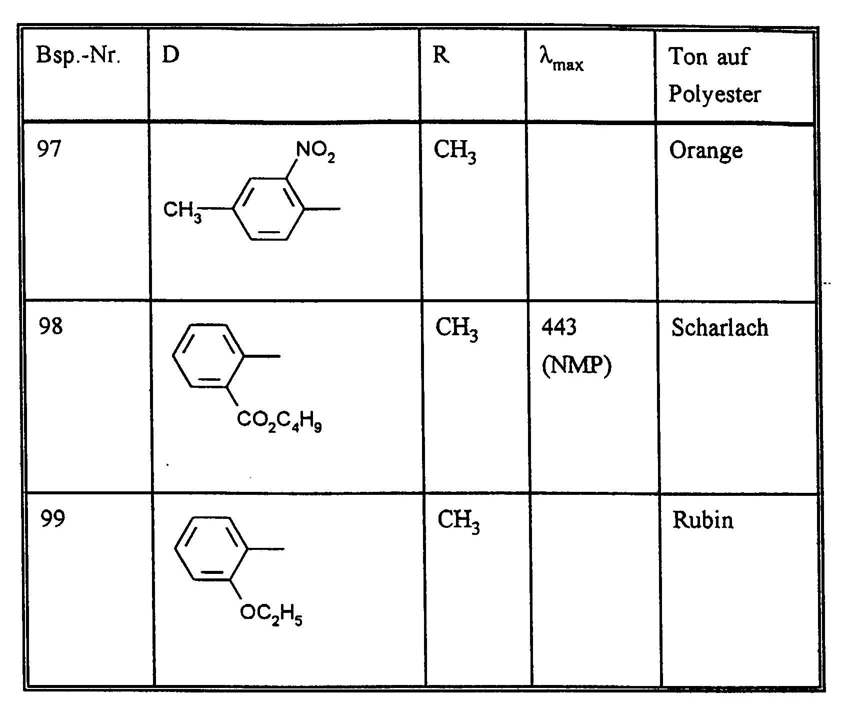

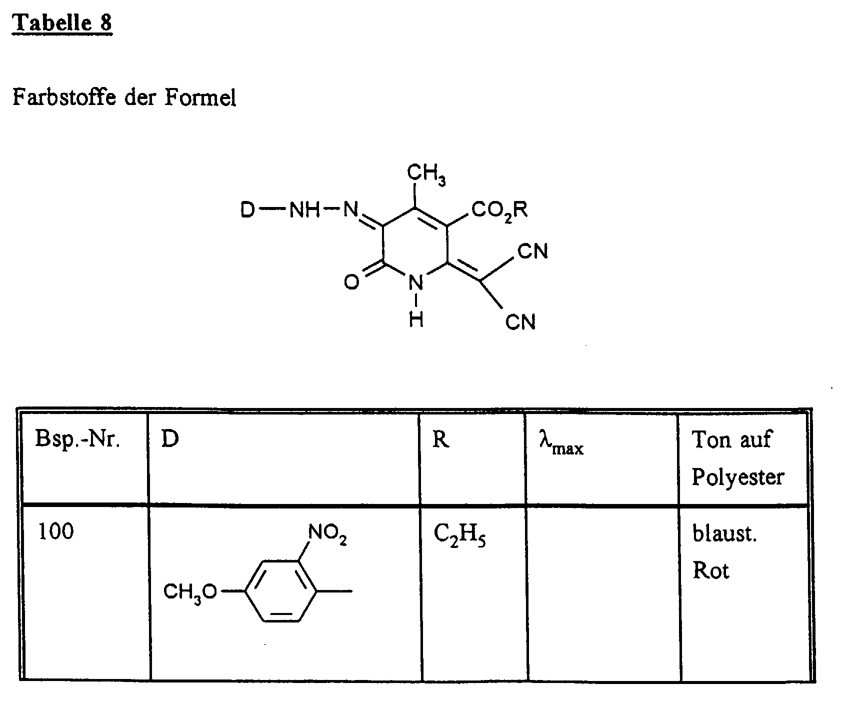

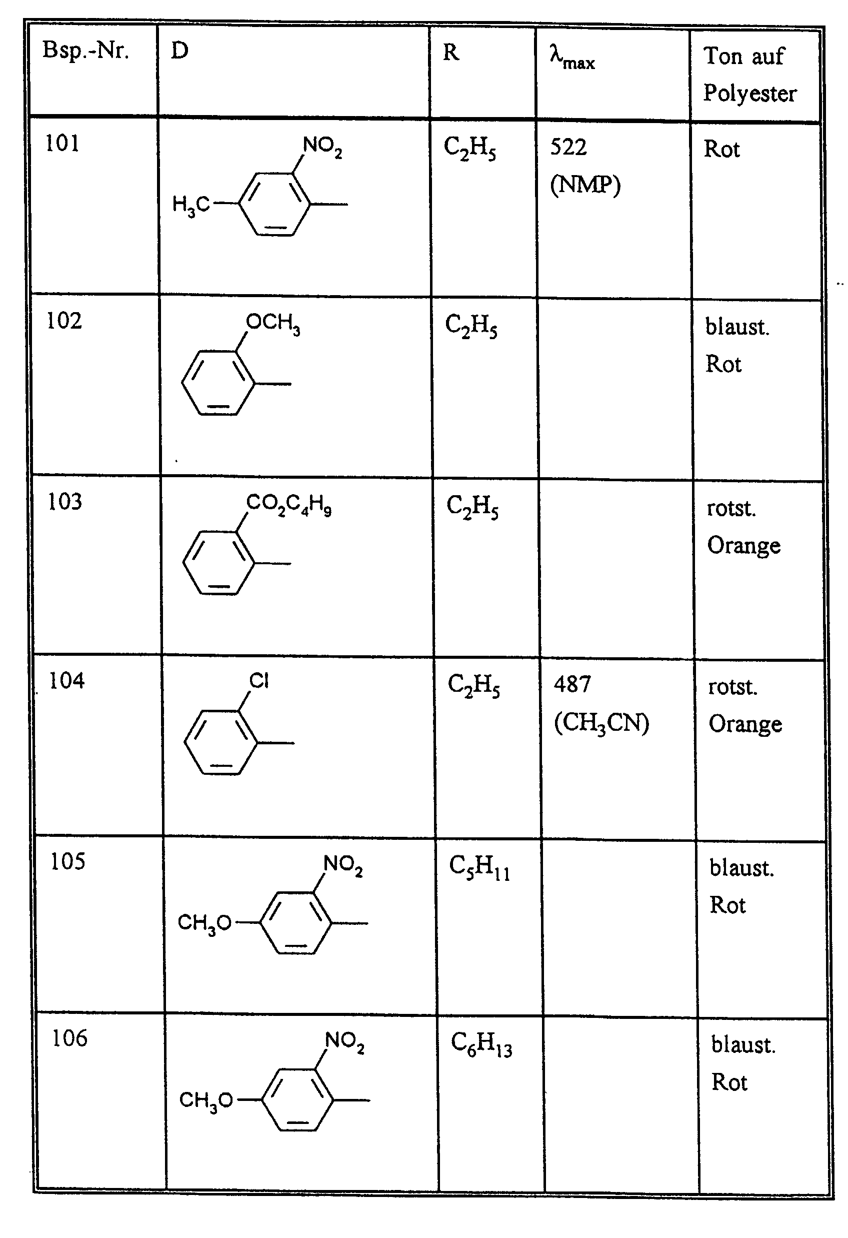

### Beispiel 107

Herstellung des Kupplers der Formel

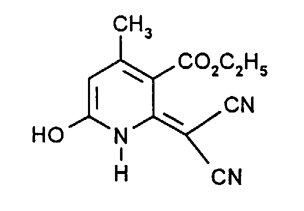

Die Herstellung verlief analog Beispiel 66 durch Umsetzung von 3-Amino-4,4-dicyan-3-butensäure-ethylester (Mittelbach M. und Junek H., Liebigs Ann. Chem. 1986, 533-544) mit Acetessigsäureethylester.

### Beispiel 108

Herstellung des Gemischs der Kuppler der Formel

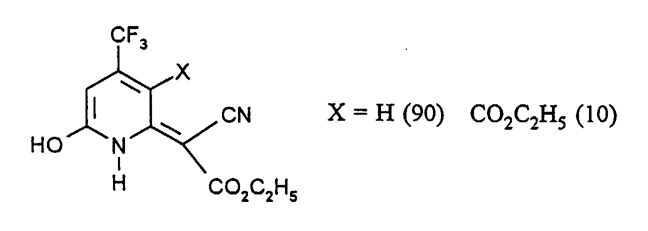

0,25 mol 3-Amino-2-cyano-pentendisäurediethylester ("Dimerer Cyanessigsäureethylester"), 0,35 mol Trifluoracetessigsäuremethylester und 0,25 mol Diazabicycloundecen wurden in 250 ml DMF 12 Stunden auf 120°C erhitzt. Die Lösung wurde mit DMF auf 400 ml aufgefüllt und direkt zur Kupplung eingesetzt. Eine eingeengte Probe ergab folgendes Massenspektrum.
MS, m/z (%): 346 (1) [M^{+.} X = CO₂C₂H₅], 301 (2), 275 (3), 274 (9) [M^{+.} X = H], 255 (7), 246 (5), 203 (11), 202 (89), 184 (14), 173 (20), 152 (44), 123 (27), 96 (27), 29 (100)

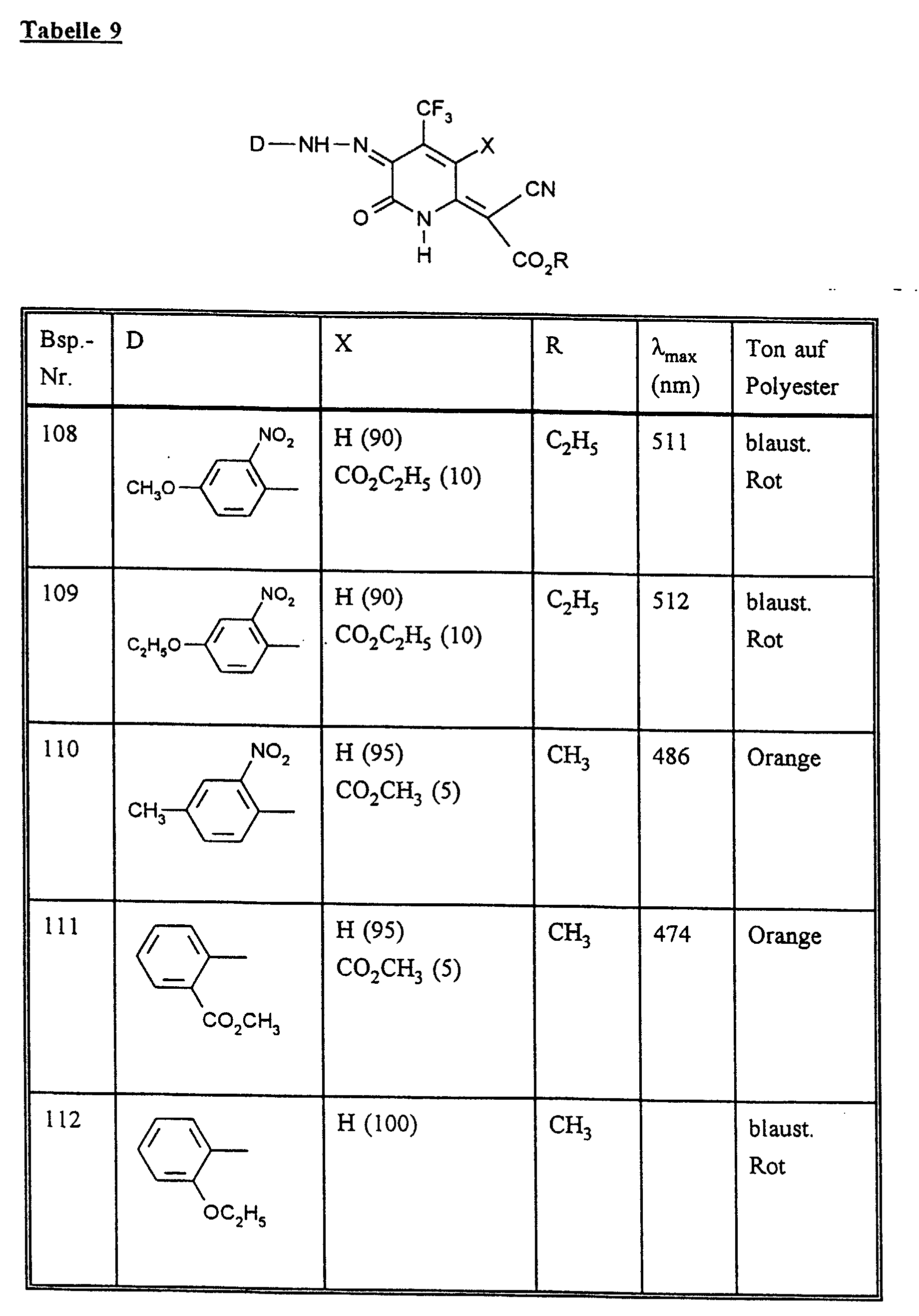

MS, m/z (%): 409 (24), 408 (100) [M^{+.}], 275 (6), 135 (12), 120 (19), 108 (27), 93 (17), 65 (11), 58 (17)
¹H-NMR (d₆-DMF, 300 MHz, ppm): δ = 1,5 (t, 3H, OCH₂-CH₃), 3,94 (s,3H,-OCH₃), 4,3 (q,2H, OCH₂-CH₃), 7,04 (s,1H, hetaromat. H), 7,18 (m,1H, aromat. H), 7,30 (m,2H, aromat. H), 7,70 (d,1H, aromat. H), 11,95 (s/br), 1H, N-H), 14,75 (s, 1H, OH oder NH).

### Beispiel 113

Herstellung des Farbstoffs der Formel

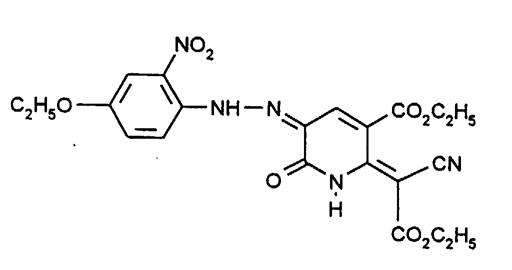

Eine Lösung von 18,6 g (2'-Nitro-4'-ethoxy)-2-phenylazo-formylessigsäureethylester, 13,6 g 3-Amino-2-cyanopentendisäurediethylester und 9,1 g Diazabicycloundecen (DBU) in 60 ml DMF wurde 30 Minuten auf 80°C erwärmt. Man verdünnte mit 120 ml Ethanol, 7,3 g 30%ige Salzsäure und 18 ml Wasser. Der entstandene Niederschlag wurde abgesaugt und mit Ethanol gewaschen. Der Farbstoff färbt Polyester in einem blaustichigen Rot mit guten Echtheiten. UV: 520 nm (CH₃CN), 538 nm (DMF)

### Beispiel 114

Herstellung des Kupplers der Formel

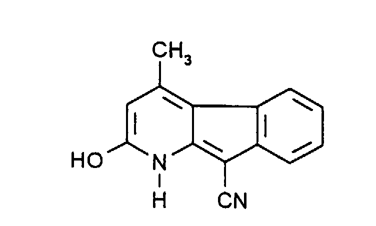

Eine Lösung von 7,8 g 2-Amino-3-cyaninden, 6,8 g Natriumethylat und 7,8 g Acetessigsäureethylester in 100 ml Ethanol wurde 7 Stunden zum Sieden unter Rückfluß erwärmt. Man saugte ab, löste den Rückstand in Wasser, stellte mit Salzsäure auf pH = 1 und saugte wiederum ab.
MS, m/z (%): 222 (100) [M^{+.}], 221 (5), 194 (6), 193 (10), 179 (7), 166 (7), 140 (4), 139 (4), 84 (8), 67 (5), 38 (5)

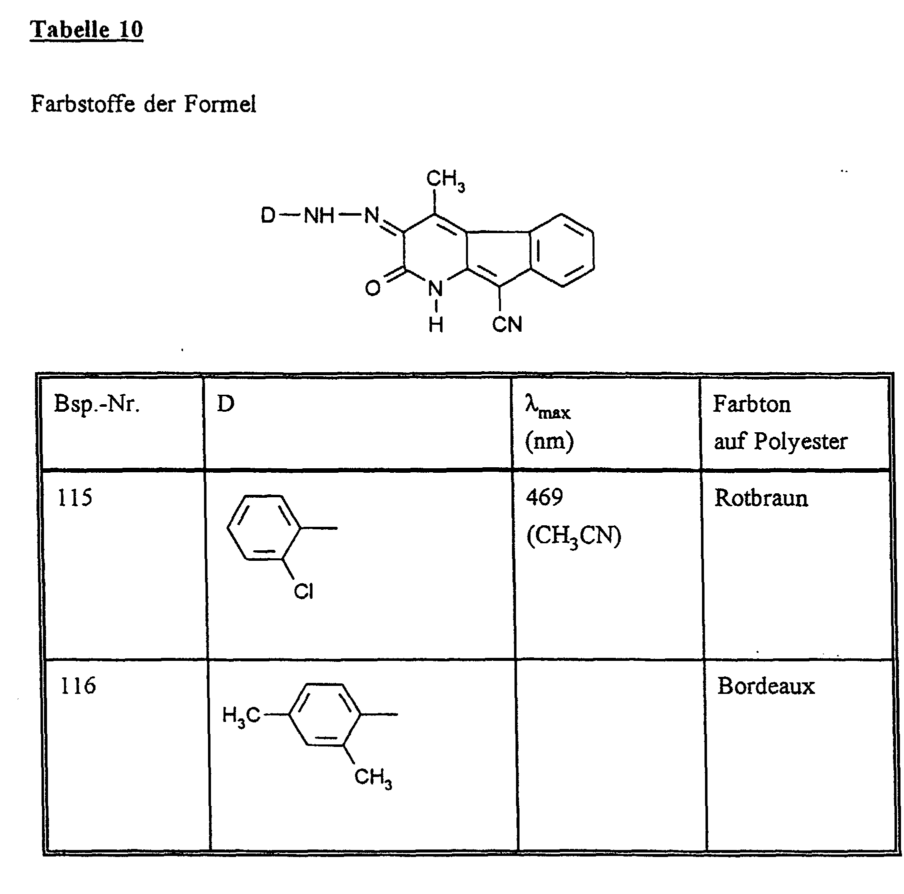

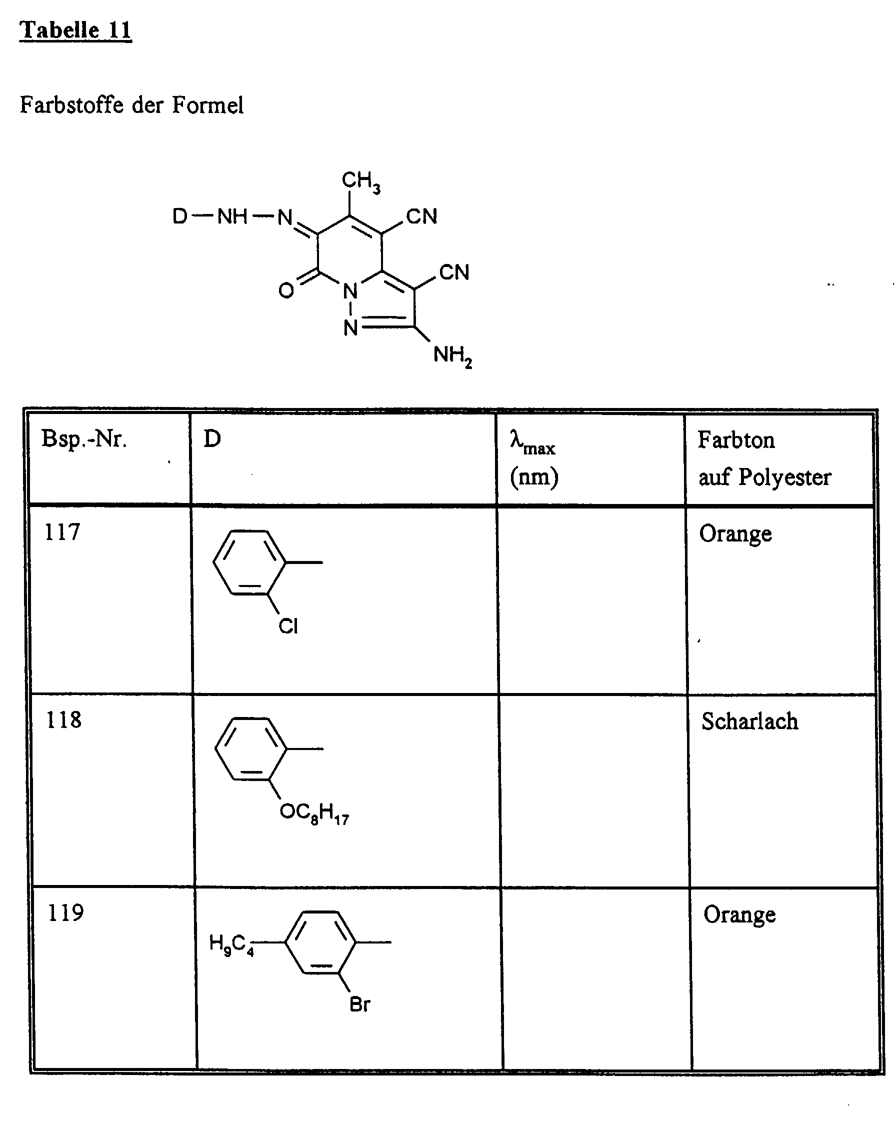

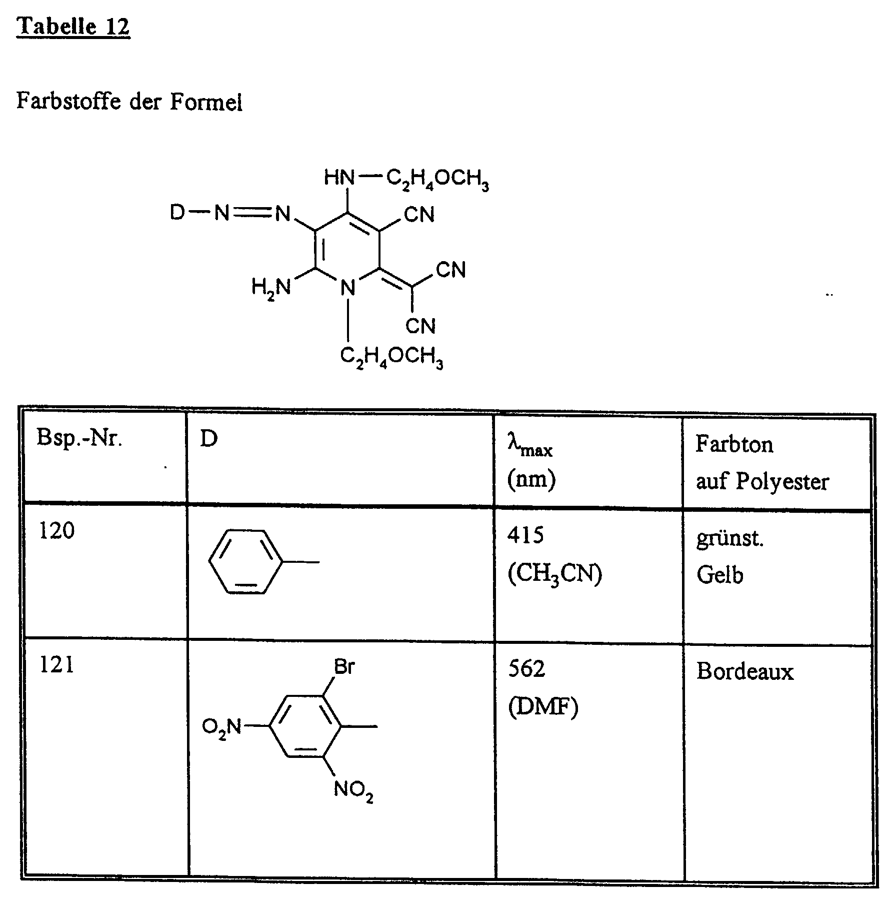

### Beispiel 122

Herstellung des Kupplers der Formel

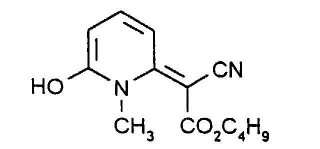

Zu einer Suspension von 1,8 g 80%igem Natriumhydrid in 30 ml DMF fügte man 4,3 g 1-Methyl-6-chlor-pyridon-2 und tropfte 8,5 g Cyanessigsäurebutylester in 15 Minuten zu. Die Temperatur stieg auf 50°C. Man rührte weitere 7 Stunden bei 50°C, kühlte auf 20°C ab und verdünnte langsam mit Wasser auf ein Volumen von 200 ml. Man stellte mit Salzsäure auf pH = 7 und extrahierte mit Methylenchlorid. Dieser Extrakt wurde verworfen. Die wässrige Phase wurde auf pH = 4 gestellt und erneut mit Methylenchlorid extrahiert. Dieser Extrakt wurde getrocknet und eingeengt. Ausbeute: 2,0 g Öl.
MS, m/z (%): 249 (8), 248 (43) [M^{+.}], 151 (12), 148 (100), 147 (11), 121 (16), 119 (22), 108 (20), 57 (74), 63 (41)

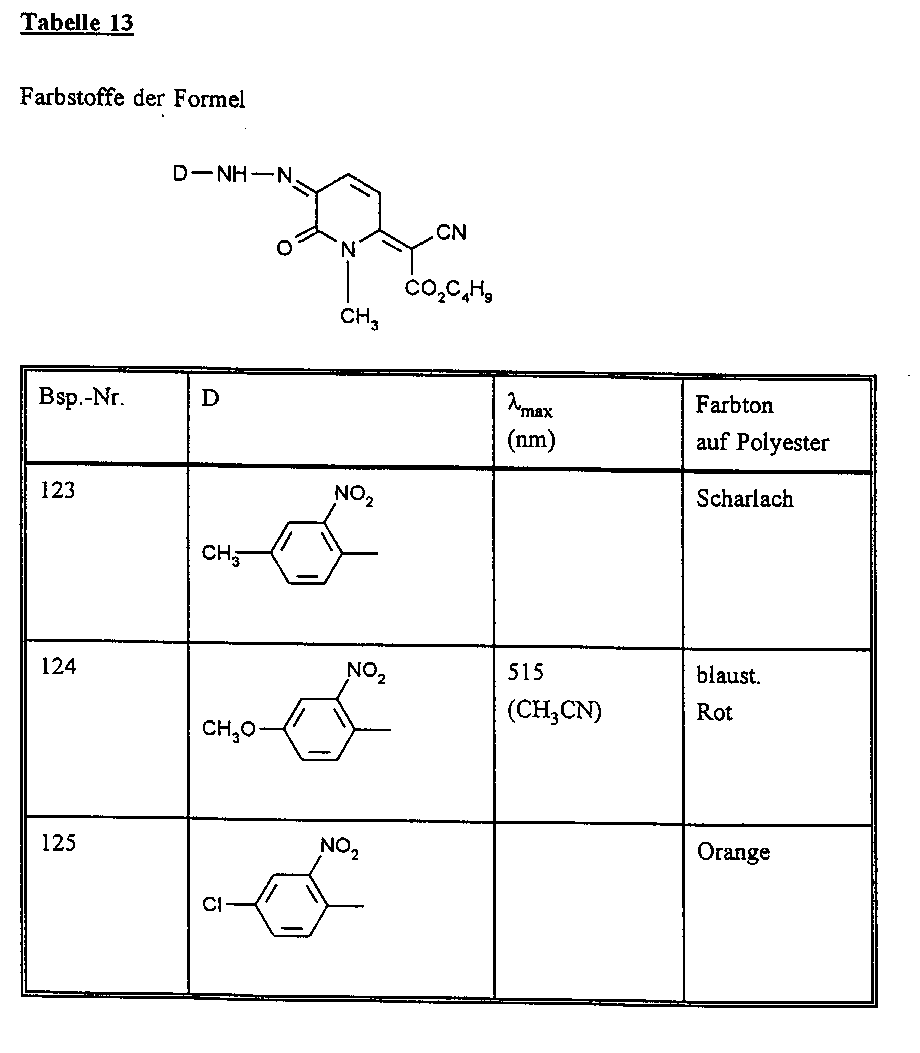

### Beispiel 126

Herstellung des Kupplers der Formel

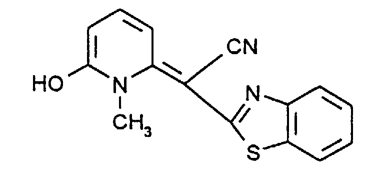

Zu einer Lösung von 5 g Natriumhydroxidpulver in 125 ml DMF fügte man 21,8 g 2-Cyanmethyl-benzthiazol und 18 g 6-Chlor-1-methyl-pyridon-2 und rührte 6 Stunden bei 40°C. Man setzte nochmals 1,8 g 6-Chlor-1-methyl-pyridon-2 nach und rührte weitere 8 Stunden bei 40°C. Man setzt 300 ml Methylenchlorid zu und trug auf 400 ml Wasser aus. Man saugte 5,5 g eines kristallinen Rohprodukts ab, verwarf die Methylenchloridphase und stellte die wässrige Phase auf pH = 5, worauf nochmals ein Niederschlag (B) ausfiel, der abgesaugt und mit Wasser gewaschen wurde: 7,8 g Ausbeute (B).
MS, m/z (%): 282 (27), 281 (100) [M^{+.}], 241 (20), 148 (91), 108 (50), 39 (51)

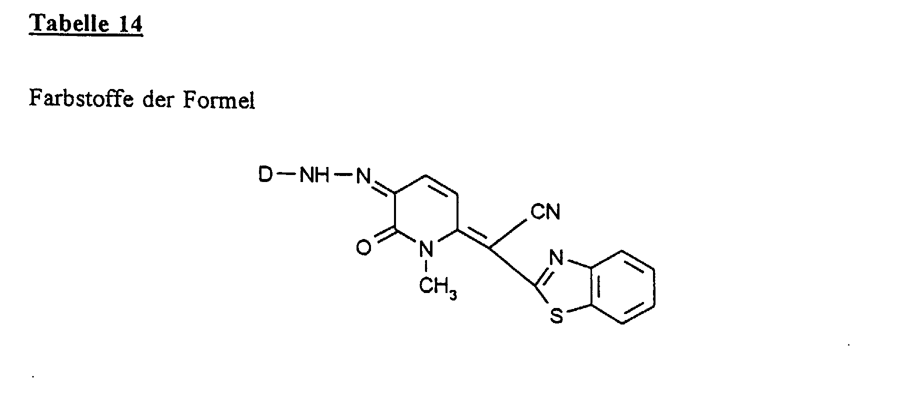

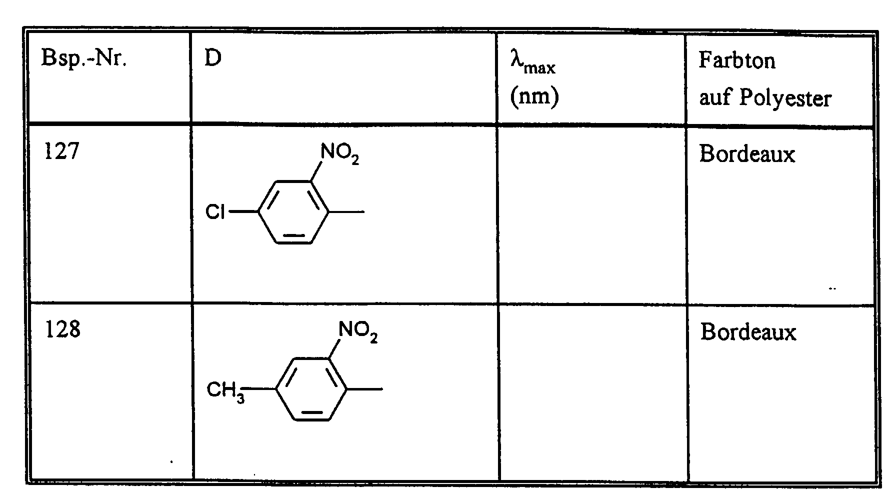

### Beispiel 129

Herstellung des Kupplers der Formel

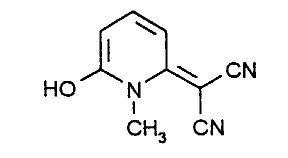

Eine Mischung von 37,4 g 2,6-Dichlorpyridin und 47,5 ml Dimethylsulfat wurde 24 Stunden bei 100°C gerührt. Nach Abkühlen verdünnte man mit 75 ml DMF und tropfte unter Eiskühlung eine Lösung von 16,5 g Malonitril in 25 ml DMF und anschließend 86,6 ml Triethylamin zu. Man ließ 20 Stunden nachrühren und saugte dann 14,7 g gelbes Produkt der Formel 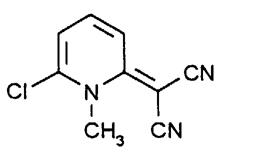 ab. Durch Verdünnen des Filtrats mit Eiswasser und Ansäuern auf pH = 1 ließen sich weitere 4 g gewinnen.
MS (CI), m/z (%): 194 (35), 192 (100) [M^{+.}], 158 (32)

76,6 g [1-Methyl-6-chlor-2(1)-pyridinyliden]-malonitril wurden in 400 ml Wasser und 400 ml NMP 10 Stunden bei 80°C und 5 Stunden bei 90°C gerührt, wobei durch Zutropfen von 30%iger Natronlauge über einen Titrator pH = 10 gehalten wurde. Man füllte mit Wasser auf ein Volumen von 1200 ml auf, stellte mit konzentrierter Salzsäure auf pH = 1, saugte ab und wusch mit Wasser. Ausbeute: 63,5 g.
MS, m/z (%): 173 (100) [M^{+.}], 146 (25), 145 (37), 144 (37), 130 (14), 119 (25), 118 (33), 108 (45), 39 (58).
¹H-NMR (d₆-DMSO, 300 MHz, ppm): δ = 3,73 (s,3H, N-CH₃), 6,15 (d, I = 7,5 Hz, 1H, 5-H), 6,53 (d, I = 7,5 Hz, 1H, 3-H), 7,48 (dd, I = 7,5 Hz, 1H, 4-H), 11,80 (s(br), OH, H₂O).

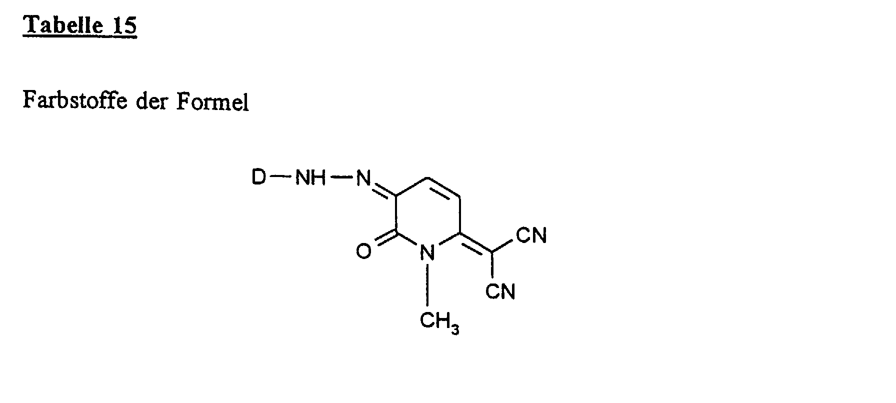

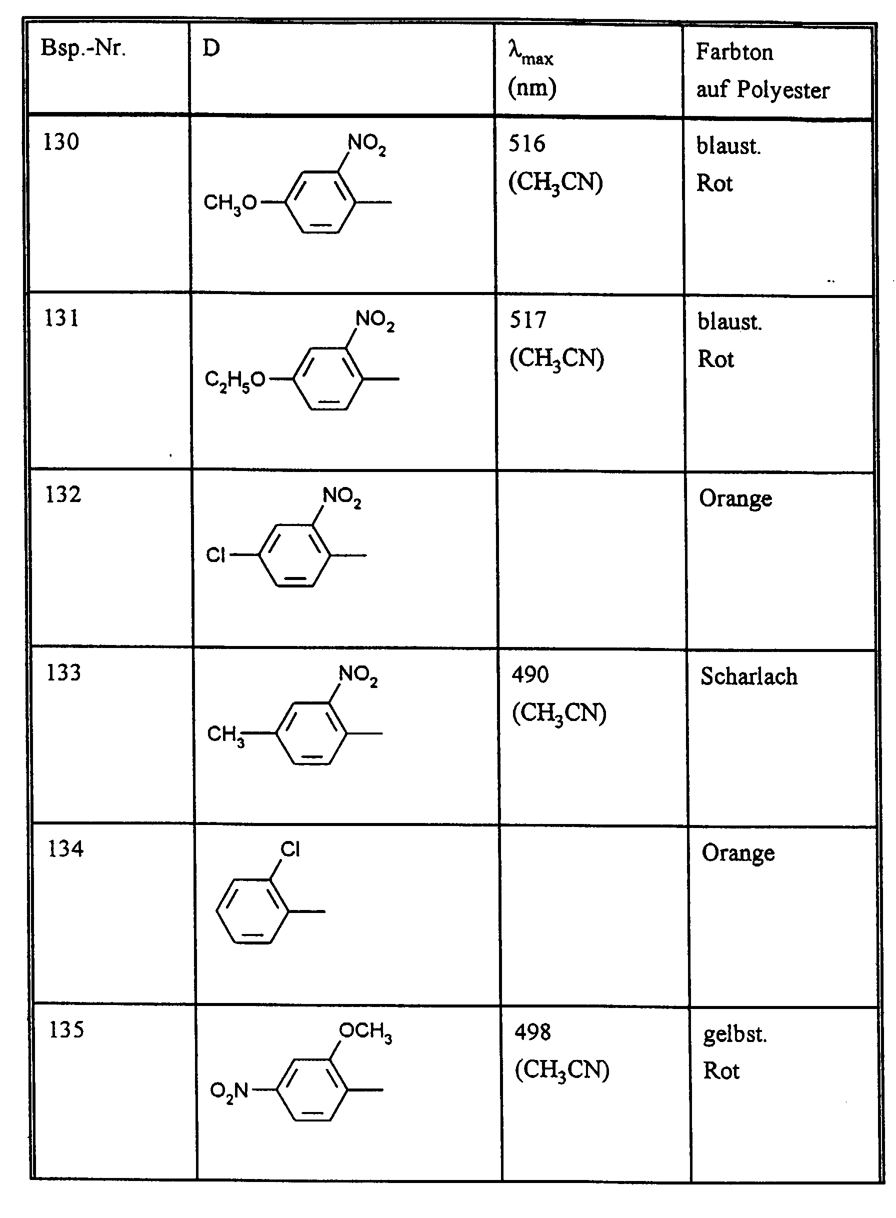

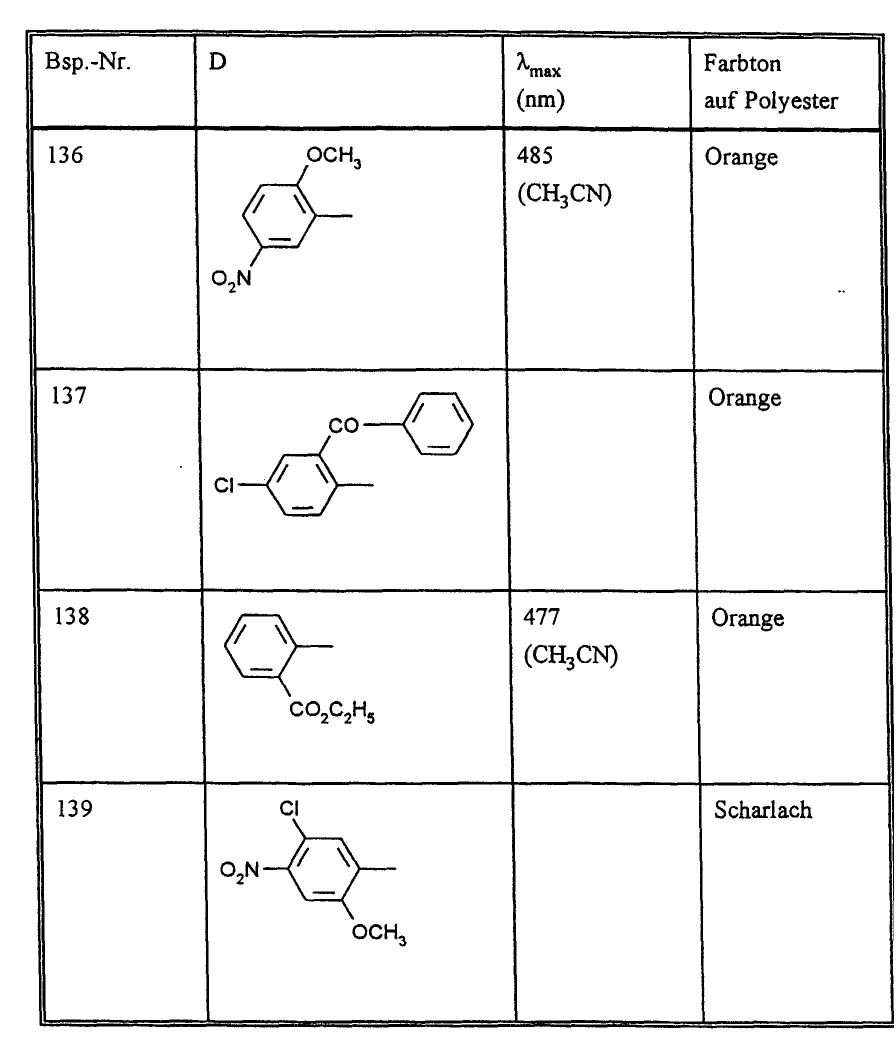

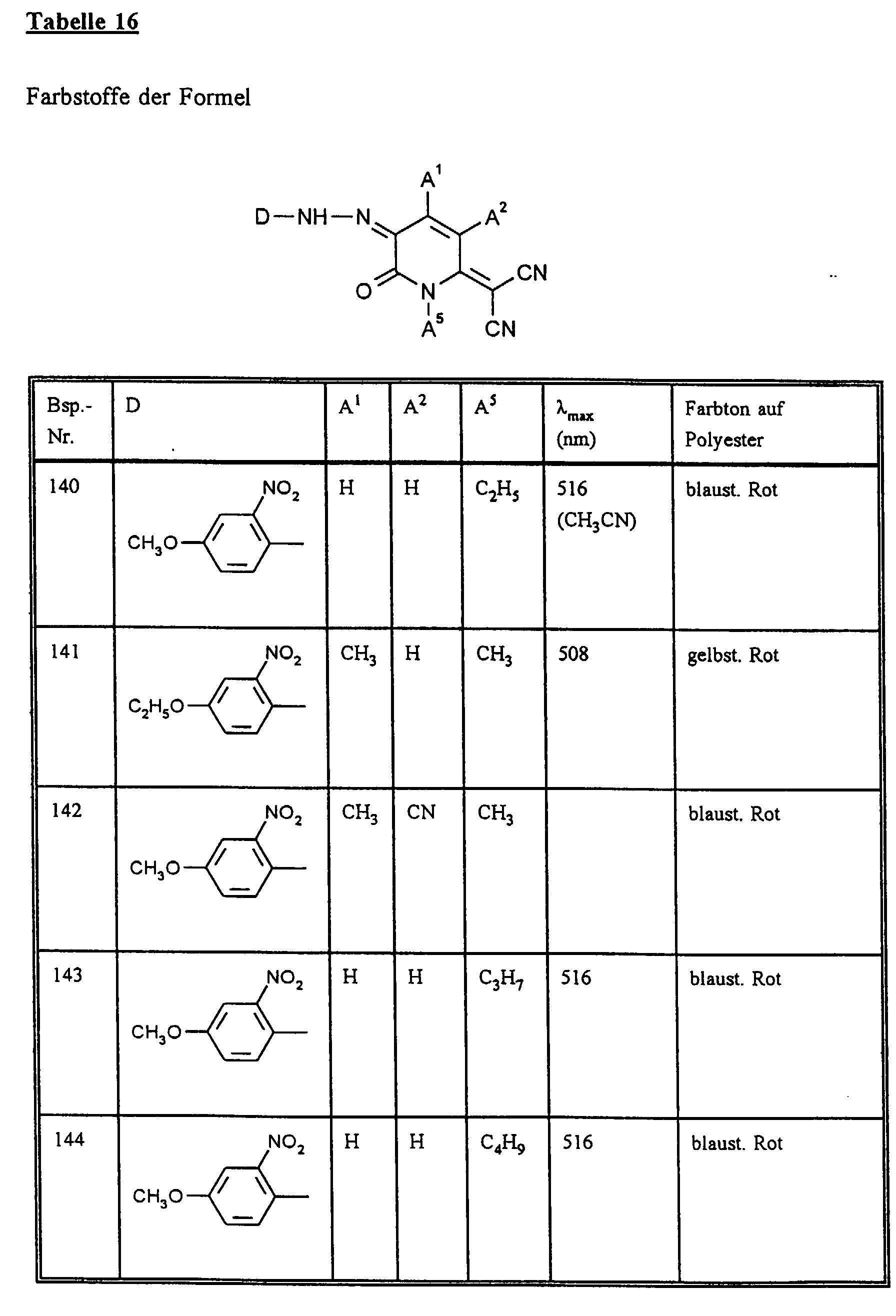

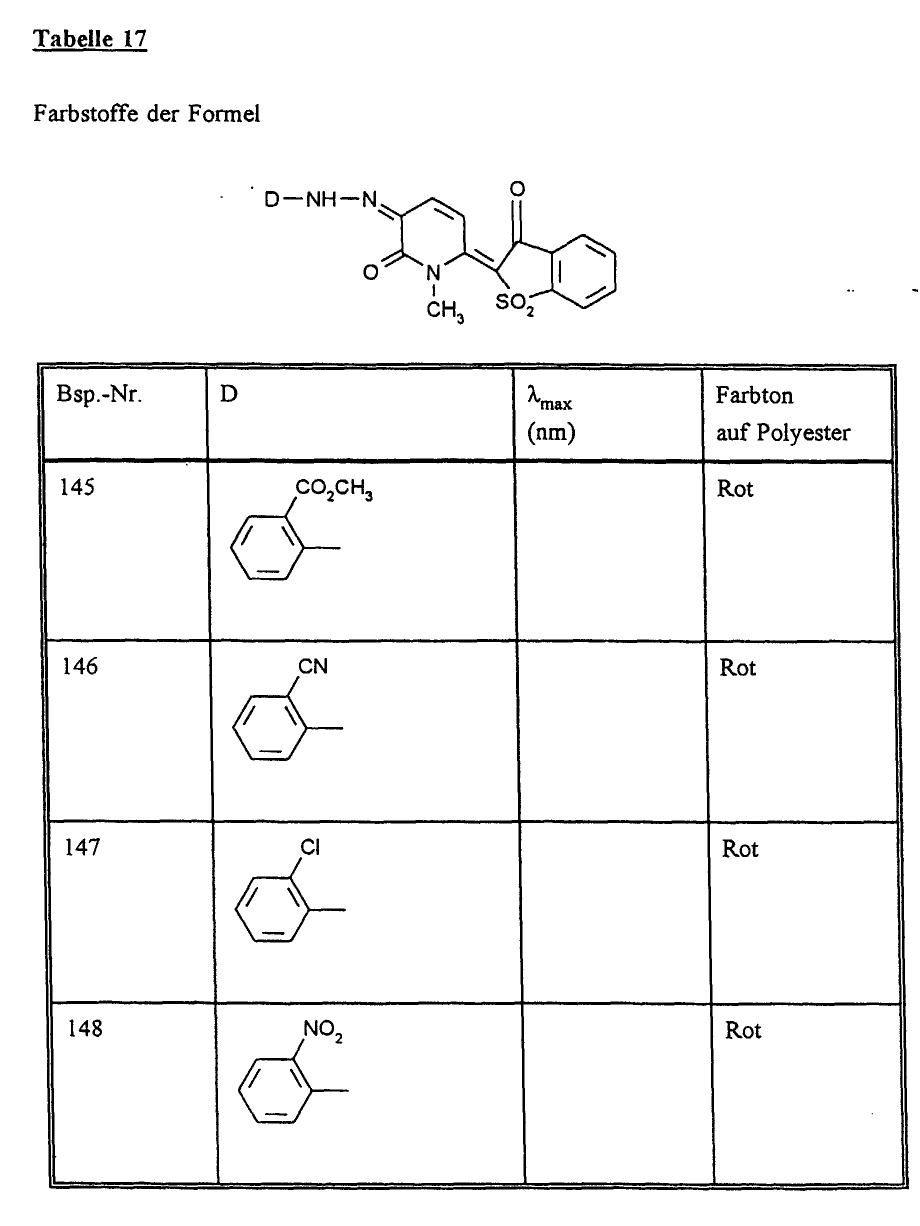

### Beispiel 149

Herstellung eines Kupplers

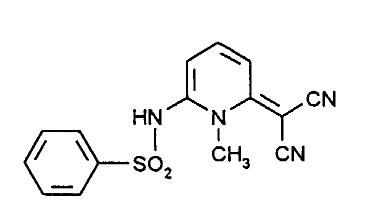

Eine Lösung von 5,4 g Natriummethylat in 120 ml Methanol wurde mit 15,7 g Benzolsulfonamid versetzt und zwei Stunden unter Rückfluß gekocht, wobei das Benzolsulfonamid in Lösung ging. Man engte zur Trockne ein, löste in 75 ml NMP und ließ eine Lösung von 19,2 g [1-Methyl-6-chlor-2(1H)-pyridinyliden]-malonitril (siehe Beispiel 129) in 75 ml NMP zulaufen. Nach 20 Stunden bei Raumtemperatur setzte man nochmals 2,7 g Natriummethylat und 3,9 g Benzolsulfonamid zu, erwärmte 5 Stunden auf 50°C und rührte 60 Stunden bei Raumtemperatur. Man verdünnte mit 600 ml Eiswasser, stellte mit Salzsäure auf pH = 1, saugte und wusch mit Wasser. Ausbeute 15 g.
¹H-NMR (d₆-DMSO, 300 MHz, ppm): δ = 3,75 (s,3H, N-CH₃), 6,38 (d, I = 7,5 Hz, 1H, heterocycl. H), 6,45 (d, I = 7,5 Hz, 1H, heterocycl. H), 7,30 (dd, I = 7,5 Hz, 1H, heterocycl. H), 7,53 (m, 3H, aromat. H), 7,78 (m, 2H, aromat. H), 9,48 (s(br), 1H, N-H).

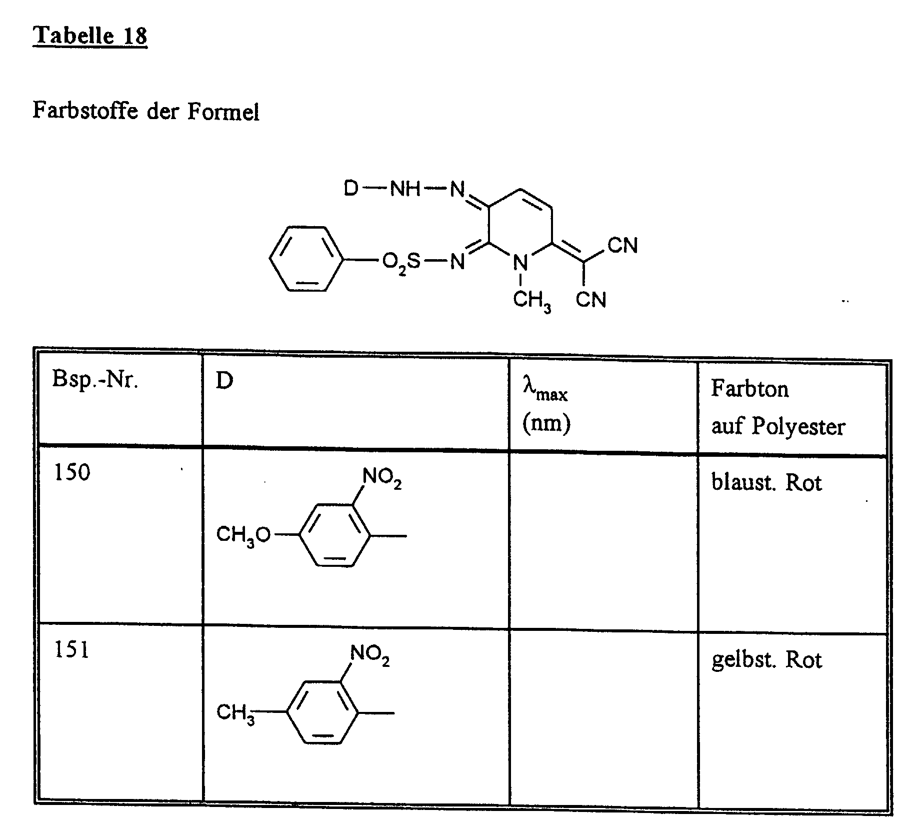

### Beispiel 152

10 g Polyestergewebe werden bei einer Temperatur von 60°C in 200 ml einer Färbeflotte gegeben, die 0,3 % feindispergierten Farbstoffs des Beispiels Nr. 134, bezogen auf das Polyestergewebe, enthält, und deren pH-Wert mittels Essigsäure auf 4,5 eingestellt ist. Man behandelt 5 Minuten bei 60°C, steigert dann die Temperatur der Flotte innerhalb von 30 Minuten auf 135°C, hält 60 Minuten bei dieser Temperatur und läßt dann innerhalb von 20 Minuten auf 60°C abkühlen.

Danach wird das ausgefärbte Polyestergewebe reduktiv gereinigt, indem man es 15 Minuten in 200 ml einer Flotte, die 5 ml/l 32 gew.-%ige Natronlauge, 3 g/l Natriumdithionit und 1 g/l eines Anlagerungsproduktes von 48 ml Ethylenoxid an 1 Mol Ricinusöl enthält, bei 65°C behandelt. Schließlich wird das Gewebe gespült, mit verdünnter Essigsäure neutralisiert, nochmals gespült und getrocknet. Man erhält eine brillante orange Färbung mit guten Echtheiten.

## Patentansprüche

1. Verbindungen, die in Form ihrer freien Säure der Formel (I) entsprechen 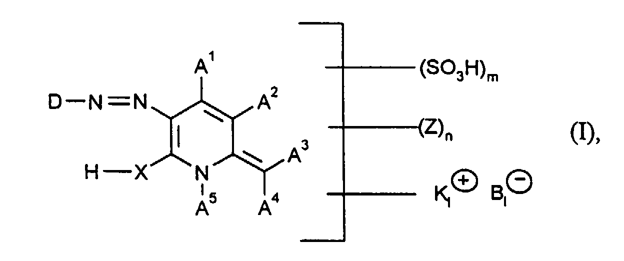 oder ihrer tautomeren Form, worin
D der Rest einer carbo- oder heterocyclische Diazokomponente ist,
A¹ und A² unabhängig voneinander H oder einen für Pyridone typische Substituenten
bedeuten,
A³ und A⁴ unabhängig voneinander einen elektronenziehenden Rest bedeuten, oder zusammen mit dem gemeinsamen C-Atom eine cyclische methylenaktive Verbindung bilden,
A⁵ für H, einen Rest der Formel T, -OT¹, -NH₂, -NHT, -NT₂, -NHCOH, -NHCOT, -N=CH-T, -N=CT₂ oder -NHSO₂T steht,
worin
T¹ = Alkyl, Cycloalkyl oder Aralkyl bedeutet und T für T¹ steht oder die Bedeutung von T² bis T⁵ annehmen kann mit
T² = Alkenyl
T³ = Alkinyl
T⁴ = Aryl
T⁵ = Hetaryl
oder
A¹ und A² und/oder
A² und A³ und/oder
A⁴ und A⁵ zusammen mit den jeweils dazwischenliegenden Atomen einen ungesättigten, gegebenenfalls substituierten 5- oder 6-gliedrigen Carbo- oder Heterocyclus bilden, mit der Maßgabe, daß bei einer Ringbildung unter Beteiligung eines der beiden Reste A³ und A⁴ der nicht beteiligte Rest einen elektronenziehenden Rest bedeutet,
X für O, NH, NT, NCOT, NCO₂T oder NSO₂T steht,
K^{⊕} -NH₃^{⊕}, -NHT₂^{⊕}, -NH₂T^{⊕}, -NT₃^{⊕} oder Cycloimmoniumion,
B^{⊖} Anion
Z ein faserreaktiver Rest ist
l für 0 bis 2,
m für 0 bis 8 und
n für 0 bis 6 steht.

2. Verbindungen gemäß Anspruch 1 der Formel (I) worin
A¹ und A² unabhängig voneinander H oder einen Rest der Formel T,-COH, -CO-T, -CO₂T, -CN, -CONH₂, -CONHT, -CONT₂, CF₃, -NH₂, -NHT, -NT₂, -NH-COT, -NT-COT, -NHSO₂T, -NTSO₂T, -NO₂, -NO, -SO₂T, -OH, -OT, -OCOT, -OCO₂T, -OSO₂T, Cl, Br, J
bedeuten, wobei
T die in Anspruch 1 angegebene Bedeutung hat.

3. Verbindungen gemäß Anspruch 1, worin 1 = 1 oder 2, n = 0 und m kleiner als 1, vorzugsweise 0 bedeuten.

4. Verbindungen gemäß Anspruch 1, worin 1 = m = n = 0 bedeuten.

5. Verbindungen gemäß Anspruch 1, worin l = 0 und m und/oder n ungleich 0 sind.

6. Verbindungen gemäß Anspruch 1, worin
A³ und A⁴ unabhängig voneinander für -CN, -CO₂T, -CONH₂, -CONHT, -CONT₂, CF₃, -CHO, -COT, -SO₂T, -SO₃T⁴, -SO₃T⁵, SO₂NH₂, SO₂NHT, SO₂NT₂, -SOT, -CH=NH, -CH=NT, -CT=NH, -CT=NT, 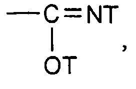 -CO-CO₂T, -NO₂, -NO, T⁴ oder T⁵
stehen, oder
A³ und A⁴ zusammen mit dem C-Atom, an das sie gebunden sind, für eine cyclische methylenaktive Verbindung der Formel (IIa) bis (IIv) stehen, wobei diese Reste in Form von 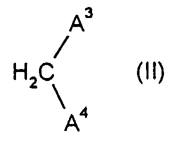 angegeben sind: 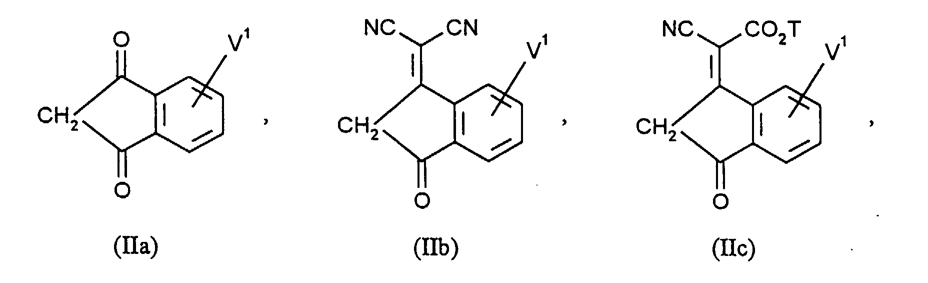 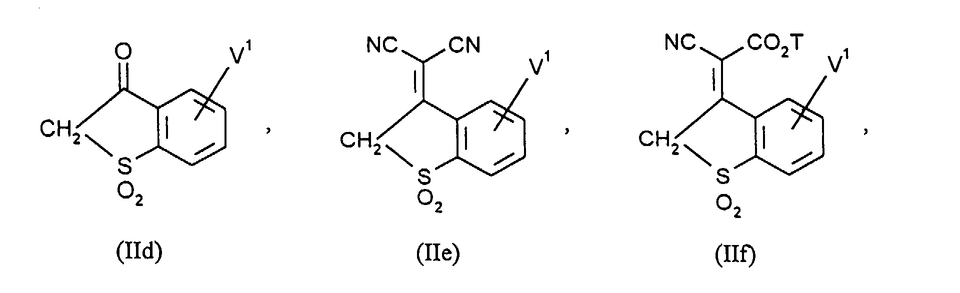 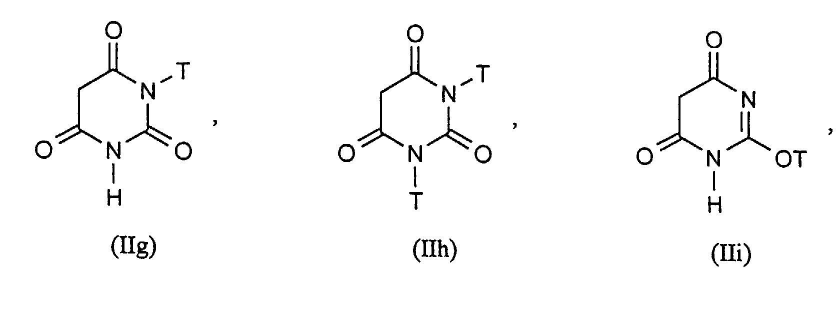 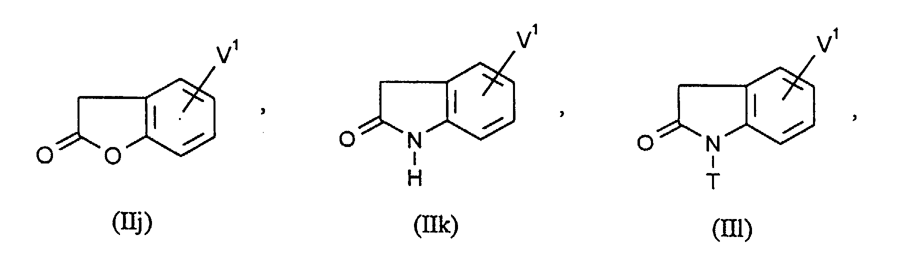 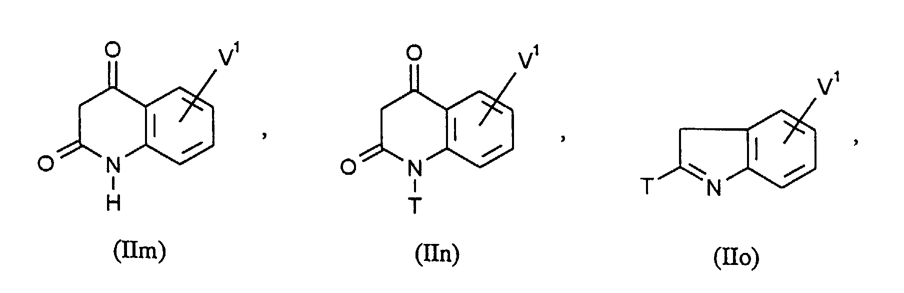 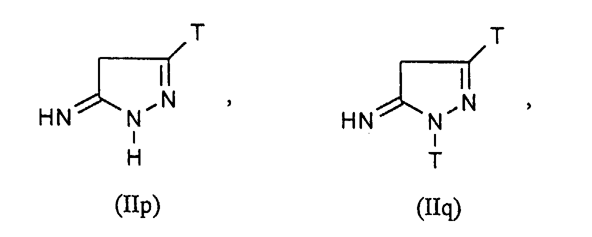 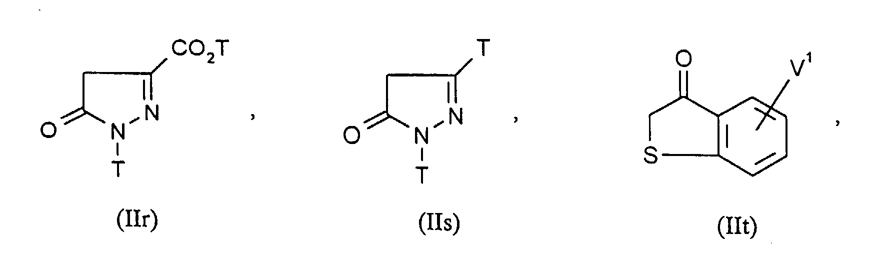 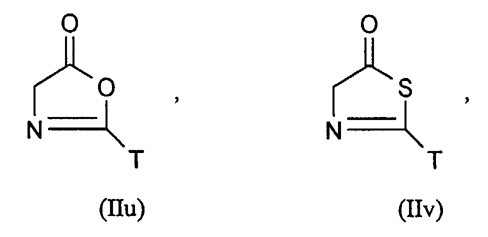
worin
V¹ für H oder einen Substituenten, insbesondere Cl, Br, CH₃, -CO₂T¹,-CN, -NO₂, -CF₃ oder -SO₂T¹ steht und worin A¹, A², A⁵, D und X die vorgenannten Bedeutungen haben.

7. Verbindungen gemäß Anspruch 4, worin
A¹ für H, T, -COH, -COT, -CO₂T, -CN, -CONH₂, -CONHT, -CONT₂, CF₃, OH oder Halogen, insbesondere Cl, Br und J steht,
A² für H, T, -COH, -COT, -CN, -CO₂T, -CONH₂, -CONHT, -CONT₂, -CF₃, -NO₂, -NO, Cl, Br, J steht,
A³ und A⁴ unabhängig voneinander für -CN, -CO₂T, -CONH₂, -CONHT,-CONT₂, CF₃, -CHO, -COT, -SO₂T, SO₃T⁴, -SO₃T⁵, SO₂NH₂, SO₂NHT, SO₂NT₂, -SOT, -CH=NH, -CH=NT, -CT=NH, -CT=NT,-CO-CO₂T, -NO₂, -NO, T⁴ oder T⁵ stehen, wobei vorzugsweise A³ und A⁴ nicht gleichzeitig für T⁴ und/oder T⁵ stehen, oder
A² und A³ zusammen mit den zwischen ihnen liegenden C-Atomen den Rest eines gegebenenfalls durch R¹ substituierten annellierten Indenringes oder
A³ und A⁴ zusammen mit dem C-Atom, an das sie gebunden sind, einen Carbo- oder Heterocyclus der Formel (IIa) bis (IIv) bilden,
A⁵ H, T¹, T², T³, T⁴, -NH₂, -NHT, NT₂, -NHCOT, -NHCOH,-NHSO₂T, -N=CT₂ bedeutet oder A⁴ und A⁵ zusammen mit den dazwischen liegenden Atomen des Pyridonringes einen annellierten Ring der Formel 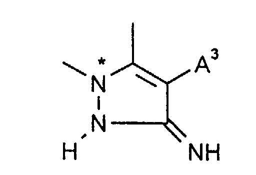 bilden, wobei das mit * markierte N-Atom dem Pyridon-Stickstoff entspricht,
T¹ C₁-C₁₀-Alkyl, C₅-C₇-Cycloalkyl oder C₆-C₁₀-Aryl-C₁-C₂-Alkyl bedeutet, die gegebenenfalls durch einen oder mehrere Substituenten aus der Gruppe -OH, -C₁-C₁₀-Alkoxy, -O[(CH₂)₂₋₁₀-O]₁₋₆-Alkyl, insbesondere -C₁-C₁₀-Alkoxy-C₂-C₅-alkoxy, -C₁-C₁₀-Alkoxy-C₂-C₅-alkoxy-C₂-C₅-alkoxy oder -O-(CH₂-CH₂O 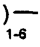 Alkyl, -OCOT, -OSO₂T, -O-(CH₂-CH₂O 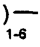 COT, -COT, -SO₂T, -CO₂T, -CN, -CO₂H, -CONT₂, -CF₃, Cl, Br, J, substituiert sind,
T², T³ unabhängig voneinander C₂-C₁₀-Alkenyl oder Alkinyl bedeuten, die gegebenenfalls durch einen oder mehrere Substituenten aus der Gruppe OH, -C₁-C₆-Alkoxy, -OCOT, -OCOH, -CO₂T, -CO₂H, -CN, Cl, Br, J substituiert sind,
T⁴ Phenyl bedeutet, das gegebenenfalls durch einen oder mehrere Substituenten wie -C₁-C₁₀-Alkoxy, 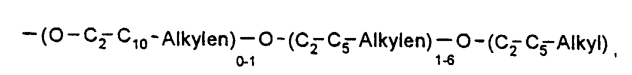 insbesondere
C₁-C₁₀-Alkoxy-C₂-C₅-alkoxy, C₁-C₅-Alkoxy-C₂-C₅-alkoxy-C₂-C₅-alkoxy, -OCOH, -OCOT, -OSO₂T, -COH, -COT, -SO₂T, -CO₂T, -CN, -CF₃, -CCl₃, -NO₂, -NO, -CO₂H, -CONH₂, -CONHT, -CONT₂, -SO₂NT², -C₁-C₁₀-Alkyl, gegebenenfalls substituiert durch -OH, -CN, -CO₂-C₁-C₆-Alkyl, Cl, Br, J, -C₂-C₁₀-Alkenyl, gegebenenfalls substituiert durch -OH, -CN, -CO₂-C₁-C₆-Alkyl, Cl, Br, J, -C₂-C₁₀-Alkinyl, gegebenenfalls substituiert durch C₁-C₁₀-Alkoxy, -OH, -OCOH, -OCOT, Cl, Br, J, substituiert ist,
T⁵ Thiophen, Furan, Pyrrol, 1,2-Isothiazol, 1,3-Thiazol, Pyrrazol, Oxazol, Isooxazol, Imidazol, Triazol, Pyridin, Pyridazin, Pyrimidin, Pyrazin, Triazin, Oxazin, Benzthiazol, Benzimidazol, Benzokazol, Chinolin, Isochinolin, Indol, Cumaron, Thionaphthen, Tetrazol, gegebenenfalls substituiert durch 1 bis 3 Substituenten, wie bereits als Substituenten für Phenyl beschrieben sind,
X O, -NCOT, -NCO₂T, -NSO₂T,
D einen Rest der Formel (IIIa) bis (IIIu) bedeutet, 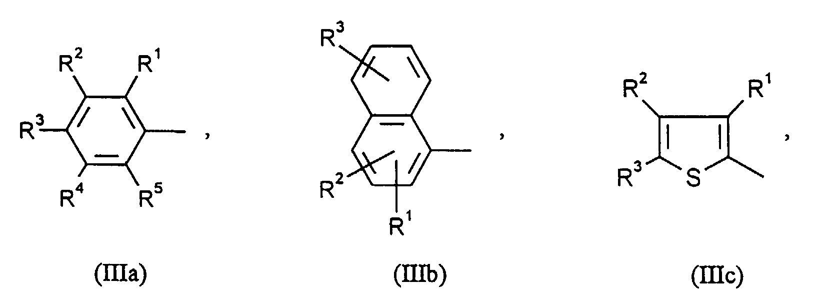 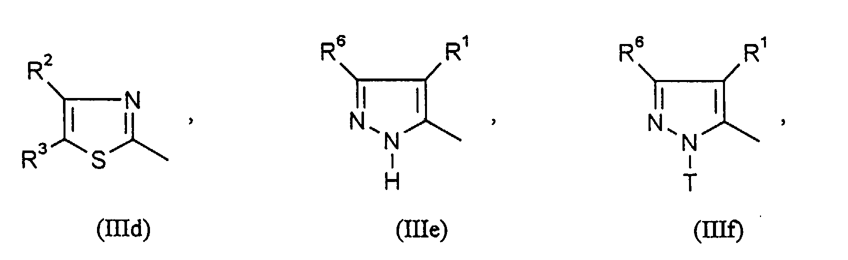 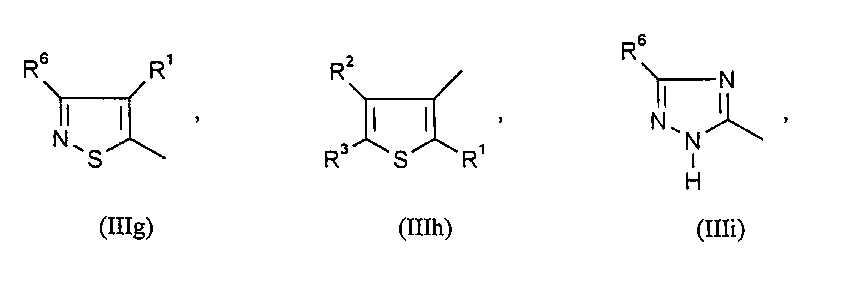 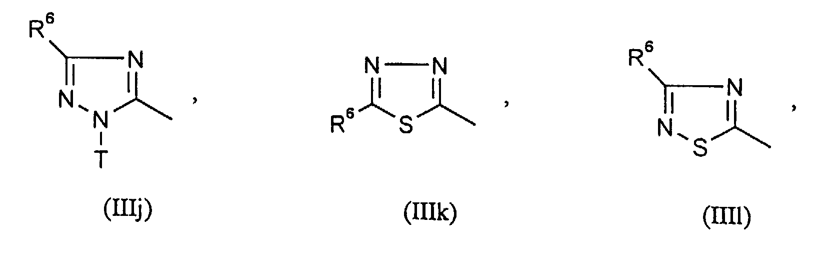 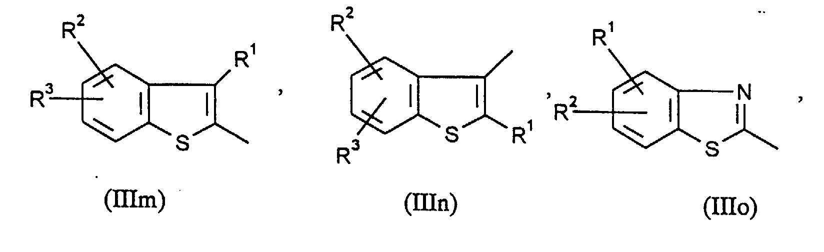 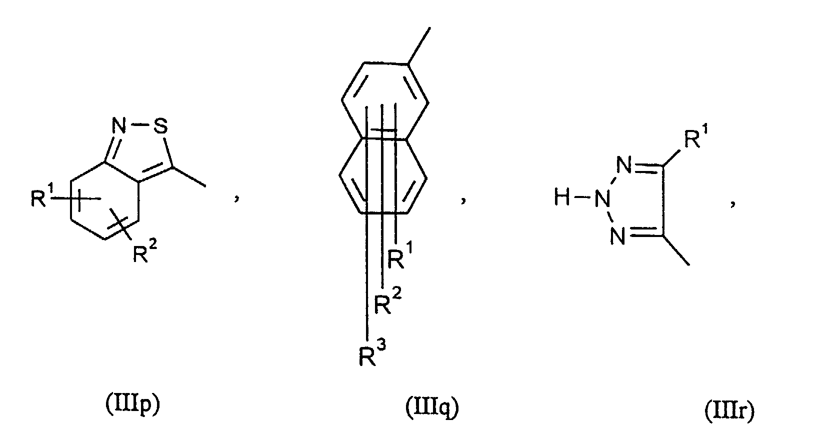 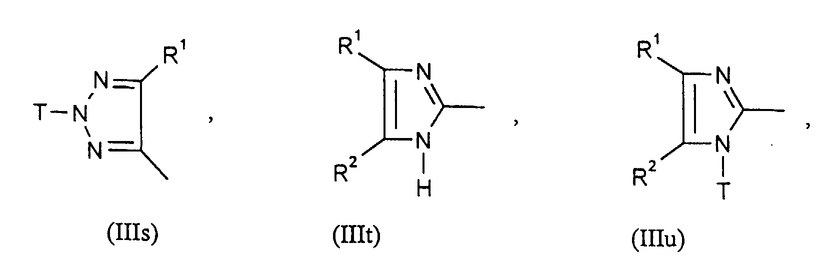
R¹, R², R³, R⁴, R⁵ und R⁶ unabhängig voneinander
H, T, F, Cl, Br, J, -CN, -NO₂, -CH=O, -COT, -CO₂T¹, -CONH₂, -CONHT, CONT₂, -CF₃, -SO₂NH₂, -SO₂NHT, -SO₂NT₂, -SOT,-SO₂T, -SO₃T, -OT, -OH, -OCOT, -OCO₂T, -OSO₂T, -NH₂, -NHT, -NT₂, 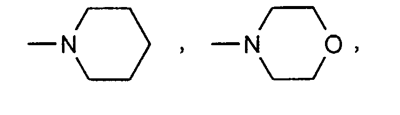 -NHCOH, -NHCOT, -NHSO₂T oder -COCO₂T bedeuten,
wobei
R³ zusätzlich -N=N-T⁴ oder -N=N-T⁵ bedeuten kann, und
R⁶ zusätzlich ST bedeuten kann.

8. Verbindungen gemäß Anspruch 4, worin
A¹ für H, T¹ oder -CF₃ steht,
A² H, -CN oder -CO₂T¹ bedeutet,
A³ für -CN oder -CO₂T¹ steht,
A⁴ A³ bedeutet, oder
A³ und A⁴ zusammen mit dem C-Atom, an das sie gebunden sind, eine cyclische methylenaktive Verbindung der Formel (IIa) oder (IId) bilden, wobei die Reste der cyclischen methylenaktiven Verbindung in Form von 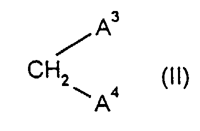 angegeben sind,
A⁵ für H, T¹ oder T² steht,
D für einen Rest der Formel 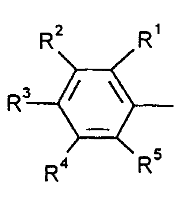 steht, worin
R¹ und R⁵ unabhängig voneinander H, Cl, Br, -CN, -NO₂, -CO₂T¹, T¹, -OT¹ oder -OT⁴ bedeuten,
R² und R⁴ unabhängig voneinander H, Cl, Br, -NO₂, T¹ oder -OT¹ bedeuten,
R³ für H, Cl, Br, -CN, -NO₂, T¹, -CO₂T¹ oder -OT¹ steht,
T¹ für C₁-C₆-Alkyl oder C₁-C₄-T⁴ steht, gegebenenfalls substituiert durch C₁-C₆-Alkoxy, -C₁-C₄-Alkoxy-C₂-C₅-alkoxy, -CO₂T¹,
T² gegebenenfalls durch Cl und/oder Br substituiertes C₂-C₆-Alkenyl bedeutet,
T⁴ für Phenyl steht, das gegebenenfalls substituiert ist durch Cl, Br, -NO₂ und/oder -CO₂T¹ und
X O bedeutet.

9. Verfahren zur Herstellung von Verbindungen gemäß Anspruch 1, dadurch gekennzeichnet, daß man Amine der Formel (IV)
D - NH₂ (IV)
diazotiert und auf Kupplungskomponenten der Formel (V) 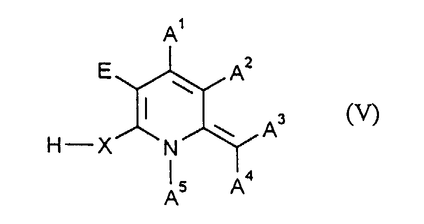 worin A¹ bis A⁵, D und X die in Anspruch 1 angegebene Bedeutung haben und
E für einen durch elektrophile Substitution verdrängbaren Substituenten, wie z.B. H, -CO₂H, -CH₂OH, -SO₃H, -CH=O, -COT, 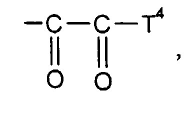 -CONH₂, -CONHT steht, kuppelt.

10. Verfahren zur Herstellung von Farbstoffen der Formel (I), worin X gleich O bedeutet, dadurch gekennzeichnet, daß Verbindungen der Formel (VII), die in den tautomeren Formen der Formel (VIIa) und (VIIb) vorliegen können, 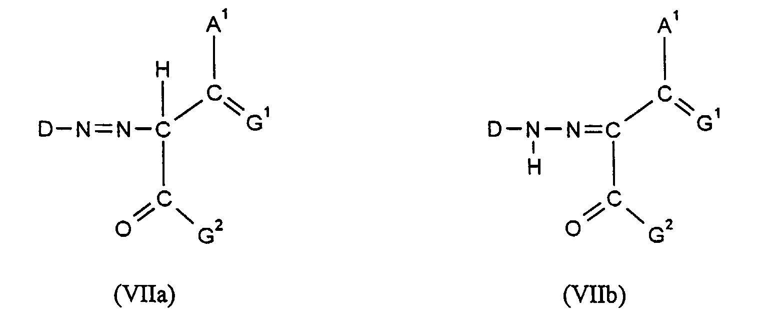 worin
G¹ O, NH oder NT, vorzugsweise O bedeutet,
G² für -OT, -NH₂, oder -NHT, vorzugsweise für OT¹ steht,
D und A¹ die vorgenannten Bedeutungen haben,
mit Enaminen der Formel (VIII) 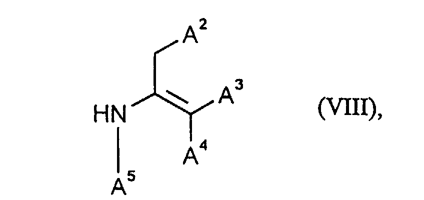 worin A² bis A⁵ die vorgenannten Bedeutungen haben, kondensiert werden.

11. Enamine der Formel (VIIIa) bis (VIIIc)

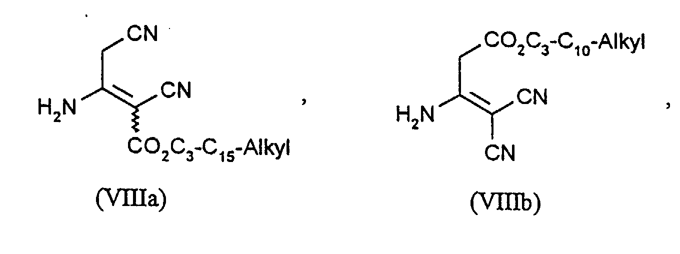

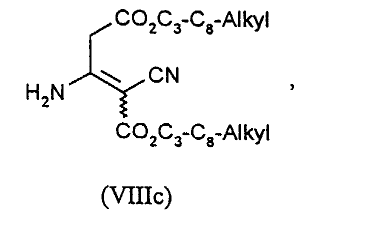

12. Verbindungen der Formel (V) 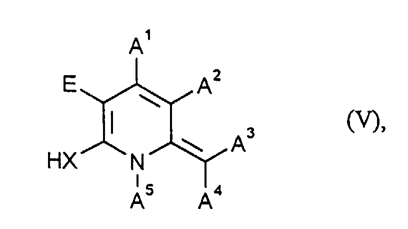 worin
E und X die in Anspruch 9, bzw. in Anspruch 1 genannten breitesten Bedeutungen haben,
A¹ und A² unabhängig voneinander H oder einen Rest der Formel T, -COH, -CO-T, -CO₂T, -CN, -CONH₂, -CONHT, -CONT₂, CF₃, -NO₂, -NO, -SO₂T, -OH, -OT, -OCOT, -OCO₂T, -OSO₂T, Cl, Br, J
bedeuten, wobei
T die Bedeutung von T¹, T², T³, T⁴ oder T⁵ annehmen kann, mit
T¹ = Alkyl, Cycloalkyl oder Aralkyl,
T² = Alkenyl,
T³ = Alkinyl,
T⁴ = Aryl,
T⁵ = Hetaryl,
A³ und A⁴ unabhängig voneinander einen elektronenziehenden Rest bedeuten, oder zusammen mit dem gemeinsamen C-Atom eine cyclische methylenaktive Verbindung bilden,
A⁵ für H, einen Rest der Formel T, -OT¹, -NH₂, -NHT, -NT₂,-NHCOH, -NHCOT, -N=CH-T, -N=CT₂ oder NHSO₂T steht oder
A¹ und A² oder
A² und A³ zusammen mit den jeweils dazwischenliegenden Atomen einen ungesättigten, gegebenenfalls substituierten 5- oder 6-gliedrigen Carbo- oder Heterocyclus bilden, wobei bei einer Ringbildung unter Beteiligung eines der Reste A³ oder A⁴ der jeweils andere einen elektronenziehenden Rest bedeutet und wobei die Reste A¹ bis A⁵ gegebenenfalls ein oder mehrere SO₃H, COOH oder K^{⊕}B^{⊖}-Gruppen tragen, worin K^{⊕} und B^{⊖} die in Anspruch 1 angegebene Bedeutung haben.

13. Verfahren zur Herstellung der Verbindungen der Formel (V) in Form der Verbindung Va bzw. Vb, worin X für O oder NH steht, dadurch gekennzeichnet, daß man Essigsäurederivate der Formel IXa bzw. IXb mit Enaminen der Formel (VIII) 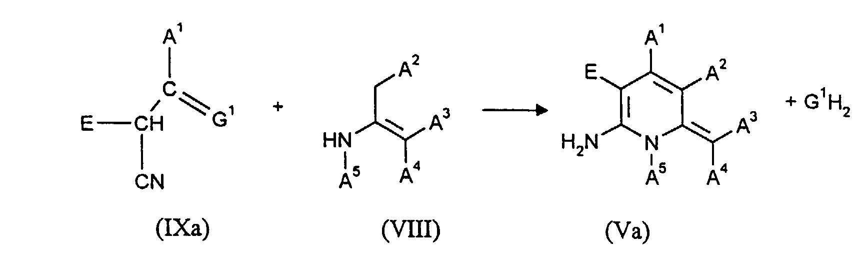 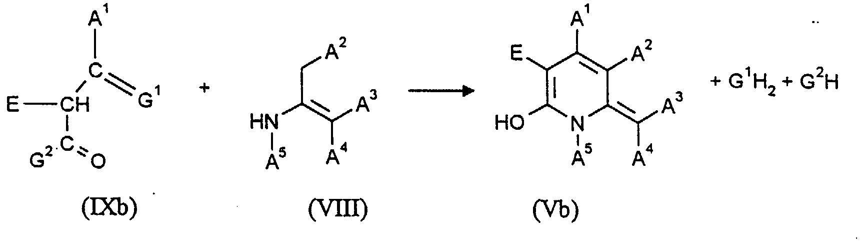 umsetzt,
worin
A¹ bis A⁵ und X die in Anspruch 1 angegebene Bedeutung haben,
E für einen durch elektrophile Substitution verdrängbaren Substituenten steht,
G¹ O, NH oder NT bedeutet,
G² für OT, NH₂ oder NHT steht, wobei T die in Anspruch 1 angegebene Bedeutung besitzt.

14. Verfahren zur Herstellung von Verbindungen der Formel V, die der Formel X entsprechen, dadurch gekennzeichnet, daß man Trifluoracetessigester der Formel (XI), mit dimeren Cyanessigsäureestern der Formel (XII), kondensiert, 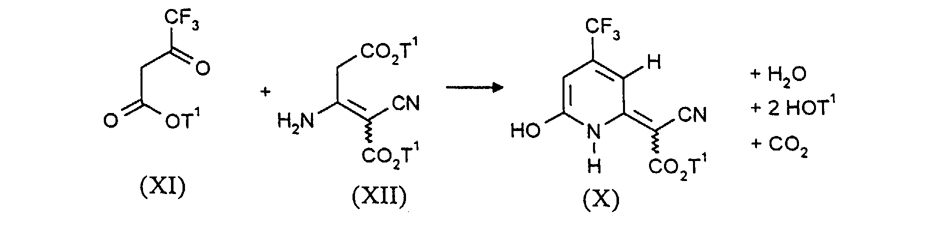 wobei T¹ die in Anspruch 1 angegebene Bedeutung hat.

15. Verfahren zur Herstellung von Verbindungen der Formel (V) 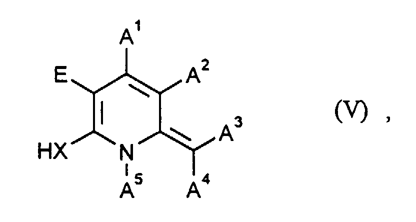 worin A⁵ für T¹, T² oder T³ steht, das dadurch gekennzeichnet ist, daß man Pyridiniumsalze der Formel (XIII) 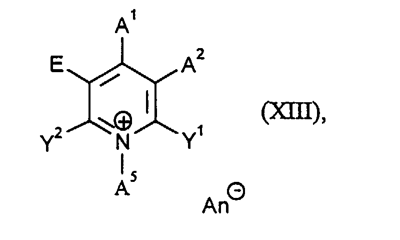 worin
A¹ und A² die in Anspruch 1 genannte Bedeutung haben,
E für einen durch elektrophile Substitution verdrängbaren Substituenten steht,
An^{⊖} ein Anion ist, das bspw, die für B^{⊖} angegebenen Bedeutungen annehmen kann, insbesondere für Cl^{⊖}, Br^{⊖} , J^{⊖,} T¹OSO₃^{⊖} oder TSO₃^{⊖} steht,
A⁵ für T¹, T² oder T³ steht,
Y¹ und Y² unabhängig voneinander für eine Abgangsgruppe wie F, Cl, Br, J, -OSO₂T, -OT, -ST oder -SO₂T stehen,
mit Verbindungen der Formel (II) 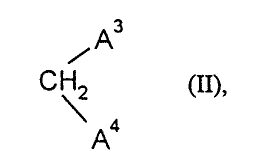 worin
A³ und A⁴ die oben genannte breiteste Bedeutung besitzen
zu Verbindungen der Formel (XIV) 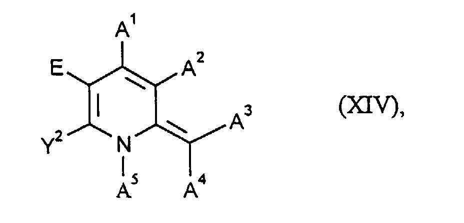 umsetzt, und diese mit Verbindungen der Formel (XV)
XH₂ (XV),
worin
X für O, NH, NT¹, NCOT, NCO₂T oder NSO₂T steht,
zu Verbindungen der Formel (V) umsetzt,
worin A⁵ für T¹, T² oder T³ steht.

16. Verfahren zur Herstellung der Kupplungskomponenten der Formel (V), worin A⁵ für T steht, dadurch gekennzeichnet, daß man Pyridinderivate der Formel (XVII) mit Verbindungen der Formel (II) umsetzt: 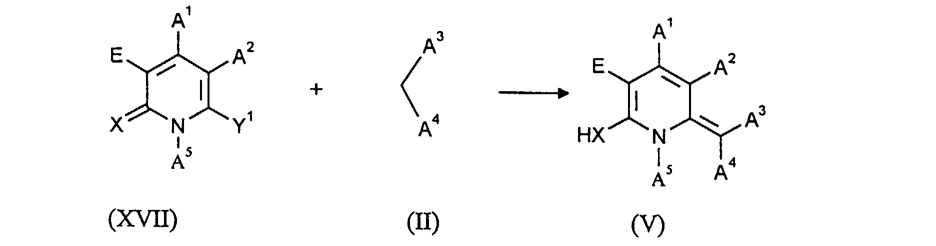 worin X=O, A¹ bis A⁴ die in Anspruch 1 genannte Bedeutung haben und A⁵ für T und
E für einen durch elektrophile Substitution verdrängbaren Substituenten steht.

17. Verfahren zur Herstellung von Verbindungen der Formel (V), die der Formel (XX) entsprechen, worin A⁵ für T¹, T² oder T³ steht, dadurch gekennzeichnet, daß man 2-Methylpyridone der Formel (XVIII) mit Phthalsäureestern der Formel (XIX) in Gegenwart von Basen umsetzt, 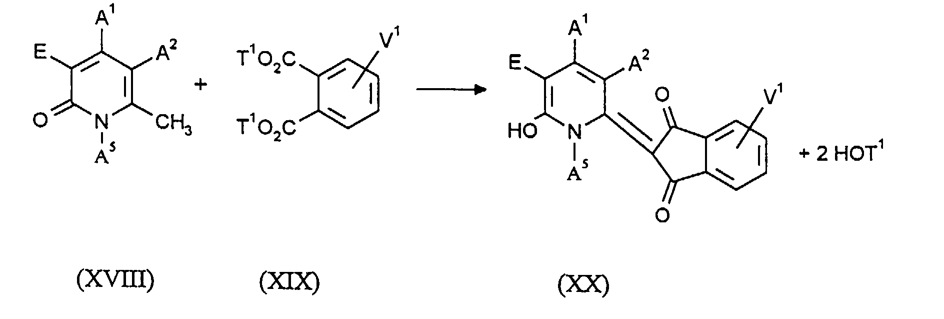 wobei A¹, A², E, T¹, V¹ die in Anspruch 8 genannte Bedeutung haben.

18. Verwendung der Farbstoffe gemäß Anspruch 1 zum Färben und Bedrucken von natürlichen und synthetischen Materialien wie beispielsweise Cellulosefasern, Baumwolle, Wolle, Seide, Polyamid, Polyacrylnitril, Polyester oder Polyolefinen.

19. Verwendung der Farbstoffe gemäß Anspruch 4 zum Färben und Bedrucken von hydrophoben synthetischen Fasermaterialien und deren Mischungen mit natürlichen Faserstoffen.

## Claims

1. Compounds which, in the form of their free acid, correspond to the formula (I) 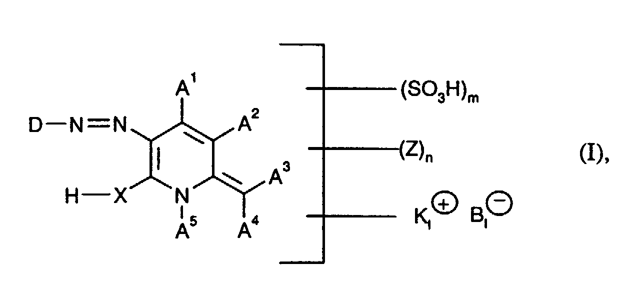 or its tautomeric form, wherein
D is the radical of a carbo- or heterocyclic diazo component,
A¹ and A² independently of one another denote H or a substituent typical for pyridone,
A³ and A⁴ independently of one another denote an electron-withdrawing radical, or, together with the common C atom, form a cyclic methylene-active compound,
A⁵ represents H or a radical of the formula T, -OT¹, -NH₂, -NHT, -NT₂, -NHCOH, -NHCOT, -N=CH-T, -N=CT₂ or -NHSO₂T,
wherein
T¹ = alkyl, cycloalkyl or aralkyl, and T represents T' or can assume the meaning of T² to T⁵, where
T² = alkenyl,
T³ = alkinyl,
T⁴ = aryl,
T⁵ = hetaryl
or
A¹ and A¹ and/or
A² and A³ and/or
A⁴ and A⁵, together with the particular atoms in between, form an unsaturated, optionally substituted 5- or 6-membered carbo- or heterocyclic radical, with the proviso that if a ring is formed with participation of one of the two radicals A³ and A⁴, the radical which does not participate denotes an electron-withdrawing radical,
X represents O, NH, NT, NCOT, NCO₂T or NSO₂T,
K^{⊕} is -NH₃^{⊕}, -NHT₂^{⊕}, -NH₂T^{⊕}, -NT₃^{⊕} or a cycloimmonium ion,
B^{⊖} is an anion,
Z is a fibre-reactive radical,
l represents 0 to 2,
m represents 0 to 8 and
n represents 0 to 6.

2. Compounds according to Claim 1 of the formula (I), wherein
A¹ and A² independently of one another denote H or a radical of the formula T, -COH, -CO-T, -CO₂T, -CN, -CONH₂, -CONHT, -CONT₂, CF₃,-NH₂, -NHT, -NT₂, -NH-COT, -NT-COT, -NHSO₂T, -NTSO₂T, -NO₂, -NO, -SO₂T, -OH, -OT, -OCOT, -OCO₂T, -OSO₂T, Cl, Br or I
wherein
T has the meaning given in Claim 1.

3. Compounds according to Claim 1, wherein 1 = 1 or 2, n = 0 and m is less than 1, preferably 0.

4. Compounds according to Claim 1, wherein 1 = m = n = 0.

5. Compounds according to Claim 1, wherein 1 = 0 and m and/or n are not equal to 0.

6. Compounds according to Claim 1, wherein
A³ and A⁴ independently of one another represent -CN, -CO₂T, -CONH₂, -CONHT, -CONT₂, CF₃, -CHO, -COT, -SO₂T, -SO₃T⁴, -SO₃T⁵, SO₂NH₂, SO₂NHT, SO₂NT₂, -SOT, -CH=NH, -CH=NT, -CT=NH, -CT=NT, 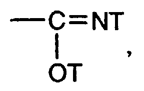 -CO-CO₂T, -NO₂, -NO, T⁴ or T⁵,
or
A³ and A⁴, together with the C atom to which they are bonded, represent a cyclic methylene-active compound of the formula (IIa) to (IIv), where these radicals are shown in the form of 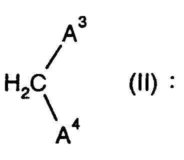 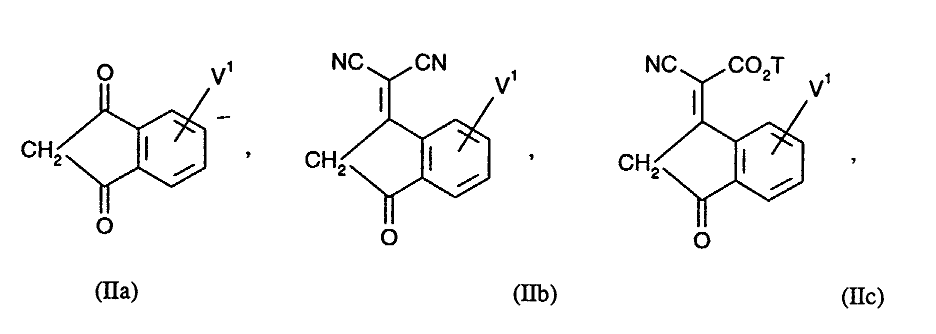 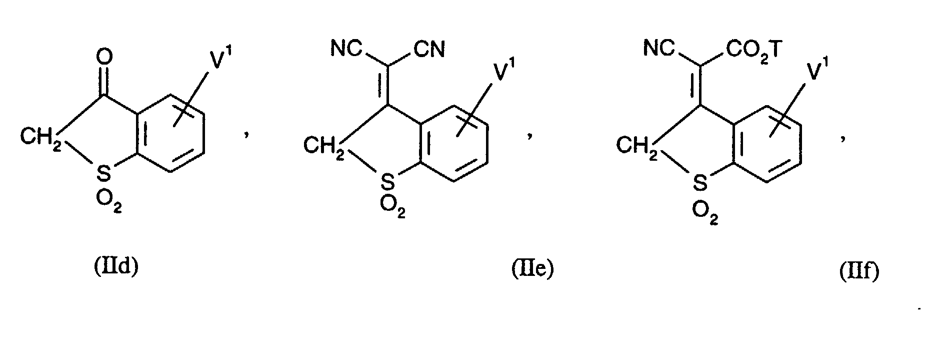 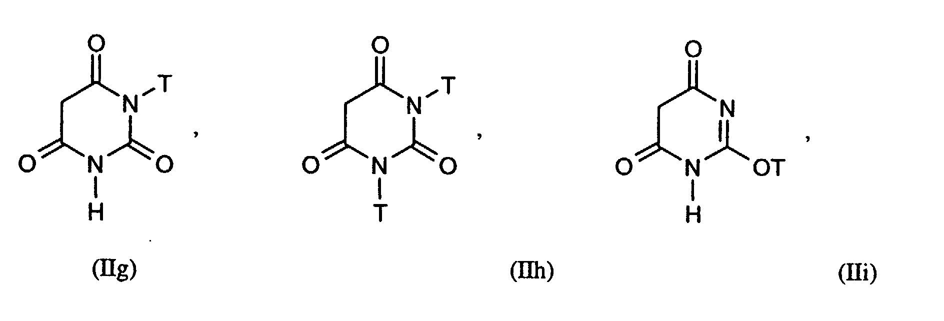 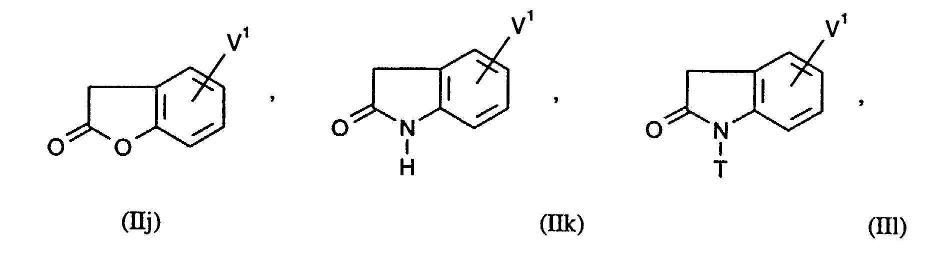 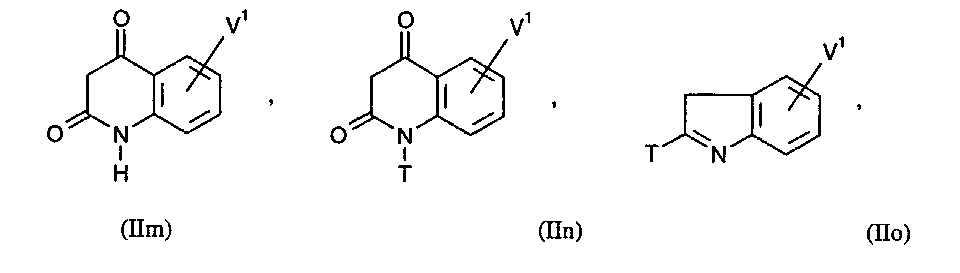 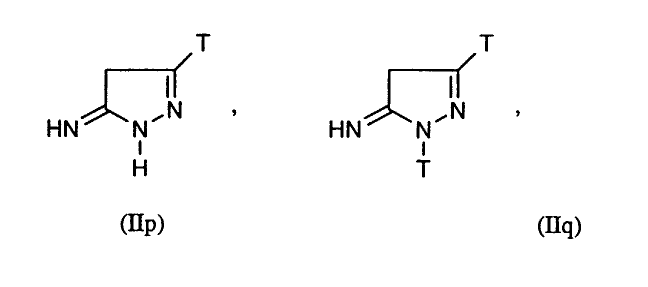 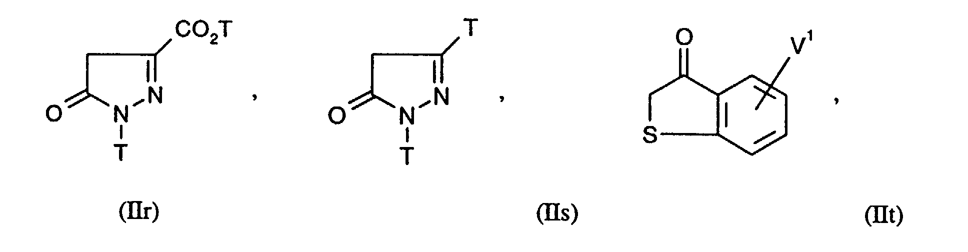 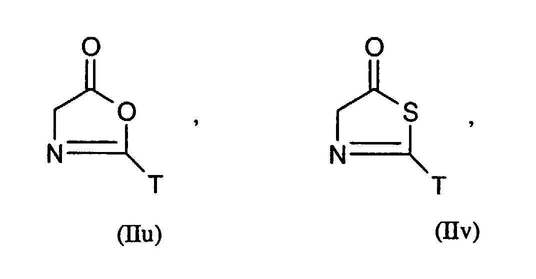
wherein
V¹ represents H or a substituent, in particular Cl, Br, CH₃, -CO₂T¹, -CN, -NO₂, -CF₃ or -SO₂T¹, and wherein A¹, A², A⁵, D and X have the abovementioned meanings.

7. Compounds according to Claim 4, wherein
A¹ represents H, T, -COH, -COT, -CO₂T, -CN, -CONH₂, -CONHT, -CONT₂, CF₃, OH or halogen, in particular Cl, Br and I,
A² represents H, T, -COH, -COT, -CN, -CO₂T, -CONH₂, -CONHT, -CONT₂, -CF₃, -NO₂, -NO, Cl, Br or I,
A³ and A⁴ independently of one another represent -CN, -CO₂T, -CONH₂, -CONHT, -CONT₂, CF₃, -CHO, -COT, -SO₂T, -SO₃T⁴, -SO₃T⁵, SO₂NH₂, SO₂NHT, SO₂NT₂, -SOT, -CH=NH, -CH=NT, -CT=NH, -CT=NT, -CO-CO₂T, -NO₂, -NO, T⁴ or T⁵, wherein A³ and A⁴ preferably do not simultaneously represent T⁴ and/or T⁵, or
A² and A³, together with the C atoms in between them, form the radical of a fused indene ring which is optionally substituted by R¹, or
A³ and A⁴, together with the C atom to which they are bonded, form a carbocyclic or heterocyclic radical of the formula (IIa) to (IIv),
A⁵ denotes H, T¹, T², T³, T⁴, -NH₂, -NHT, NT₂, -NHCOT, -NHCOH, -NHSO₂T or -N=CT₂ or A⁴ and A⁵, together with the atoms of the pyridone ring in between, form a fused ring of the formula 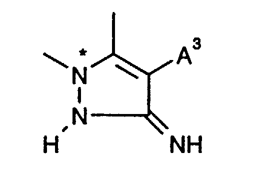 wherein the N atom labelled with * corresponds to the pyridone nitrogen,
T¹ denotes C₁-C₁₀-alkyl, C₅-C₇-cycloalkyl or C₆-C₁₀-aryl-C₁-C₆-alkyl, which are optionally substituted by one or more substituents from the group consisting of -OH, -C₁-C₁₀-alkoxy, -O[(CH₂)₂₋₁₀-O]₁₋₆-alkyl, in particular -C₁-C₁₀-alkoxy-C₂-C₅-alkoxy, -C₁-C₅-alkoxy-C₂-C₅-alkoxy-C₂-C₅-alkoxy or -O-(CH₂-CH₂O 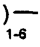 alkyl, -OCOT, -OSO₂T, -O-(CH₂-CH₂O 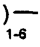 COT, -COT, -SO₂T, -CO₂T, -CN, -CO₂H, -CONT₂, -CF₃, Cl, Br and I,
T² and T³ independently of one another denote C₂-C₁₀-alkenyl or alkinyl, which are optionally substituted by one or more substituents from the group consisting of OH, -C₁-C₆-alkoxy, -OCOT, -OCOH, -CO₂T, -CO₂H, -CN, Cl, Br and I,
T⁴ denotes phenyl, which is optionally substituted by one or more substituents such as -C₁-C₁₀-alkoxy, 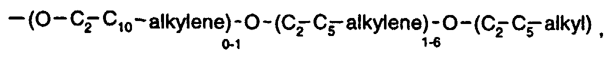
in particular
C₁-C₁₀-alkoxy-C₂-C₅-alkoxy, C₁-C₅-alkoxy-C₂-C₅-alkoxy-C₂-C₅-alkoxy, -OCOH, -OCOT, -OSO₂T, -COH, -COT, -SO₂T, -CO₂T, -CN, -CF₃, -CCl₃, -NO₂, -NO, -CO₂H, -CONH₂, -CONHT, -CONT₂, -SO₂NT², -C₁-C₁₀-alkyl, optionally substituted by -OH, -CN, -CO₂-C₁-C₆-alkyl, Cl, Br or -C₂-C₁₀-alkenyl, optionally substituted by -OH, -CN, -CO₂-C₁-C₆-alkyl, Cl, Br or I, and -C₂-C₁₀-alkinyl, optionally substituted by C₁-C₁₀-alkoxy, -OH, -OCOH, -OCOT, Cl, Br or I,
T⁵ denotes thiophene, furan, pyrrole, 1,2-isothiazole, 1,3-thiazole, pyrrazole, oxazole, isooxazole, imidazole, triazole, pyridine, pyridazine, pyrimidine, pyrazine, triazine, oxazine, benzothiazole, benzimidazole, benzoxazole, quinoline, isoquinoline, indole, coumarone, thionaphthene or tetrazole, optionally substituted by 1 to 3 substituents such as are already described as substituents for phenyl,
X denotes O, -NCOT, -NCO₂T or -NSO₂T,
D denotes a radical of the formula (IIIa) to (IIIu) 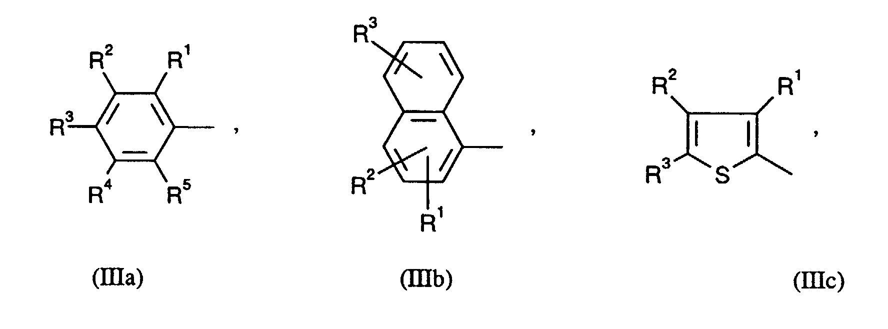 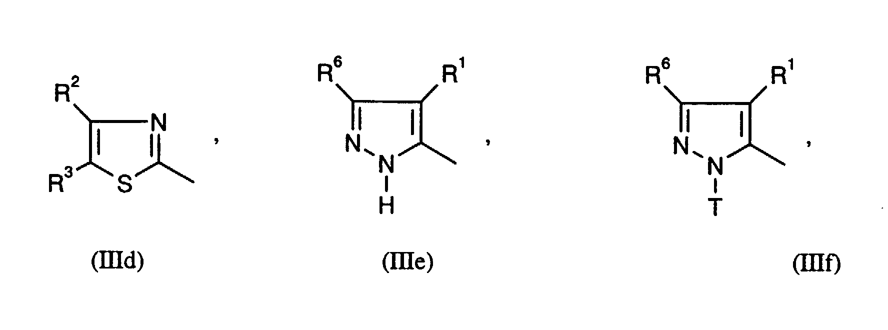 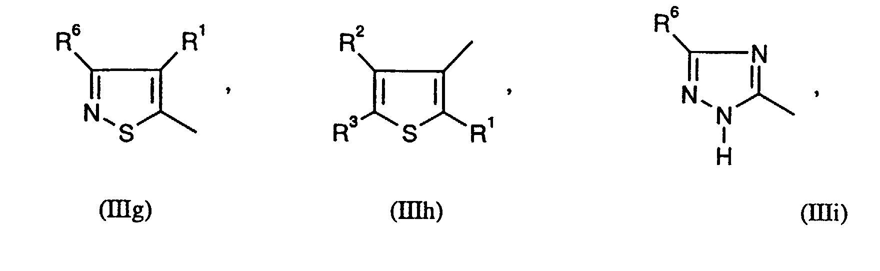 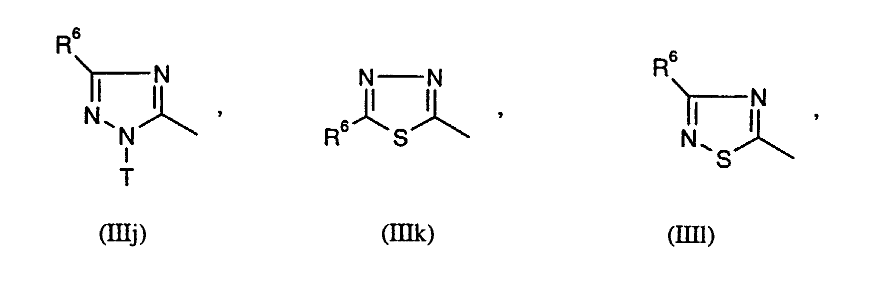 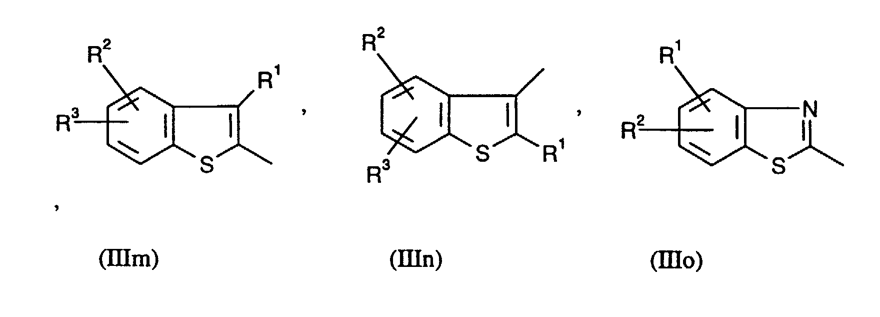 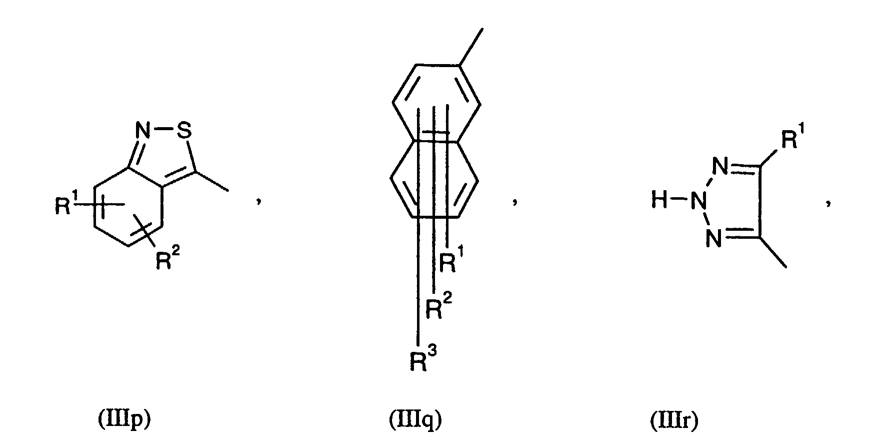 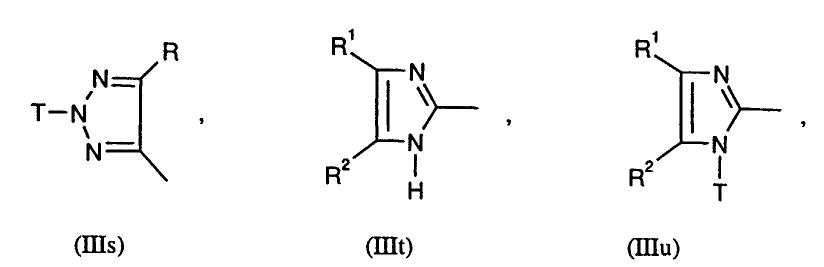
R¹, R², R³, R⁴, R⁵ and R⁶ independently of one another denote
H, T, F, Cl, Br, I, -CN, -NO₂, -CH=O, -COT, -CO₂T¹, -CONH₂, -CONHT, CONT₂, -CF₃, -SO₂NH₂, -SO₂NHT, -SO₂NT₂, -SOT, -SO₂T, -SO₃T, -OT,-OH, -OCOT, -OCO₂T, -OSO₂T, -NH₂, -NHT, -NT₂, 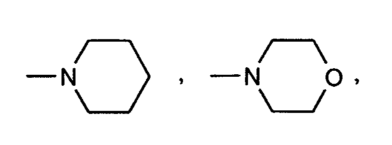 -NHCOH, -NHCOT, -NHSO₂T or -COCO₂T,
wherein
R³ can additionally denote -N=N-T⁴ or -N=N-T⁵ and
R⁶ can additionally denote ST.

8. Compounds according to Claim 4, wherein
A¹ represents H, T¹ or -CF₃,
A² denotes H, -CN or -CO₂T¹,
A³ represents -CN or -CO₂T¹,
A⁴ denotes A³, or
A³ and A⁴, together with the C atom to which they are bonded, form a cyclic methylene-active compound of the formula (IIa) or (IId), where the radicals of the cyclic methylene-active compound are shown in the form of 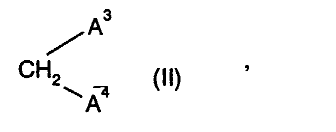
A⁵ represents H, T¹ or T²,
D represents a radical of the formula 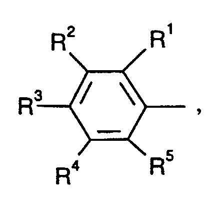 wherein
R¹ and R⁵ independently of one another denote H, Cl, Br, -CN, -NO₂, -CO₂T¹, T¹, -OT¹ or -OT⁴,
R² and R⁴ independently of one another denote H, Cl, Br, -NO₂, T¹ or -OT¹,
R³ represents H, Cl, Br, -CN, -NO₂, T¹, -CO₂T¹ or -OT¹,
T¹ represents C₁-C₆-alkyl or C₁-C₄-T⁴, optionally substituted by C₁-C₆-alkoxy, -C₁-C₄-alkoxy-C₂-C₅-alkoxy or -CO₂T¹,
T² denotes C₂-C₆-alkenyl which is optionally substituted by Cl and/or Br,
T⁴ represents phenyl, which is optionally substituted by Cl, Br, -NO₂ and/or -CO₂T¹ and
X denotes 0.

9. Process for the preparation of compounds according to Claim 1, characterized in that amines of the formula (IV)
D-NH₂ (IV)
are diazotized and the diazotization products are coupled to coupling components of the formula (V) 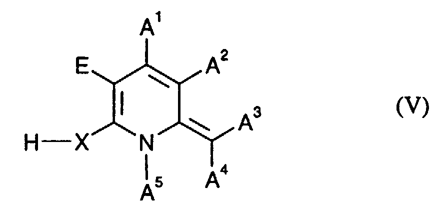 wherein A¹ to A⁵, D and X have the meaning given in Claim 1 and
E represents a substituent which can be replaced by electrophilic substitution, such as, for example, H, -CO₂H, -CH₂OH, -SO₃H, -CH=O, -COT, 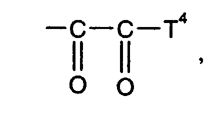 -CONH₂ or -CONHT.

10. Process for the preparation of dyestuffs of the formula (I), wherein X is O, characterized in that compounds of the formula (VII), which may be present in the tautomeric forms of the formulae (VIIa) and (VIIb), 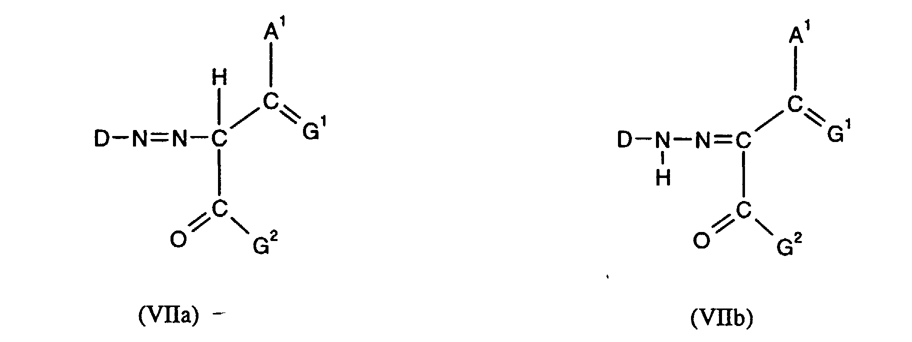 wherein
G¹ denotes O, NH or NT, preferably O,
G² represents -OT, -NH₂ or -NHT, preferably OT¹, and
D and A¹ have the abovementioned meanings,
are subjected to a condensation reaction with enamines of the formula (VIII) 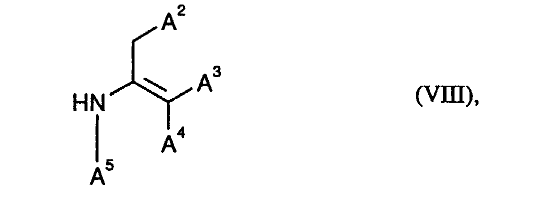 wherein A² to A⁵ have the abovementioned meanings.

11. Enamines of the formula (VIIIa) to (VIIIc)

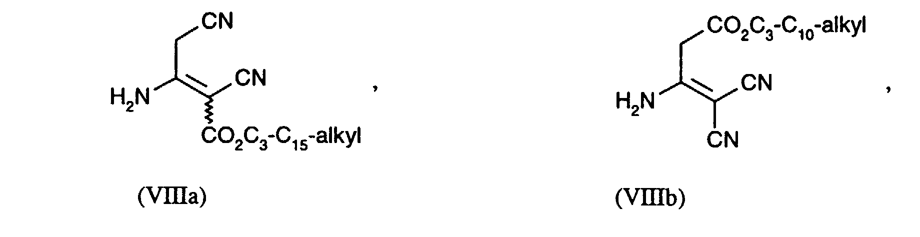

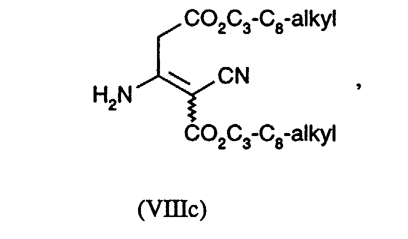

12. Compounds of the formula (V) 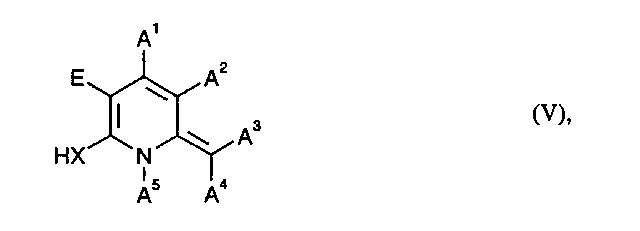 wherein
E and X have the broadest meanings mentioned in Claim 9 and Claim 1,
A¹ and A² independently of one another denote H or a radical of the formula T, -COH, -CO-T, -CO₂T, -CN, -CONH₂, -CONHT, -CONT₂, CF₃, -NO₂, NO, -SO₂T, -OH, -OT, -OCOT, -OCO₂T, -OSO₂T, Cl, Br or I,
wherein
T can assume the meaning of T¹, T², T³, T⁴ or T⁵, where
T¹ = alkyl, cycloalkyl or aralkyl,
T² = alkenyl,
T³ = alkinyl,
T⁴ = aryl,
T⁵ = hetaryl,
A³ and A⁴ independently of one another denote an electron-withdrawing radical, or together with the common C atom form a cyclic methylene-active compound,
A⁵ represents H or a radical of the formula T, -OT¹, -NH₂, -NHT, -NT₂, -NHCOH, -NHCOT, -N=CH-T, -N=CT₂ or NHSO₂T, or
A¹ and A² or
A² and A³, together with the particular atoms in between, form an unsaturated, optionally substituted 5- or 6-membered carbo- or heterocyclic radical, where, in the case of ring formation with participation of one of the radicals A³ or A⁴, the other particular radical denotes an electron-withdrawing radical, and where the radicals A¹ to A⁵ optionally carry one or more SO₃H, COOH or K^{⊕} and B^{⊖} groups, wherein K^{⊕} and B^{⊖} have the above meanings.

13. Process for the preparation of compounds of the formula (V) in the form of the compound Va or Vb, wherein X represents O or NH, characterized in that acetic acid derivatives of the formula IXa or IXb are reacted with enamines of the formula (VIII) 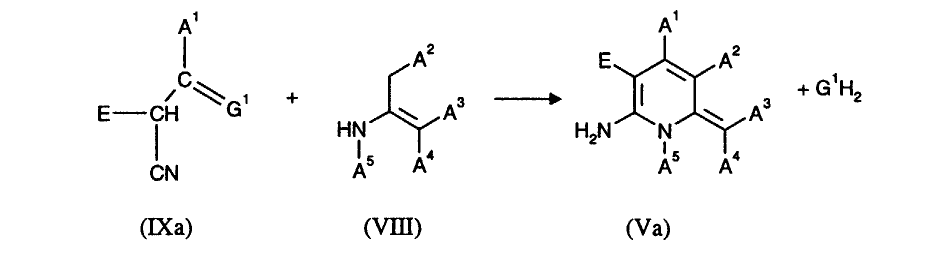 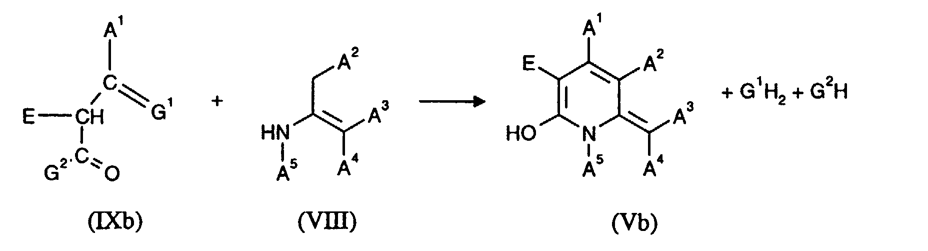 wherein
A¹ to A⁵ and X have the meaning given in Claim 1,
E represents a substituent which can be replaced by electrophilic substitution,
G¹ denotes O, NH or NT and
G² represents OT, NH₂ or NHT, wherein T has the meaning given in Claim 1.

14. Process for the preparation of compounds of the formula V which correspond to the formula X, characterized in that trifluoroacetic esters of the formula (XI) are subjected to a condensation reaction with dimeric cyanoacetic acid esters of the formula (XII) 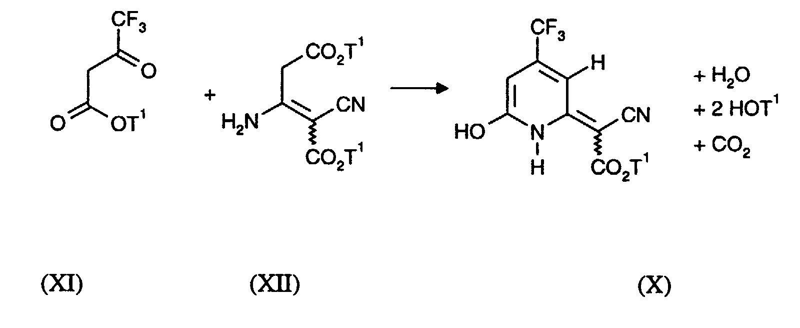 wherein T¹ has the meaning given in Claim 1.

15. Process for the preparation of compounds of the formula (V) 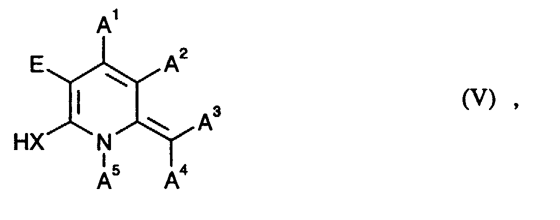 wherein A⁵ represents T¹, T² or T³, which is characterized in that pyridinium salts of the formula (XIII) 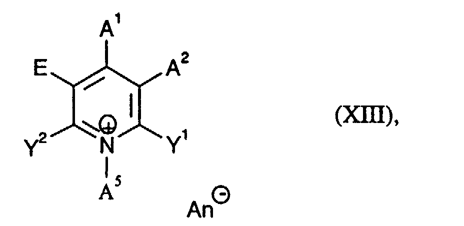 wherein
A¹ and A² have the meaning given in Claim 1,
E represents a substituent which can be replaced by electrophilic substitution,
An^{⊖} is an anion, which can assume, for example, the meaning given for B^{⊖}, and in particular represents Cl^{⊖} , Br^{⊖} , I^{⊖}, T¹OSO₃^{⊖} or TSO₃^{⊖},
A⁵ represents T¹, T² or T³ and
Y¹ and Y² independently of one another represent a leaving group, such as F, Cl, Br, I, -OSO₂T, -OT, -ST or -SO₂T,
are reacted with compounds of the formula (II) 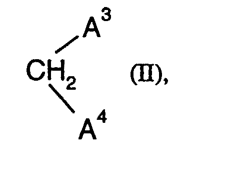 wherein
A³ and A⁴ have the abovementioned broadest meaning,
to give compounds of the formula (XIV) 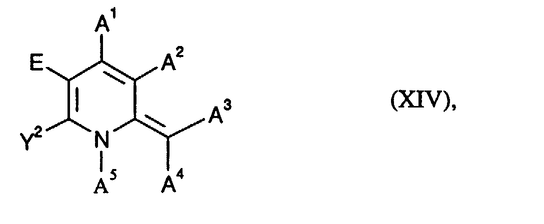 and these are reacted with compounds of the formula (XV)
XH₂ (XV)
wherein
X represents O, NH, NT¹, NCOT, NCO₂T or NSO₂T,
to give compounds of the formula (V)
wherein A⁵ represents T¹, T² or T³.

16. Process for the preparation of the coupling components of the formula (V), wherein A⁵ represents T, characterized in that pyridine derivatives of the formula (XVII) are reacted with compounds of the formula (II): 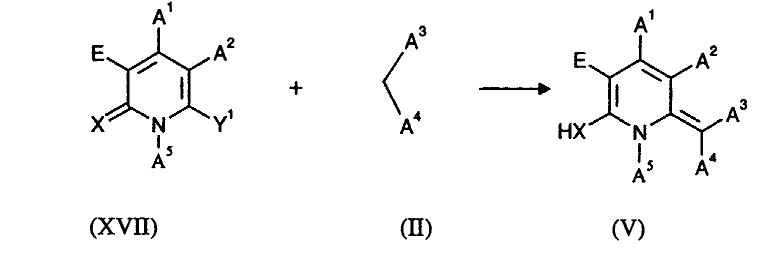 wherein X=O, A¹ to A⁴ have the meanings given in Claim 1 and A⁵ represents T and
E represents a substituent which can be replaced by electrophilic substitution.

17. Process for the preparation of compounds of the formula (V) which correspond to the formula (XX), wherein A⁵ represents T¹, T² or T³, characterized in that 2-methylpyridone of the formula (XVIII) are reacted with phthalic acid esters of the formula (XIX) in the presence of bases 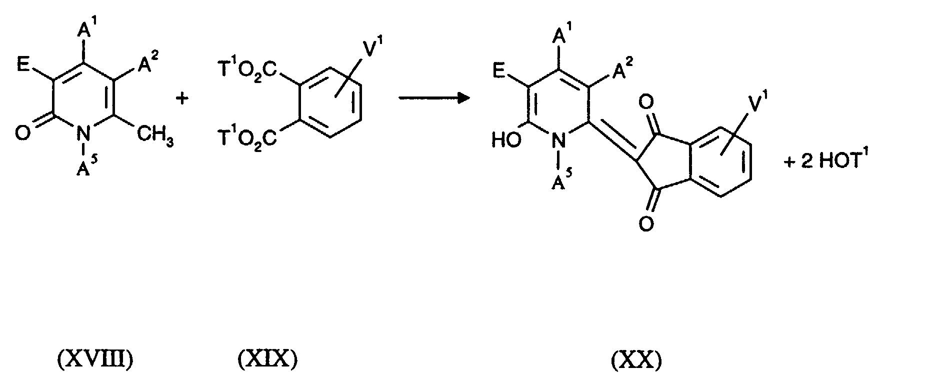 wherein A¹, A², E, T¹ and V¹ have the meaning given in Claim 8.

18. Use of the dyestuffs according to Claim 1 for dyeing and printing naturally occurring synthetic materials, such as, for example, cellulose fibres, cotton, wool, silk, polyamide, polyacrylonitrile, polyester or polyolefins.

19. Use of the dyestuffs according to Claim 4 for dyeing and printing hydrophobic synthetic fibre materials and mixtures thereof with naturally occurring fibre materials.

## Revendications

1. Composés qui, sous forme de l'acide libre, correspondent à la formule (I) ou à sa forme tautomère, 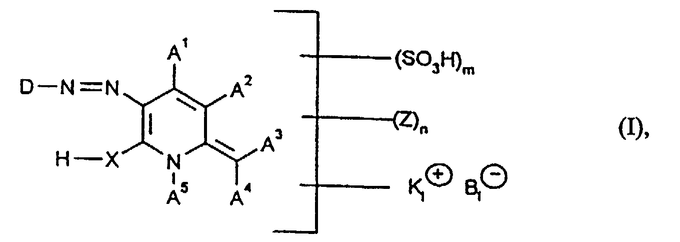 dans laquelle
D représente le reste d'un constituant diazoïque carbocyclique ou hétérocyclique,
A¹ et A² représentent indépendamment l'un de l'autre H ou un substituant typique de pyridone,
A³ et A⁴ représentent indépendamment l'un de l'autre un reste attracteur d'électrons ou forment ensemble, avec l'atome de carbone commun, un composé cyclique à méthylène actif,
A⁵ représente H, un reste de formule T, -OT¹, -NH₂, -NHT, -NT₂, -NHCOH, -NHCOT, -N=CH-T, -N=CT₂ ou -NHSO₂T, où
T¹ représente un reste alkyle, cycloalkyle ou aralkyle, et T est T¹ ou peut prendre la signification de T² à T⁵, où
T² est un reste alcényle,
T³ est un reste alcynyle,
T⁴ est un reste aryle,
T⁵ est un reste hétéroaryle, ou
A¹ et A² et/ou
A² et A³ et/ou
A⁴ et A⁵ forment ensemble, avec les atomes correspondants qui se trouvent entre eux, un noyau carbocyclique ou hétérocyclique de 5 ou 6 chaînons insaturé, éventuellement substitué, à condition que, dans le cas de la formation d'un cycle faisant intervenir l'un des deux restes A³ et A⁴, le reste n'intervenant pas soit un reste attracteur d'électrons,
X représente O, NH, NT, NCOT, NCO₂T ou NSO₂T,
K^{⊕} est -NH₃^{⊕}, -NHT₂^{⊕}, -NH₂T^{⊕}, -NT₃^{⊕} ou un ion cycloimmonium,
B^{⊖} est un anion,
Z représente un reste réactif avec les fibres,
l est un nombre de 0 à 2,
m est un nombre de 0 à 8 et
n est un nombre de 0 à 6.

2. Composés de formule (I) selon la revendication 1, dans lesquels
A¹ et A² représentent indépendamment l'un de l'autre H ou un reste de formule T, -COH, -CO-T, -CO₂T, -CN, -CONH₂, -CONHT, -CONT₂, CF₃, -NH₂, -NHT, -NT₂, -NH-COT, -NT-COT, -NHSO₂T, -NTSO₂T, -NO₂, -NO, -SO₂T, -OH, -OT, -OCOT, -OCO₂T, -OSO₂T, Cl, Br, I, où
T a la signification indiquée dans la revendication 1.

3. Composés selon la revendication 1, dans lesquels 1 = 1 ou 2, n = 0 et m est inférieur à 1, et est de préférence égal à 0.

4. Composés selon la revendication 1, dans lesquels 1 = m = n = 0.

5. Composés selon la revendication 1, dans lesquels 1 = 0 et m et/ou n sont différents de 0.

6. Composés selon la revendication 1, dans lesquels
A³ et A⁴ représentent indépendamment l'un de l'autre un reste -CN, -CO₂T, -CONH₂, -CONHT, -CONT₂, -CF₃, -CHO, -COT, -SO₂T, -SO₃T⁴, -SO₃T⁵, -SO₂NH₂, -SO₂NHT, -SO₂NT₂, -SOT, -CH=NH, -CH=NT, -CT=NH, -CT=NT, 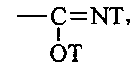 -CO-CO₂T, -NO₂, -NO, T⁴ ou T⁵
ou
A³ et A⁴ représentent ensemble, avec l'atome de carbone auquel ils sont liés, un composé cyclique à méthylène actif de formule (IIa) à (IIv), ces restes étant indiqués sous forme de 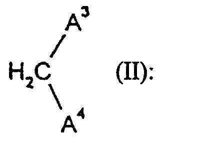 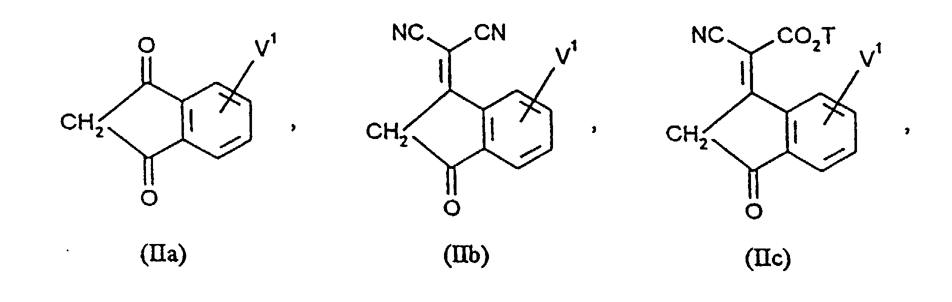 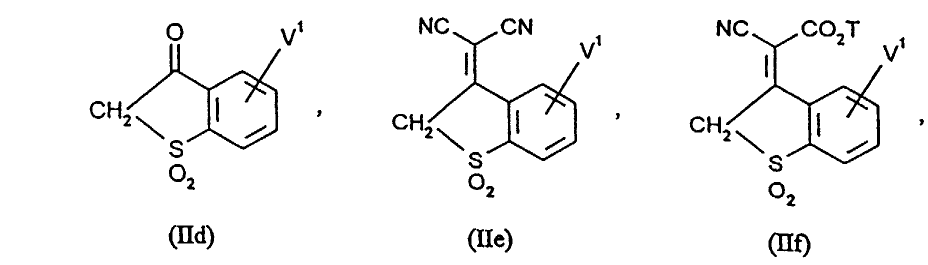 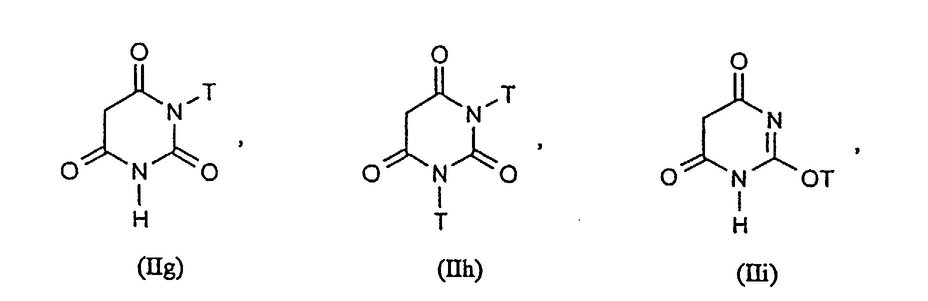 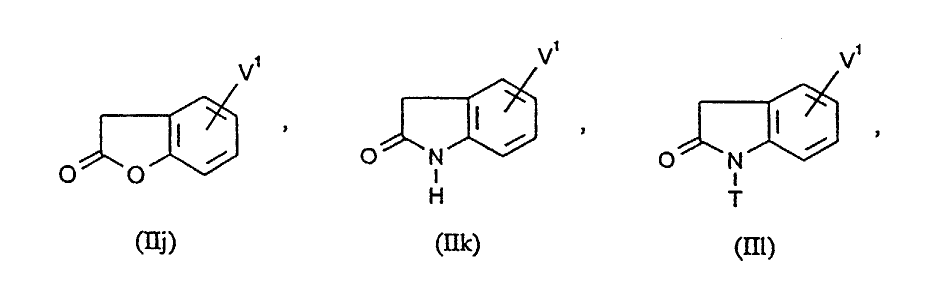 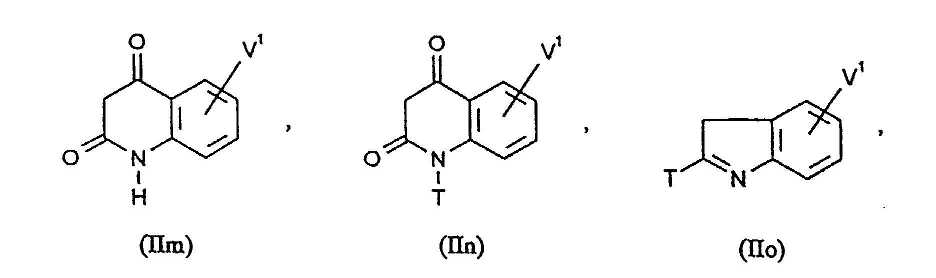 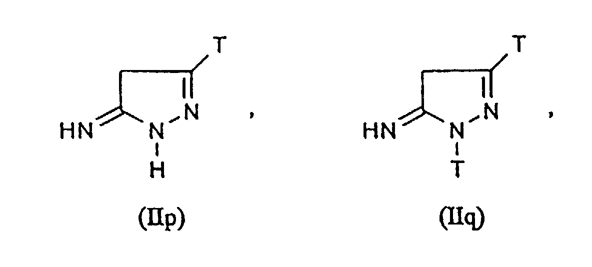 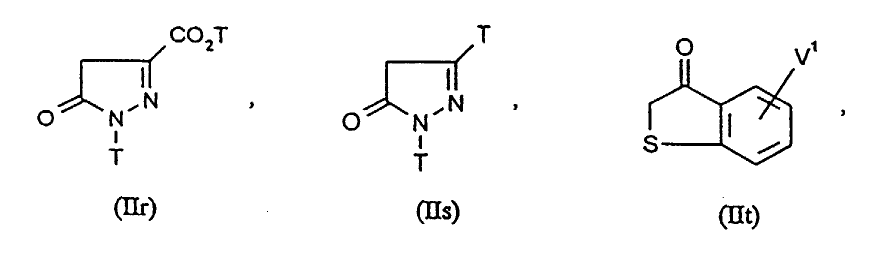 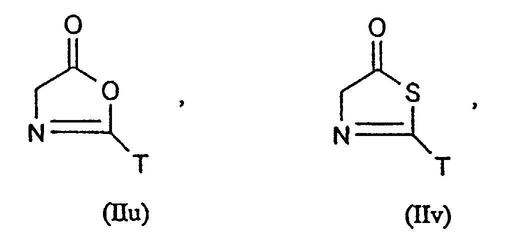
où
V¹ représente H ou un substituant, en particulier Cl, Br, CH₃, -CO₂T¹, -CN, -NO₂, -CF₃ ou -SO₂T¹, et A¹, A², A⁵, D et X ont la signification donnée précédemment.

7. Composés selon la revendication 4, dans lesquels
A¹ représente H, T, -COH, -COT, -CO₂T, -CN, -CONH₂, -CONHT, -CONT₂, -CF₃, OH ou un halogène, en particulier Cl, Br et I,
A² représente H, T, -COH, -COT, -CN, -CO₂T, -CONH₂, -CONHT, -CONT₂, -CF₃, -NO₂, -NO, Cl, Br, I,
A³ et A⁴ représentent indépendamment l'un de l'autre -CN, -CO₂T, -CONH₂, -CONHT, -CONT₂, -CF₃, -CHO, -COT, -SO₂T, -SO₃T⁴, -SO₃T⁵, -SO₂NH₂, -SO₂NHT, -SO₂NT², -SOT, -CH=NH, -CH=NT, -CT=NH, -CT=NT, -CO-CO₂T, -NO₂, -NO, T⁴ ou T⁵, A³ et A⁴ n'étant de préférence pas T⁴ et/ou T⁵ en même temps, ou
A² et A³ forment ensemble, avec les atomes de carbone se trouvant entre eux, le reste d'un cycle indène condensé éventuellement substitué par R¹, ou
A³ et A⁴ forment ensemble, avec l'atome de carbone auquel ils sont liés, un noyau carbocyclique ou hétérocyclique de formule (IIa) à (IIv),
A⁵ représente H, T¹, T², T³, T4, -NH₂, -NHT, -NT₂, -NHCOT, -NHCOH, -NHSO₂T, -N=CT₂, ou A⁴ et A⁵ forment ensemble, avec les atomes du noyau pyridone qui se trouvent entre eux, un noyau condensé de formule 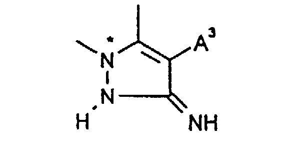
où l'atome d'azote désigné par * correspond à l'azote de la pyridone,
T¹ est un reste alkyle en C₁-C₁₀, cycloalkyle en C₅-C₇ ou (aryl en C₆-C₁₀)-(akyle en C₁-C₆), éventuellement substitué par un ou plusieurs substituants du groupe constitué par -OH, alcoxy en C₁-C₁₀, -O[(CH₂)₂₋₁₀-O]₁₋₆-alkyle, en particulier -(alcoxy en C₁-C₁₀)-(alcoxy en C₂-C₅), -(alcoxy en C₁-C₅)-(alcoxy en C₂-C₅)-(alcoxy en C₂-C₅) ou -O-(CH₂-CH₂O)₁₋₆-alkyle, -OCOT, -OSO₂T, -O-(CH₂-CH₂O)₁₋₆-COT, -COT, -SO₂T, -CO₂T, -CN, -CO₂H, -CONT₂, -CF₃, Cl, Br, I,
T² et T³ représentent indépendamment l'un de l'autre un reste alcényle ou alcynyle en C₂-C₁₀ éventuellement substitué par un ou plusieurs substituants du groupe constitué par -OH, alcoxy en C₁-C₆, -OCOT, -OCOH, -CO₂T, -CO₂H, -CN, Cl, Br, I,
T⁴ représente un reste phényle éventuellement substitué par un ou plusieurs substituants comme alcoxy en C₁-C₁₀, -(O-alkylène en C₂-C₁₀)₀₋₁-O-(alkylène en C₂-C₅)₁₋₆-O-(alkyle en C₂-C₅), en particulier (alcoxy en C₁-C₁₀)-(alcoxy en C₂-C₅), (alcoxy en C₁-C₅)-(alcoxy en C₂-C₅)-(alcoxy en C₂-C₅), -OCOH, -OCOT, -OSO₂T, -COH, -COT, -SO₂T, -CO₂T, -CN, -CF₃, -CCl₃, -NO₂, -NO, -CO₂H, -CONH₂, -CONHT, -CONT₂, -SO₂NT², alkyle en C₁-C₁₀, éventuellement substitué par -OH, -CN, -CO₂-(dkyle en C₁-C₆), Cl, Br, I, alcényle en C₂-C₁₀, éventuellement substitué par -OH, -CN, -CO₂-(dkyle en C₁-C₆), Cl, Br, I, alcynyle en C₂-C₁₀, éventuellement substitué par alcoxy en C₁-C₁₀, -OH, -OCOH, -OCOT, Cl, Br, I,
T⁵ représente un reste thiophène, furane, pyrrole, 1,2-isothiazole, 1,3-thiazole, pyrazole, oxazole, isoxazole, imidazole, triazole, pyridine, pyridazine, pyrimidine, pyrazine, triazine, oxazine, benzothiazole, benzimidazole, benzoxazole, quinoléine, isoquinoléine, indole, coumarone, thionaphtène, tétrazole, éventuellement substitué par 1 à 3 substituants comme ceux déjà décrits comme substituants pour le groupe phényle,
X représente O, -NCOT, -NCO₂T, -NSO₂T,
D représente un reste de formule (IIIa) à (IIIu) 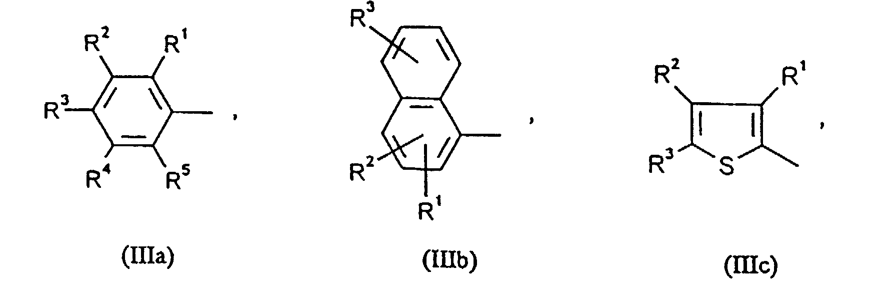 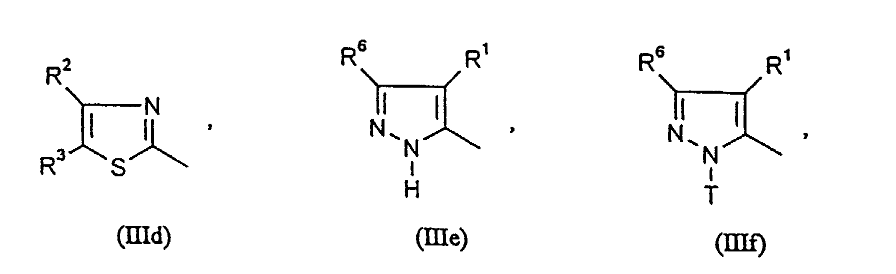 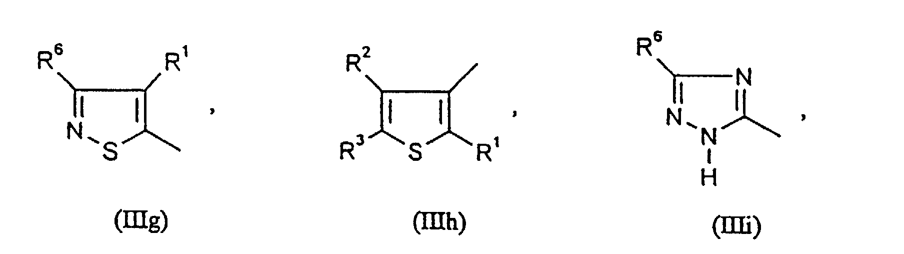 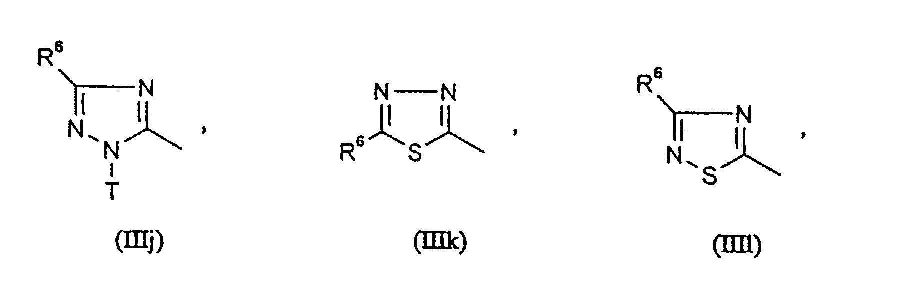 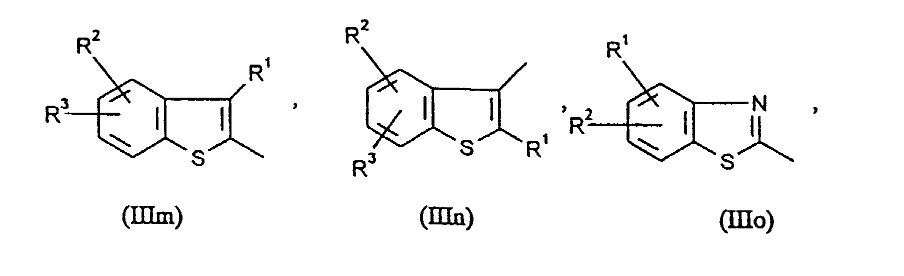 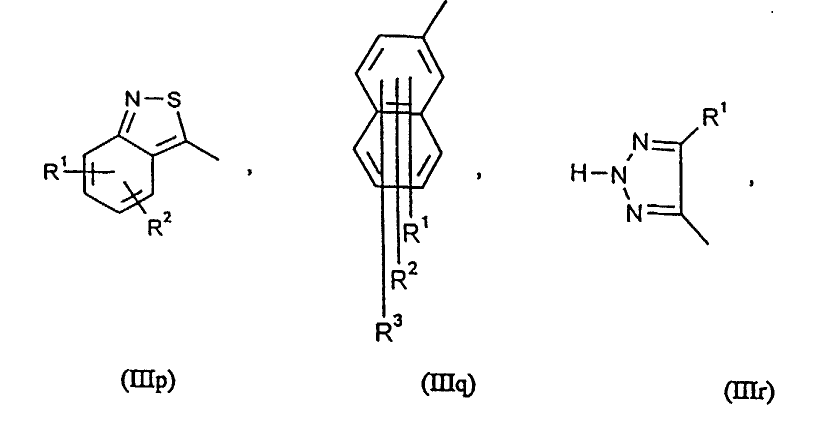 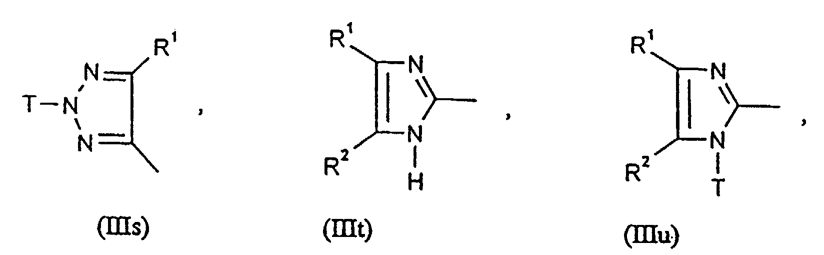
R¹, R², R³, R⁴, R⁵ et R⁶ représentent, indépendamment les uns des autres, H, T, F, Cl, Br, I, -CN, -NO₂, -CH=O, -COT, -CO₂T¹, -CONH₂, -CONHT, -CONT₂, -CF₃, -SO₂NH₂, -SO₂NHT, -SO₂NT₂, -SOT, -SO₂T, -SO₃T, -OT, -OH, -OCOT, -OCO₂T, -OSO₂T, -NH₂, -NHT, -NT₂, 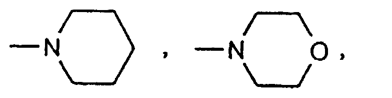 -NHCOH, -NHCOT, -NHSO₂T ou -COCO₂T,
R³ pouvant être en outre -N=N-T⁴ ou -N=N-T⁵, et
R⁶ pouvant représenter en outre ST.

8. Composés selon la revendication 4, dans lesquels
A¹ représente H, T¹ ou -CF₃,
A² représente H, -CN ou -CO₂T¹,
A³ représente -CN ou -CO₂T¹,
A⁴ représente A³, ou
A³ et A⁴ forment ensemble, avec l'atome de carbone auquel ils sont liés, un composé cyclique à méthylène actif de formule (IIa) ou (IId), les restes du composé cyclique à méthylène actif étant donnés sous forme de 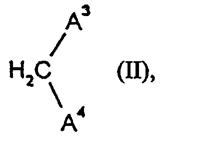
A⁵ représente H, T¹ ou T²,
D représente un reste de formule 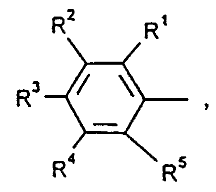 dans laquelle
R¹ et R⁵ représentent indépendamment l'un de l'autre H, Cl, Br, -CN, -NO₂, -CO₂T¹, T¹, -OT¹ ou -OT⁴,
R² et R⁴ représentent indépendamment l'un de l'autre H, Cl, Br, -NO₂, T¹ ou -OT¹,
R³ représente H, Cl, Br, -CN, -NO₂, T¹, -CO₂T¹ ou -OT¹,
T¹ représente un reste alkyle en C₁-C₆ ou C₁-C₄-T⁴, éventuellement substitué par alcoxy en C₁-C₆, (alcoxy en C₁-C₄)-(alcoxy en C₂-C₅), -CO₂T¹,
T² représente un reste alcényle en C₂-C₆, éventuellement substitué par Cl et/ou Br,
T⁴ représente un reste phényle éventuellement substitué par Cl, Br, -NO₂ et/ou -CO₂T¹, et
X représente O.

9. Procédé de préparation de composés selon la revendication 1, caractérisé en ce que l'on diazote des amines de formule (IV)
D-NH₂ (IV)
et on les soumet à une copulation avec des copulants de formule (V) 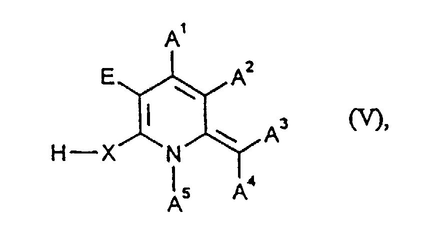 dans laquelle A¹ à A⁵, D et X ont la signification donnée dans la revendication 1 et E représente un substituant déplaçable par substitution électrophile comme, par exemple, H, -CO₂H, -CH₂OH, -SO₃H, -CH=O, -COT, 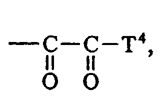 -CONH₂, -CONHT.

10. Procédé de préparation de colorants de formule (I) dans laquelle X est O, caractérisé en ce que l'on condense des composés de formule (VII), qui peuvent exister sous les formes tautomères de formule (VIIa) et (VIIb) 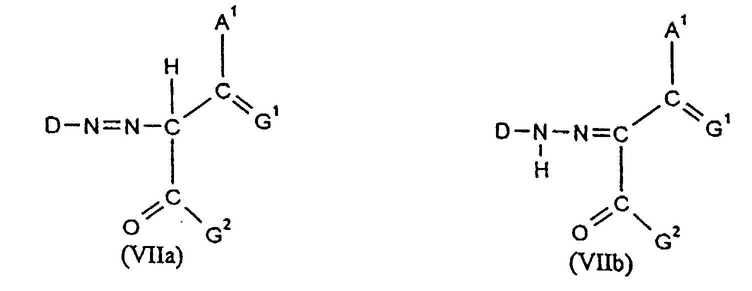 dans lesquelles
G¹ est O, NH ou T, de préférence O,
G² est -OT, -NH₂ ou -NHT, de préférence OT¹,
D et A¹ ont la signification indiquée précédemment,
avec des énamines de formule (VIII) 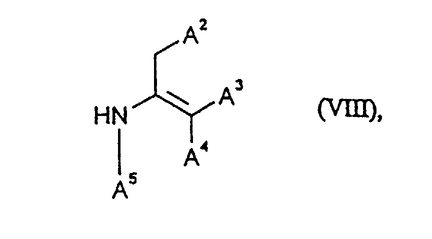 dans laquelle A² à A⁵ ont la signification indiquée précédemment.

11. Enamines de formule (VIIIa) à (VIIIc)

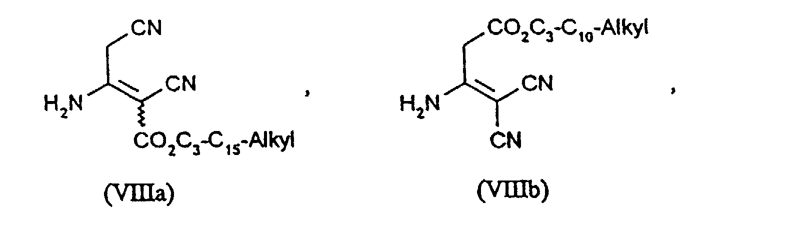

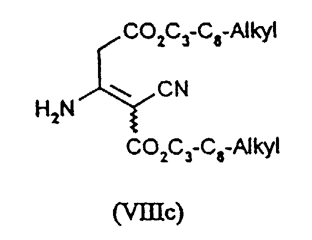

12. Composés de formule (V) 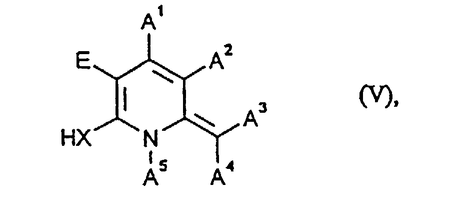 dans laquelle
E et X ont les significations les plus larges indiquées dans la revendication 9 et la revendication 1,
A¹ et A² représentent, indépendamment l'un de l'autre, H ou un reste de formule T, -COH, -CO-T, -CO₂T, -CN, -CONH₂, -CONHT, -CONT₂, -CF₃, -NO₂, -NO, -SO₂T, -OH, -OT, -OCOT, -OCO₂T, -OSO₂T, Cl, Br, I, où
T peut prendre la signification de T¹, T², T³, T⁴ ou T⁵, où
T¹ = alkyle, cycloalkyle ou aralkyle,
T² = alcényle,
T³ = alcynyle,
T⁴ = aryle,
T⁵ = hétéroaryle,
A³ et A⁴ représentent indépendamment l'un de l'autre un reste attracteur d'électrons ou forment ensemble, avec l'atome de carbone commun, un composé cyclique à méthylène actif,
A⁵ représente H, un reste de formule T, -OT¹, -NH₂, -NHT, -NT₂, -NHCOH, -NHCOT, -N=CH-T, -N=CT₂ ou -NHSO₂T, ou
A¹ et A² ou
A² et A³ forment ensemble, avec les atomes correspondants se trouvant entre eux, un noyau carbocyclique ou hétérocyclique de 5 ou 6 chaînons insaturé, éventuellement substitué,
et, dans le cas de la formation d'un cycle avec participation de l'un des restes A³ ou A⁴, l'autre reste représente un reste attracteur d'électrons, et les restes A¹ à A⁵ portent éventuellement un ou plusieurs groupes SO₃H, COOH ou K^{⊕}B^{⊖} , K^{⊕} et B^{⊖} ayant la signification indiquée dans la revendication 1.

13. Procédé de préparation des composés de formule (V) sous forme du composé Va ou Vb, dans lesquels X est O ou NH, caractérisé en ce que l'on fait réagir des dérivés d'acide acétique de formule IXa ou IXb avec des énamines de formule (VIII): 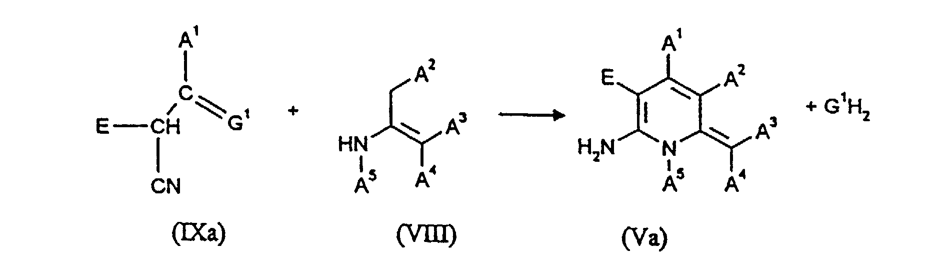 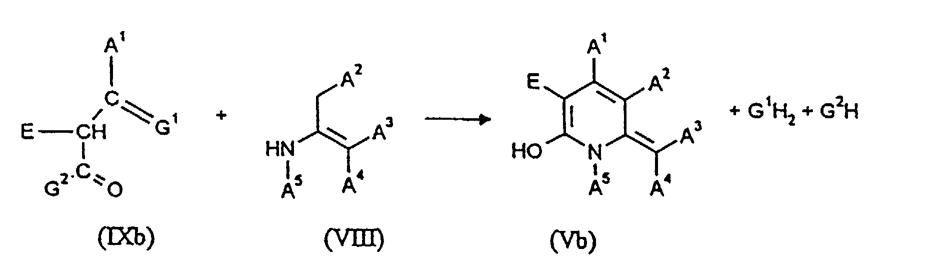 où
A¹ à A⁵ et X ont la signification indiquée dans la revendication 1,
E représente un substituant déplaçable par substitution électrophile,
G¹ représente O, NH ou NT,
G² représente OT, NH₂ ou NHT, T ayant la signification indiquée dans la revendication 1.

14. Procédé de préparation de composés de formule V correspondant à la formule X, caractérisé en ce que l'on condense des trifluoroacétoacétates de formule (XI) avec des esters d'acide cyanoacétique dimères de formule (XII) 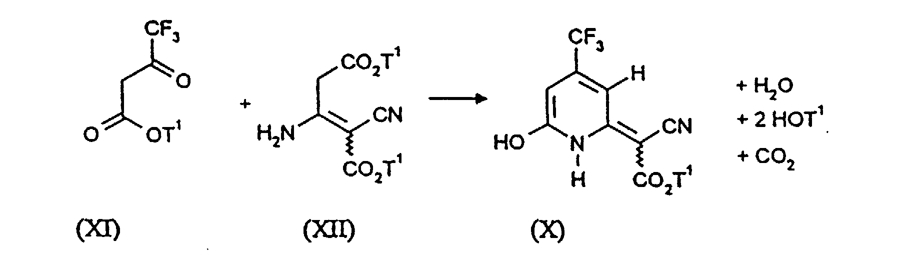 où T¹ a la signification indiquée dans la revendication 1.

15. Procédé de préparation de composés de formule (V) 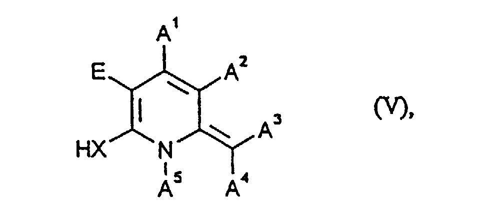 dans laquelle A⁵ représente T¹, T² ou T³, caractérisé en ce que l'on fait réagir des sels de pyridinium de formule (XIII) 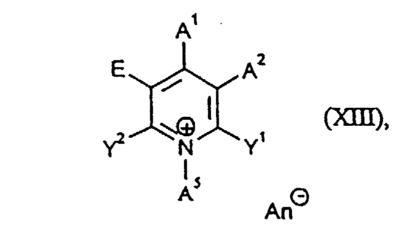 dans laquelle
A¹ et A² ont la signification indiquée dans la revendication 1,
E est un substituant déplaçable par substitution électrophile,
An^{⊖} est un anion qui peut avoir par exemple les significations indiquées pour B^{⊖}, en particulier Cl^{⊖}, Br^{⊖}, I^{⊖} , T¹OSO₃^{⊖} ou TSO₃^{⊖} ,
A⁵ représente T¹, T² ou T³,
Y¹ et Y² représentent, indépendamment l'un de l'autre, un groupe partant comme F, Cl, Br, I, -OSO₂T, -OT, -ST ou -SO₂T,
avec des composés de formule (II) 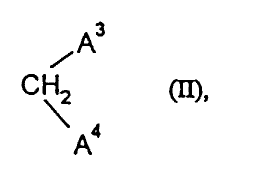 dans laquelle
A³ et A⁴ ont la signification la plus large indiquée précédemment,
pour obtenir des composés de formule (XIV): 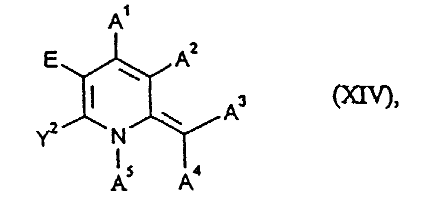 que l'on fait réagir avec des composés de formule (XV)
XH₂ (XV)
dans laquelle
X représente O, NH, NT¹, NCOT, NCO₂T ou NSO₂T,
pour obtenir des composés de formule (V) dans laquelle A⁵ représente T¹, T² ou T³.

16. Procédé de préparation des copulants de formule (V) dans laquelle A⁵ est T, caractérisé en ce que l'on fait réagir des dérivés de pyridine de formule (XVII) avec des composés de formule (II): 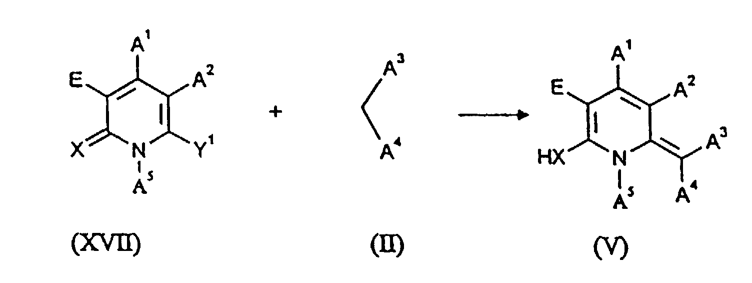 où X = O, A¹ à A⁴ ont la signification donnée dans la revendication 1, A⁵ représente T et
E représente un substituant déplaçable par substitution électrophile.

17. Procédé de préparation de composés de formule (V) correspondant à la formule (XX) dans laquelle A⁵ est T¹, T² ou T³, caractérisé en ce que l'on fait réagir des 2-méthylpyridones de formule (XVIII) avec des esters d'acide phtalique de formule (XIX) en présence de bases 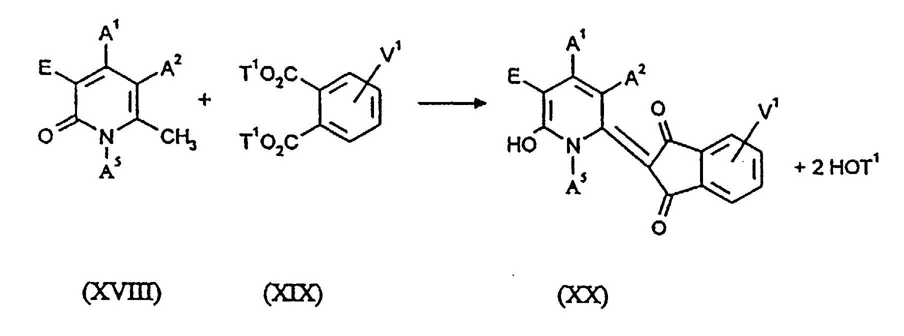 où A¹, A², E, T¹, V¹ ont la signification indiquée dans la revendication 8.

18. Utilisation des colorants selon la revendication 1 pour la teinture et l'impression de matières naturelles et synthétiques comme, par exemple, des fibres de cellulose, du coton, de la laine, de la soie, du polyamide, du polyacrylonitrile, du polyester ou des polyoléfines.

19. Utilisation des colorants selon la revendication 4 pour la teinture et l'impression de matières fibreuses synthétiques hydrophobes et de leurs mélanges avec des matières fibreuses naturelles.
